# NOUVEAU FASCICULE DE BREVET EUROPEEN

(11) **EP 0 971 682 B2**
(45) Date de publication et mention de la décision concernant l'opposition: **23.09.2009**
(45) Mention de la délivrance du brevet: 17.03.2004
(21) Numéro de dépôt: 98947622.1
(22) Date de dépôt: 07.10.1998
(51) Int. Cl.: A61Q 5/10

(54) **COMPOSITION DE TEINTURE DES FIBRES KERATINIQUES ET PROCEDE DE TEINTURE METTANT EN OEUVRE CETTE COMPOSITION**
FÄRBEMITTEL FÜR KERATINISCHE FASER UND VERFAHREN ZUM FÄRBEN UNTER VERWENDUNG DIESER ZUSAMMENSETZUNG
DYEING COMPOSITION FOR KERATIN FIBRES AND DYEING METHOD USING SAME

(30) Priorité: 22.10.1997 FR 9713242
(43) Date de publication de la demande: 19.01.2000
(73) Titulaire: L'ORÉAL, 75008 Paris (FR)
(72) Inventeur: LANG, Gérard, F-40400 Begaar (FR); AUDOUSSET, Marie-Pascale, F-92600 Asnières (FR)
(74) Mandataire: Dossmann, Gérard
(86) Numéro de dépôt international: PCT/FR1998/002144
(87) Numéro de publication internationale: WO 1999/020234

(56) Documents cités:
- WO-A-93/09759
- WO-A-95/01772
- WO-A-95/15144
- DE-A- 3 824 122
- US-A- 3 985 499
- US-A- 4 025 301

## Description

L'invention a pour objet une composition pour la teinture des fibres kératiniques, et en particulier des fibres kératiniques humaines telles que les cheveux comprenant, dans un milieu approprié pour la teinture, au moins un colorant direct cationique, et au moins un colorant auto-oxydable, ainsi que le procédé de teinture mettant en oeuvre cette composition.

Il est connu de teindre les fibres kératiniques et en particulier les cheveux humains avec des compositions tinctoriales contenant des colorants auto-oxydables tels que des dérivés benzéniques comportant au moins trois groupements hydroxyle et/ou amino et des dérivés indoliques, tels que le 5,6-dihydroxy indole. Ces colorants auto-oxydables ont la particularité de pouvoir s'oxyder sans autre agent oxydant que l'oxygène de l'air, pour donner naissance à des molécules colorées et colorantes. Cependant les colorations obtenues en mettant en oeuvre ces colorants ne sont pas toujours satisfaisantes notamment du point de vue de leur puissance et de leur chromaticité.

Il est également connu, entre autres par les brevets US 3985499 et US 4025301, de teindre les fibres kératiniques avec des colorants directs et en particulier avec des colorant directs cationiques. Les colorants directs ont l'inconvénient, lorsqu'ils sont incorporés dans des compositions tinctoriales, de conduire à des colorations présentant une ténacité insuffisante, en particulier vis-à-vis des shampooings.

Or, la demanderesse vient maintenant de découvrir qu'il est possible d'obtenir de nouvelles compositions pour la teinture des fibres kératiniques capables de conduire à des colorations puissantes, peu sélectives et résistant bien aux diverses agressions que peuvent subir les cheveux, en associant au moins un colorant direct cationique, et au moins un colorant auto-oxydable benzénique ou indolique.

Cette découverte est à la base de la présente invention.

L'invention a donc pour premier objet une composition prête à l'emploi, pour la teinture des fibres kératiniques et en particulier des fibres kéraliniques humaines telles que les cheveux, caractérisée par le fait qu'elle comprend, dans un milieu approprié pour la teinture :
- au moins un colorant direct cationique,
- au moins un colorant auto-oxydable benzénique ou indolique.

La composition tinctoriale prête à l'emploi conforme à l'invention conduit à des colorations puissantes, chromatiques, présentant une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture des fibres kératiniques mettant en oeuvre cette composition tinctoriale prête à l'emploi.
Le ou les colorants directs cationiques pouvant être utilisés dans la composition tinctoriale prête à l'emploi conforme à l'invention sont de préférence choisis parmi les amino-anthraquinoniques cationiques, les mono- ou di-azoïques cationiques, les naphtoquinones cationiques.

A titre de d'exemple, on peut notamment citer le chlorure de [8-[(p-aminophényl)azo]-7-hydroxy-2-naphtyl]triméthylammonium (également appelé Basic Brown 16 ou Arianor Mahogany 306002 dans le Color Index), le chlorure de 3-[(4-amino-6-bromo-5,8-dihydro-1-hydroxy-8-imino-5-oxo-2-naphtalényl)-amino]-N,N,N-triméthyl-benzénaminium (également dénommé Basic Blue 99 ou Arianor Steel Blue 306004 dans le Color Index), le chlorure de 7-hydroxy-8-[(2-méthoxyphényl)azo]-N,N,N-triméthyl-2-naphtalènaminium (également appelé le Basic Red 76 ou Arianor Madder Red dans le Color Index), le chlorure de [8-[(4-amino-2-nitrophényl)azo]-7-hydroxy-2-naphtyl]triméthyl-ammonium (également appelé Basic Brown 17 ou Arianor Sienna Brown 306001 dans le Color Index) et le chlorure de 3-[(4,5-dihydro-3-méthyl-5-oxo-1-phényl-1H-pyrazol-4-yl)azo]-N,N,N-triméthyl-benzènaminium (également appelé Basic Yellow 57 ou Arianor Straw Yellow 306005 dans le Color Index).

Le ou les colorants directs cationiques peuvent également être choisis parmi :
a) les composés de formule (I) suivante : 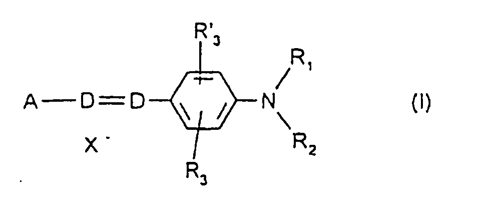 dans laquelle :
   D représente un atome d'azote ou le groupement -CH,
   R₁ et R₂, identiques ou différents, représentent un atome d'hydrogène ; un radical alkyle en C₁-C₄ pouvant être substitué par un radical -CN, -OH ou -NH₂ ; ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
   X⁻ représente un anion de préférence choisi parmi le chlorure, le méthyl sulfate et l'acétate,
   A représente un groupement choisi par les structures A1 à A19 suivantes : 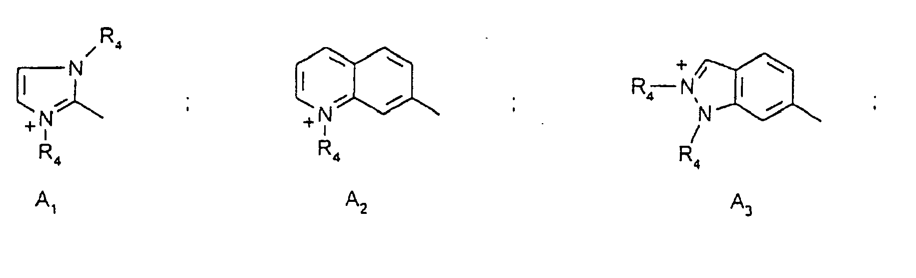 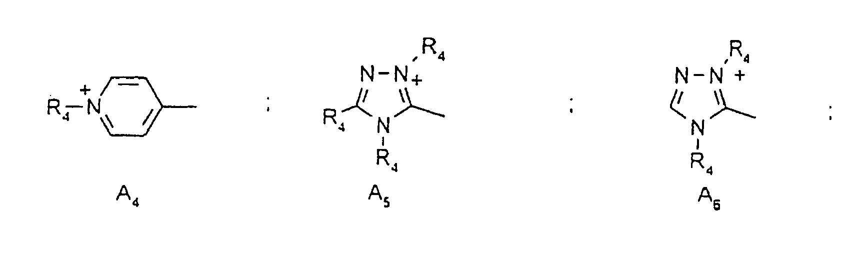 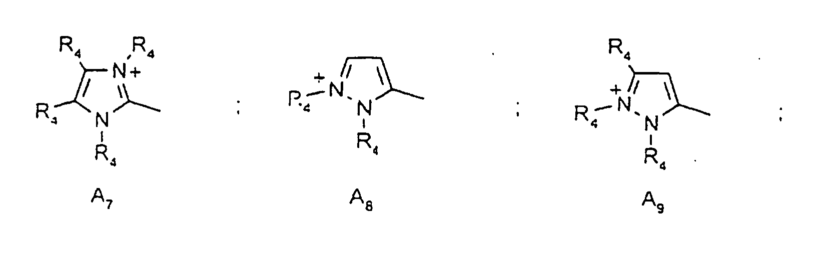 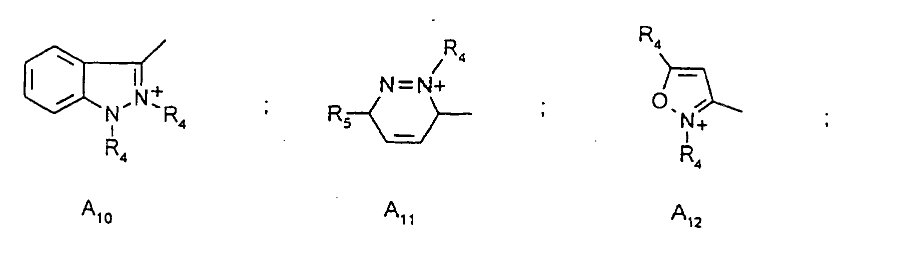 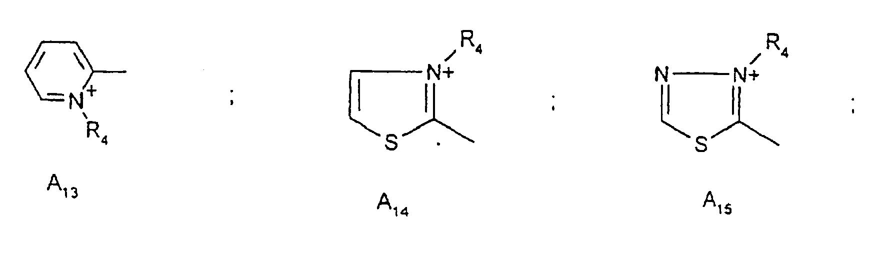 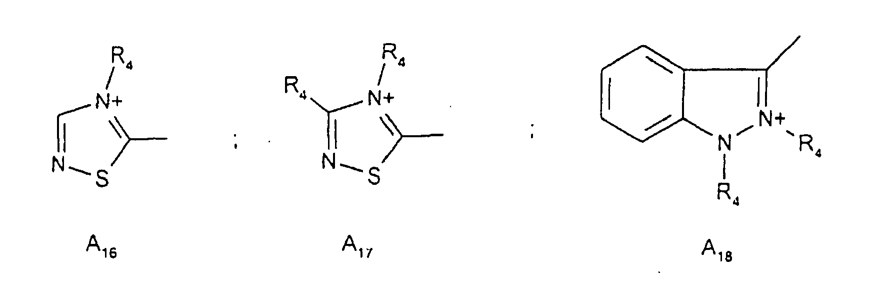 et 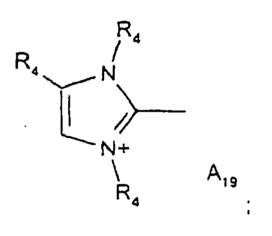 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
b) les composés de formule (II) suivante : 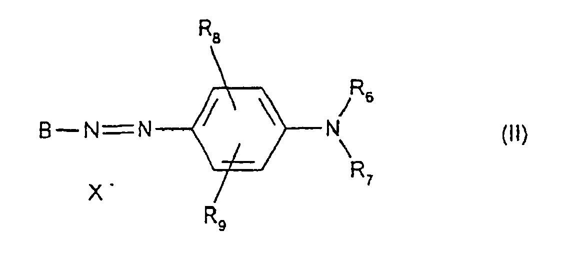 dans laquelle :
   R₅ représente un atome d'hydrogène ou un radical alkyle en C₁-C₄,
   R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
   R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
   X⁻ représente un anion de préférence choisi parmi le chlorure, le méthyl sulfate et l'acétate,
   B représente un groupement choisi par les structures B1 à B6 suivantes : 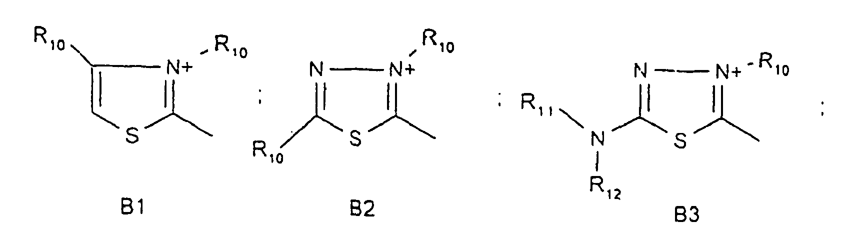 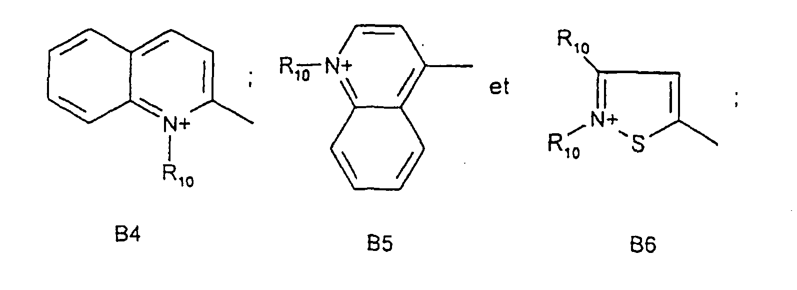 dans lesquelles R₁₀ représente un radical alkyle en C₁-C₄, R₁₁ et R₁₂, identiques ou différents, représentent un atome d'hydrogène ou un radical alkyle en C₁-C₄ ;
c) les composés de formules (III) et (III') suivantes : 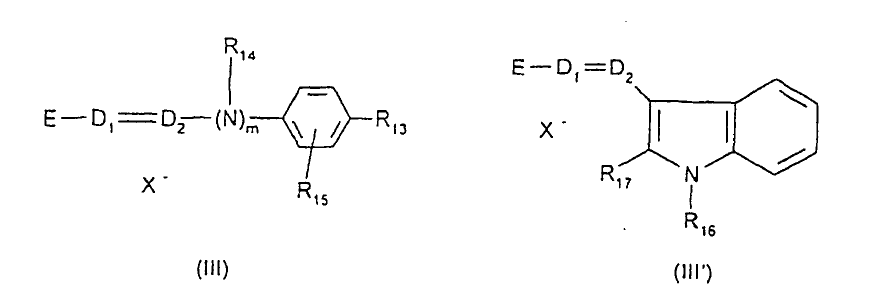 dans lesquelles :
   R₁₃ représente un atome d'hydrogène, un radical alcoxy en C₁-C₄, un atome d'halogène tel que le brome, le chlore, l'iode ou le fluor ou un radical amino,
   R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₁₅ représente un atome d'hydrogène ou d'halogène tel que le brome, le chlore, l'iode ou le fluor,
   R₁₆ et R₁₇, identiques ou différents, représentent un atome d'hydrogène ou un radical alkyle en C₁-C₄,
   D₁ et D₂, identiques ou différents, représentent un atome d'azote ou le groupement -CH,
   m = 0 ou 1,
   étant entendu que lorsque R₁₃ représente un groupement amino non substitué, alors D₁ et D₂ représentent simultanément un groupement -CH et m = 0,
   X⁻ représente un anion de préférence choisi parmi le chlorure, le méthyl sulfate et l'acétate,
   E représente un groupement choisi par les structures E1 à E8 suivantes : 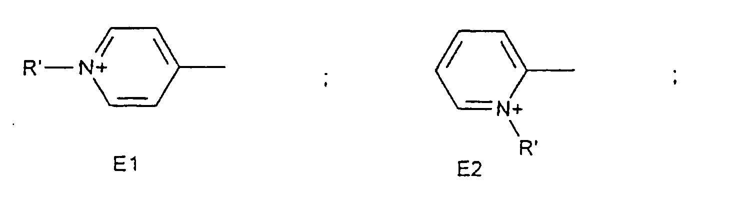 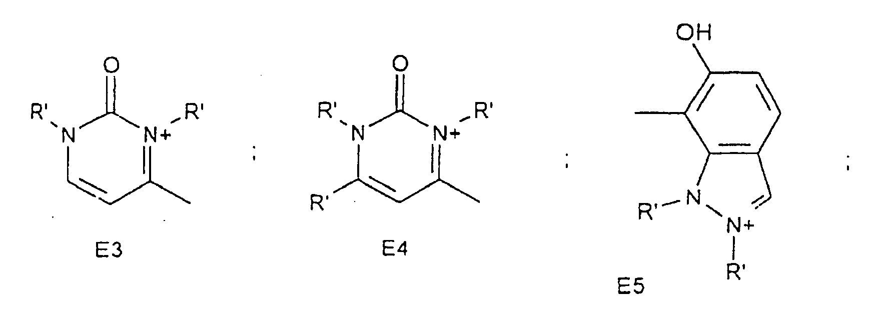 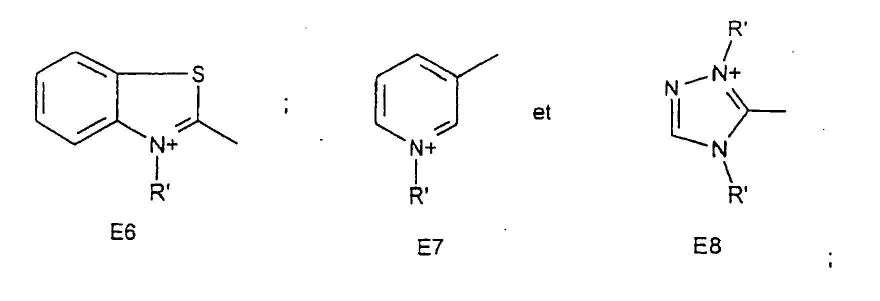 dans lesquelles R' représente un radical alkyle en C₁-C₄ ;
   lorsque m = 0 et que D₁ représente un atome d'azote, alors E peut également désigner un groupement de structure E9 suivante : 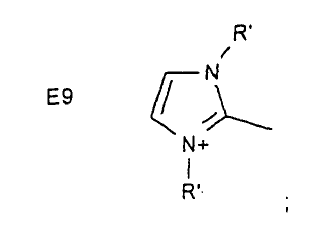 dans laquelle R' représente un radical alkyle en C₁-C₄.

Les colorants directs cationiques de formules (I), (II), (III) et (III') utilisables dans les compositions tinctoriales prêtes à l'emploi conformes à l'invention, sont des composés connus et sont décrits par exemple dans les demandes de brevets WO 95/01772, WO 95/15144 et EP-A-0 714 954.

Parmi les colorants directs cationiques de formule (I) utilisables dans les compositions tinctoriales prêtes à l'emploi conformes à l'invention, on peut plus particulièrement citer les composés répondant aux structures (I1) à (I52) suivantes : 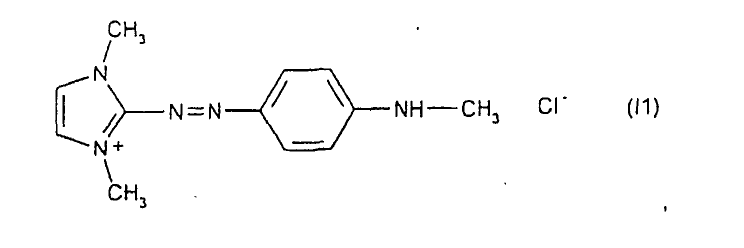 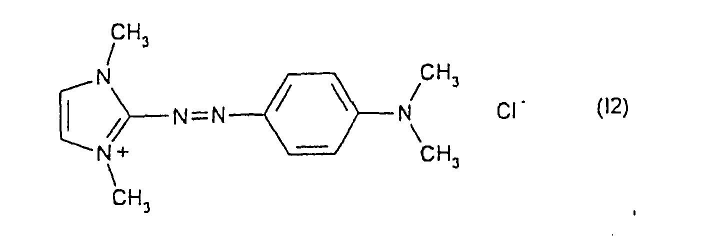 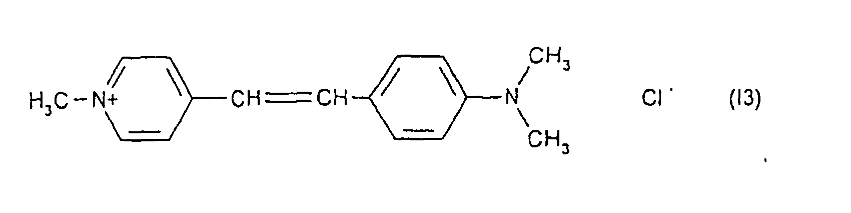 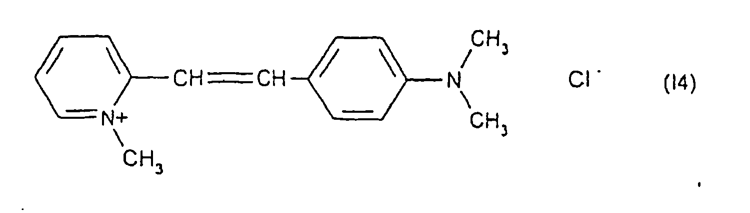 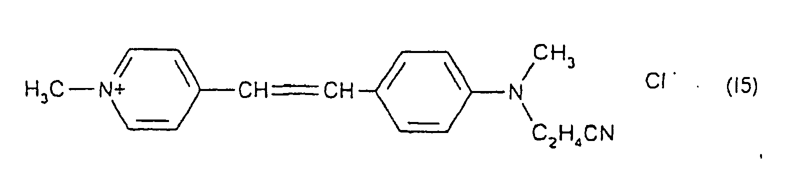 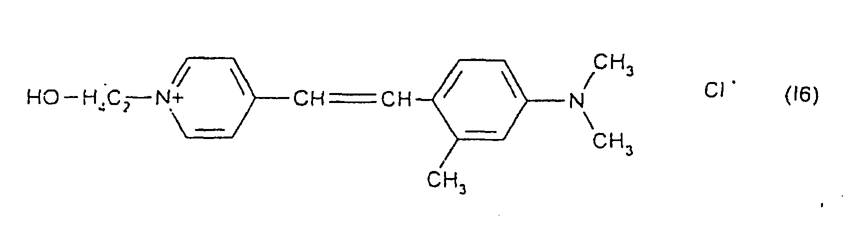 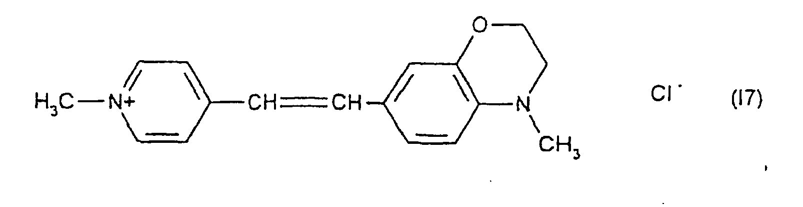 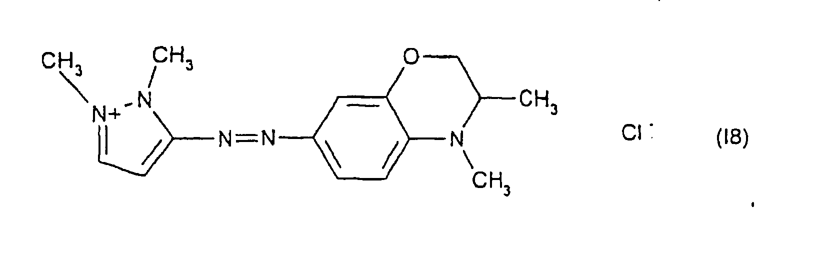 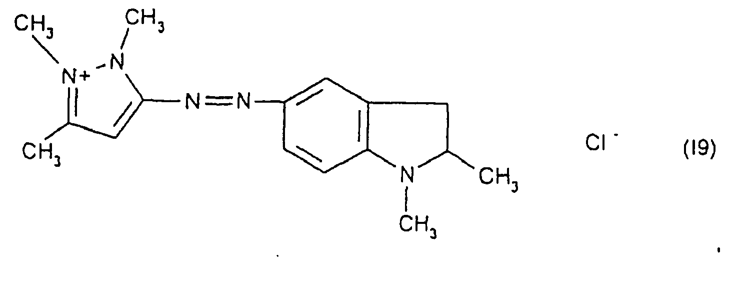 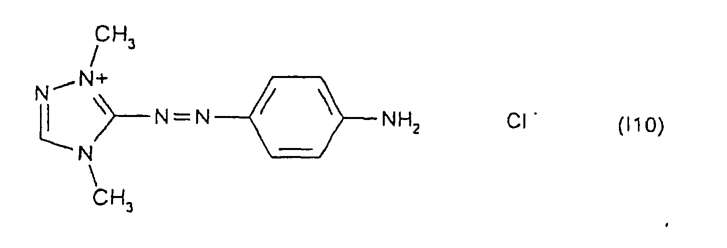 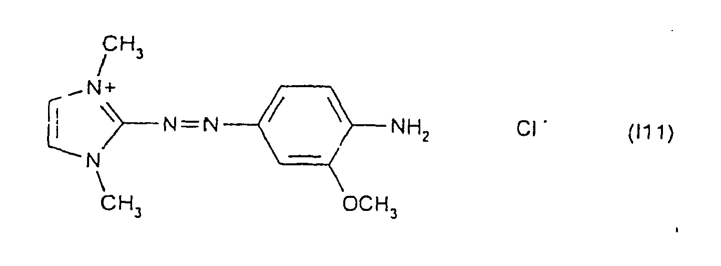 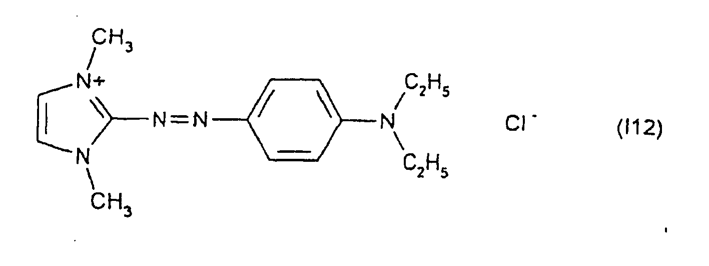 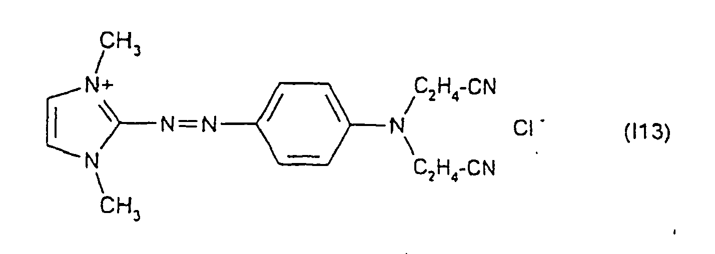 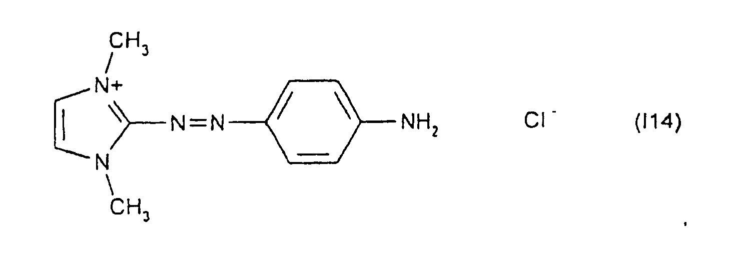 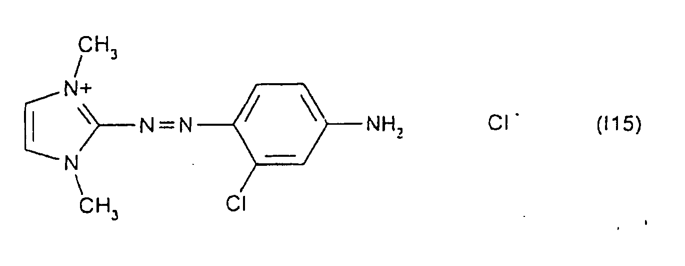 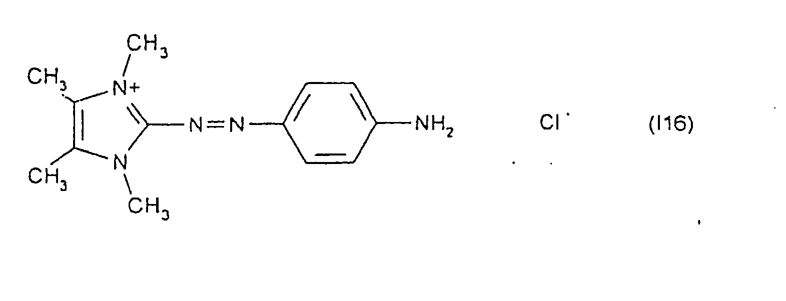 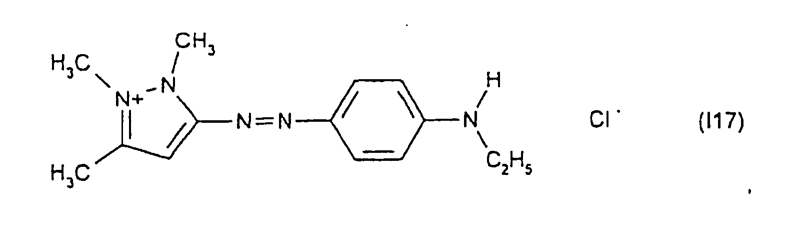 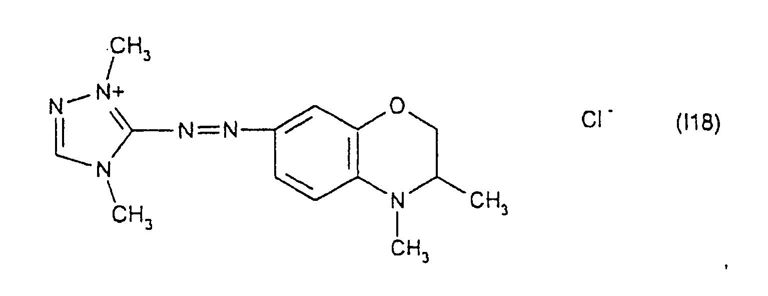 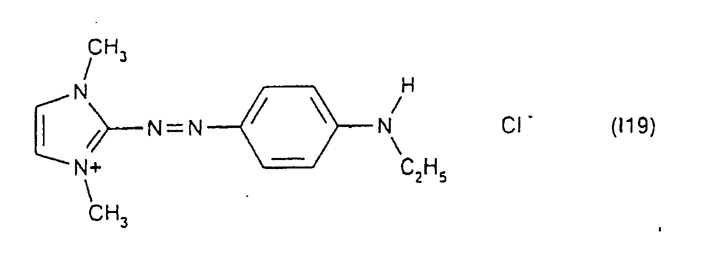 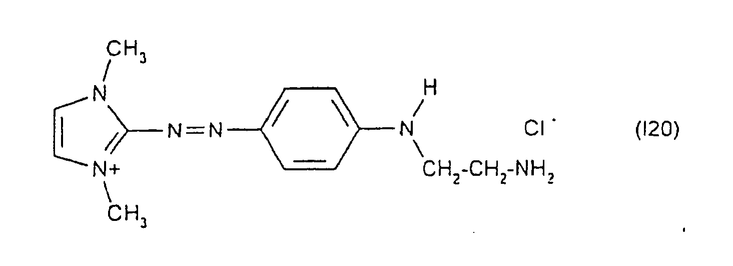 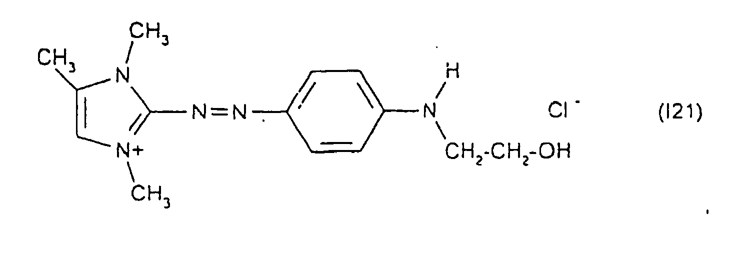 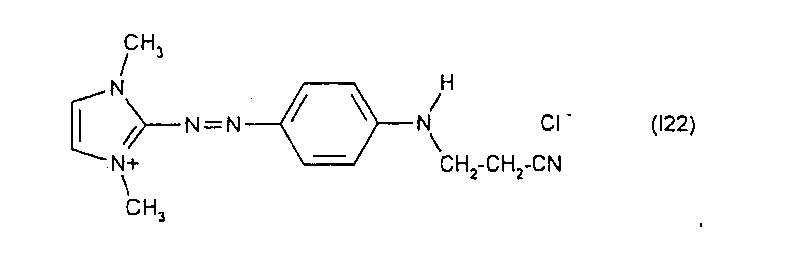 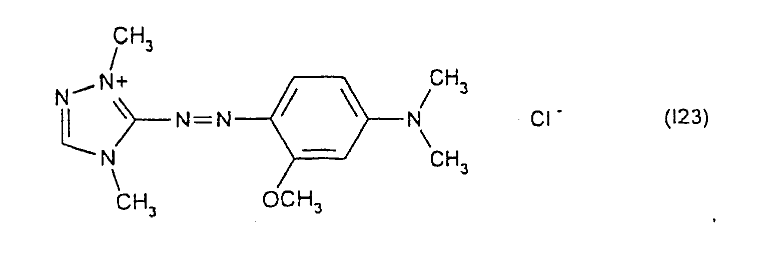 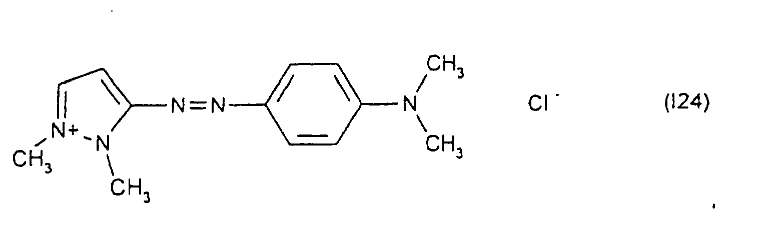 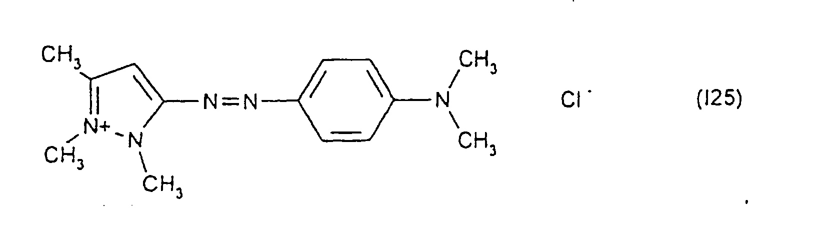 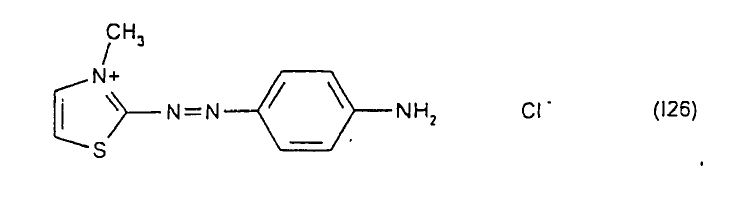 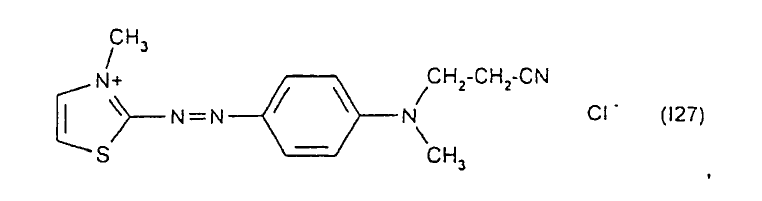 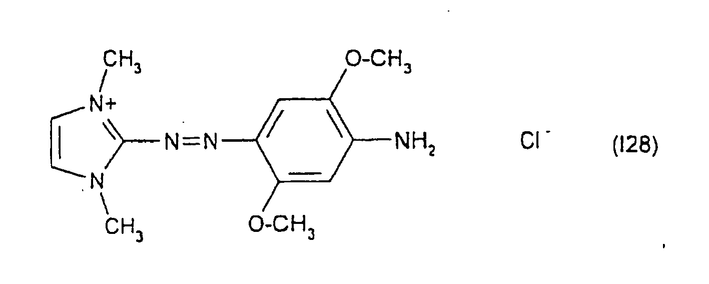 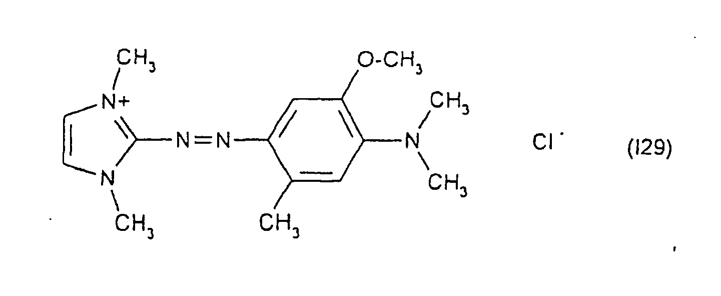 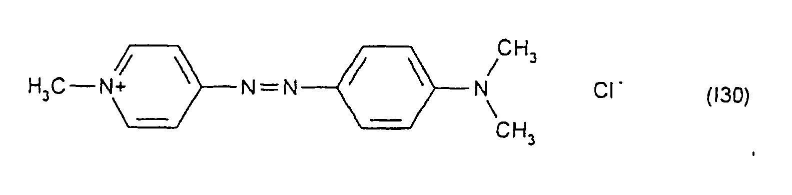 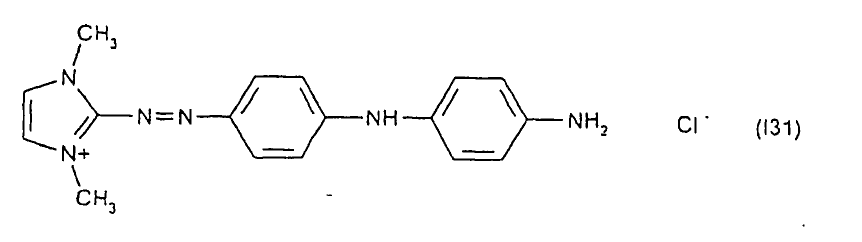 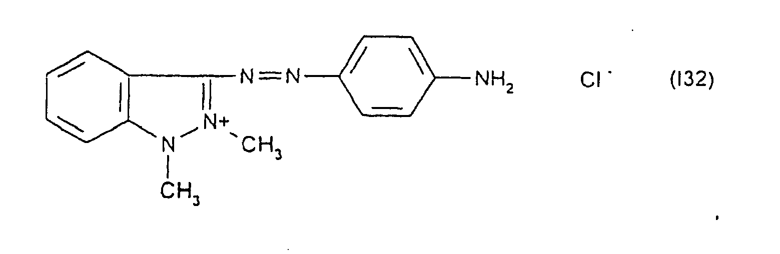 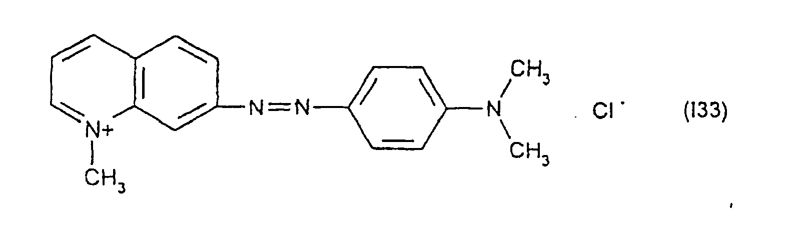 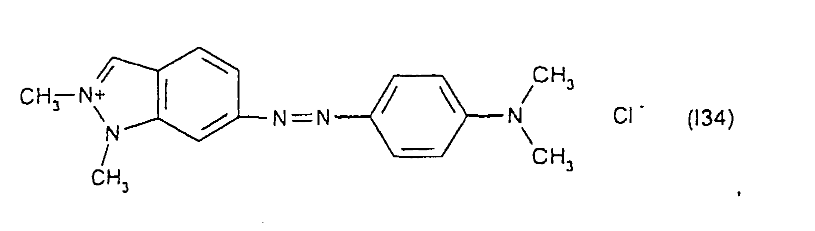 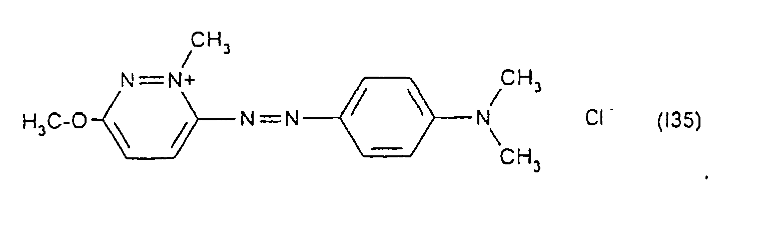 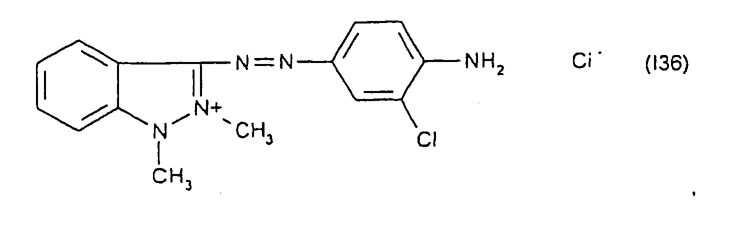 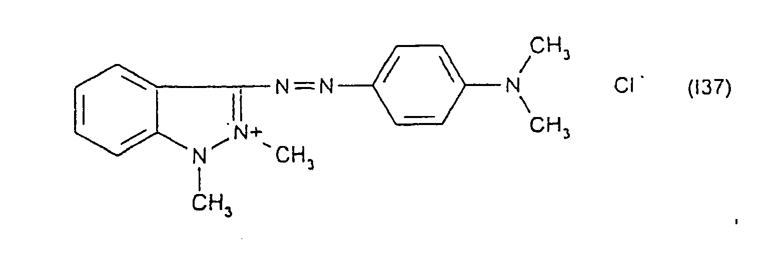 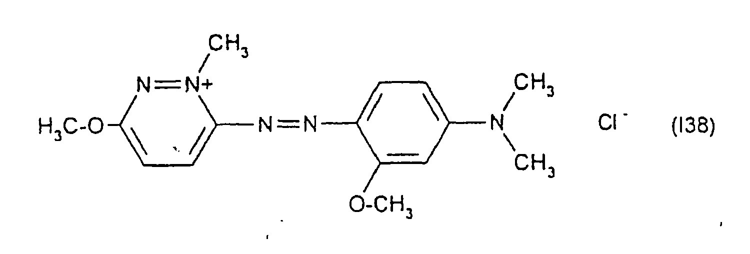 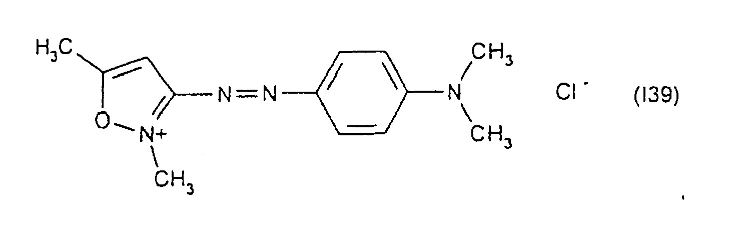 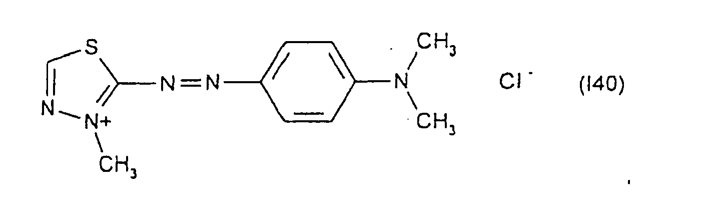 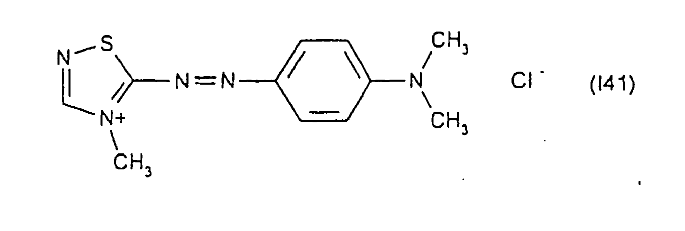 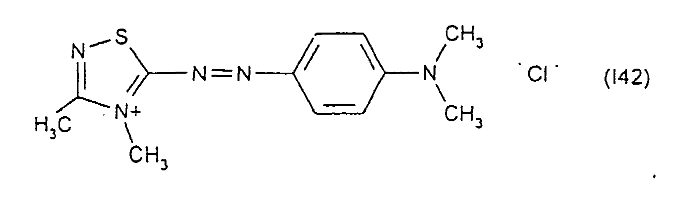 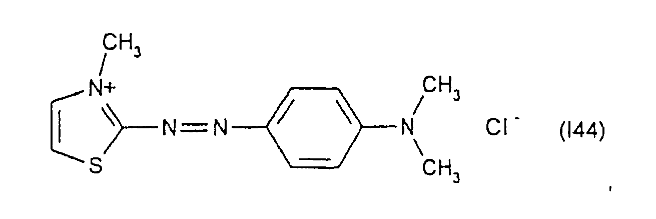 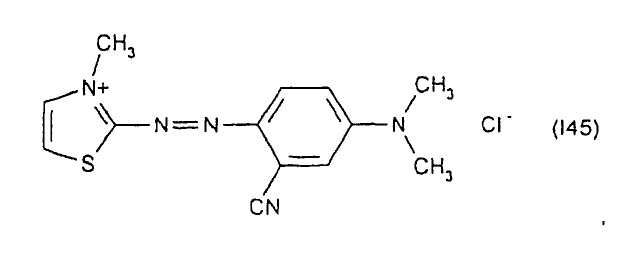 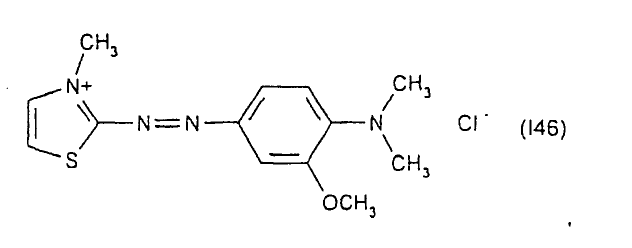 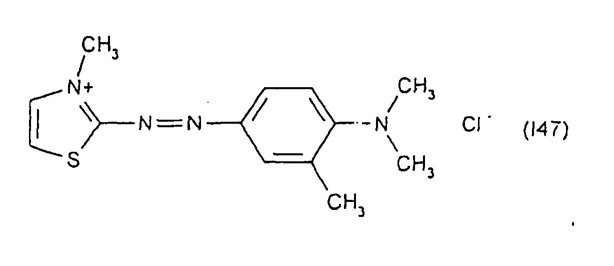 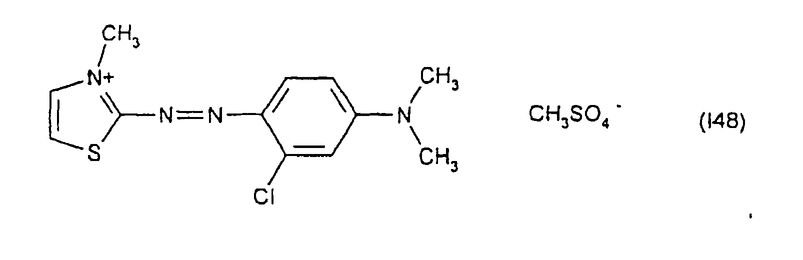 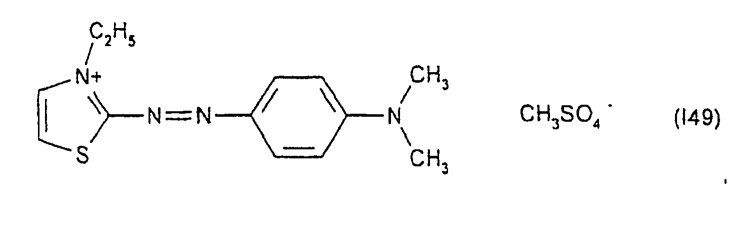 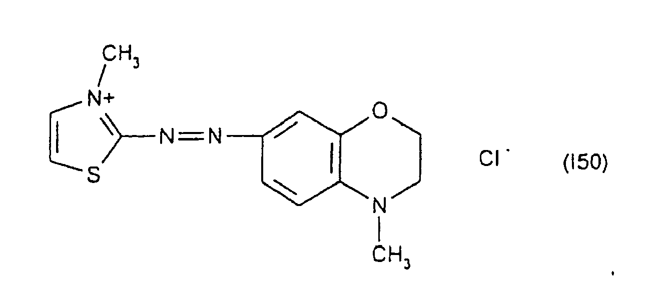 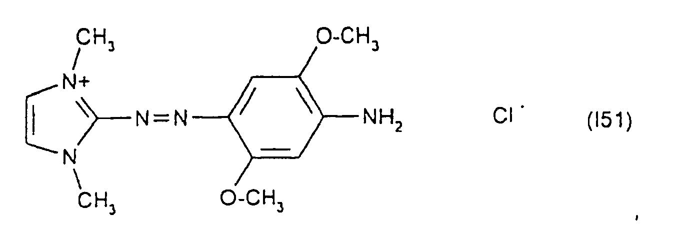 et

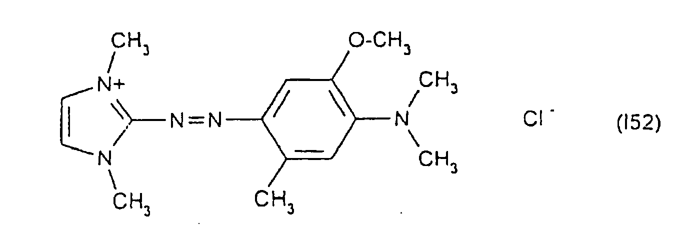

Parmi les composés de structures (I1) à (I52) décrits ci-dessus, on préfère tout particulièrement les composés répondant aux structures (I1), (I2), (I14) et (I31).

Parmi les colorants directs cationiques de formule (II) utilisables dans les compositions tinctoriales prêtes à l'emploi conformes à l'invention, on peut plus particulièrement citer les composés répondant aux structures (II1) à (II12) suivantes : 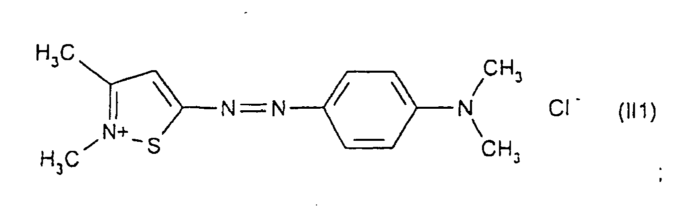 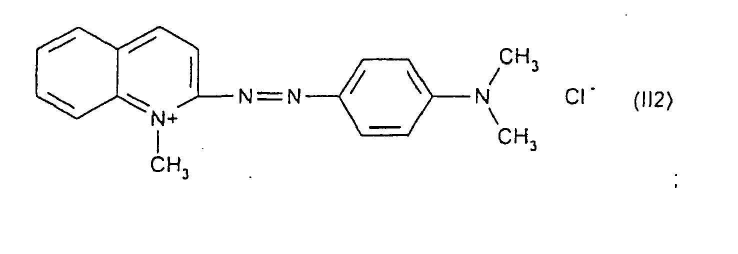 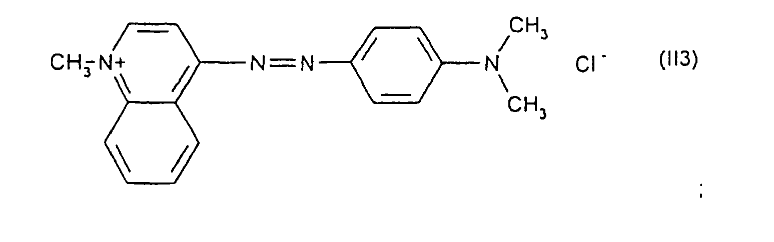 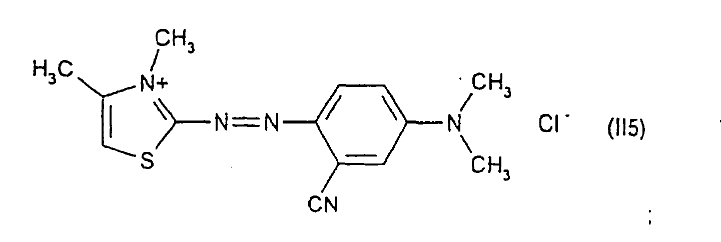 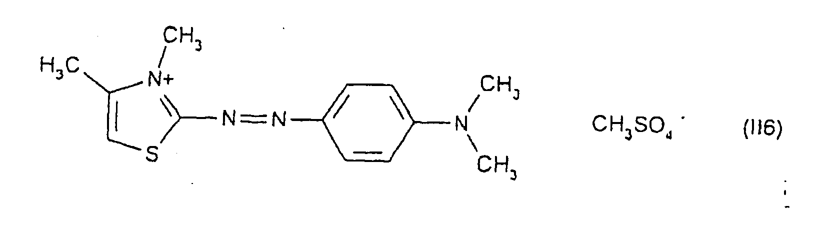 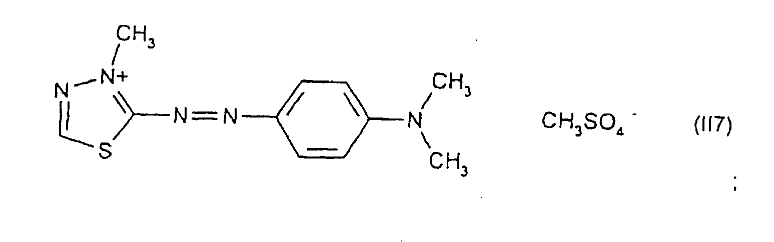 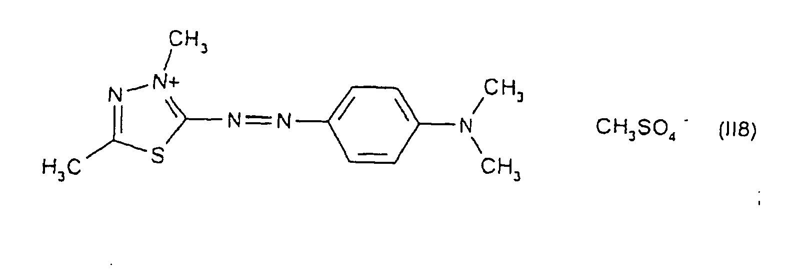 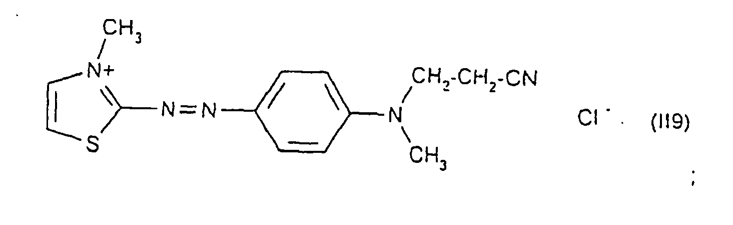 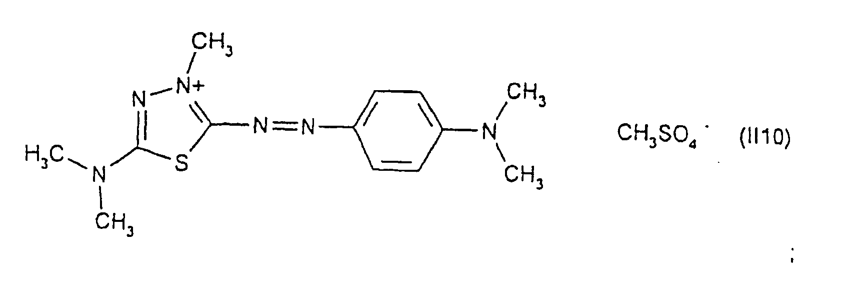 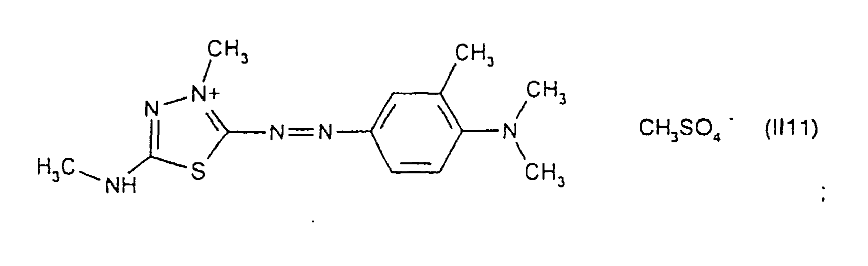 et

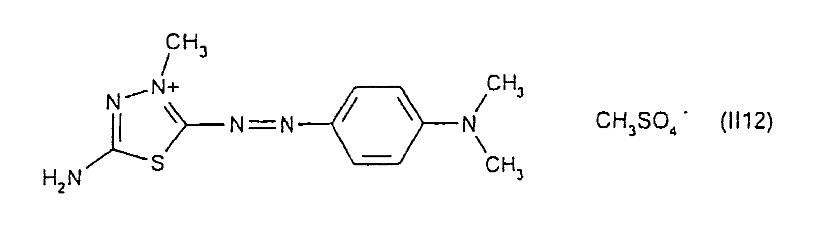

Parmi les colorants directs cationiques de formule (III), utilisables dans les compositions tinctoriales prêtes à l'emploi conformes à l'invention, on peut plus particulièrement citer les composés répondant aux structures (III1) à (III18) suivantes : 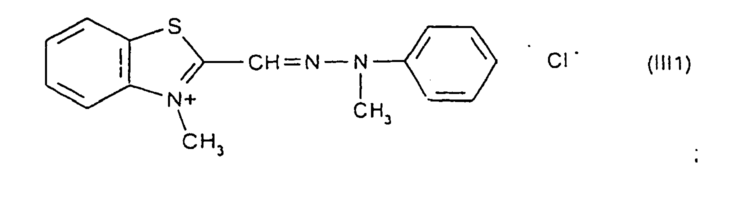 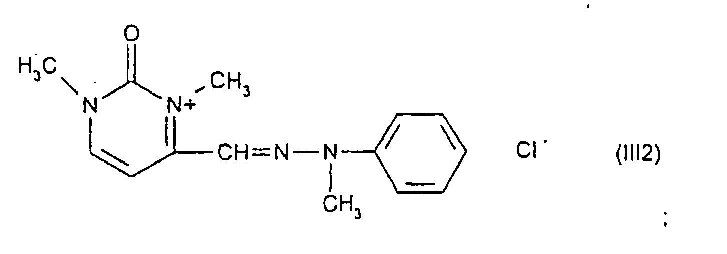 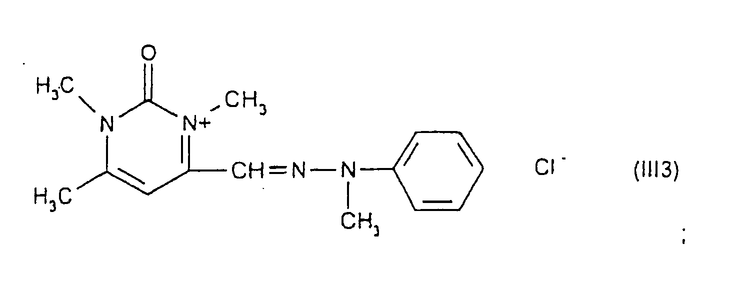 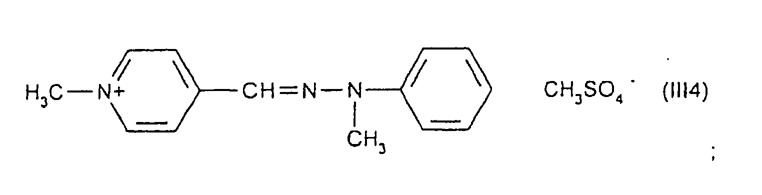 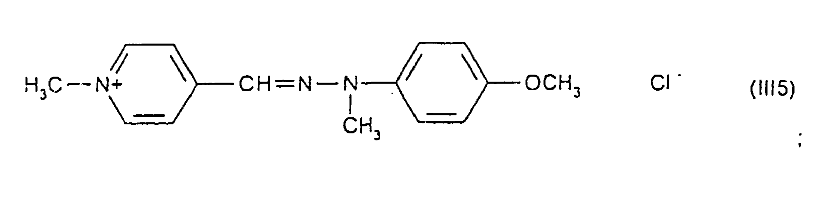 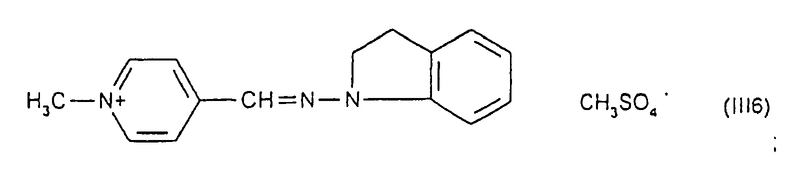 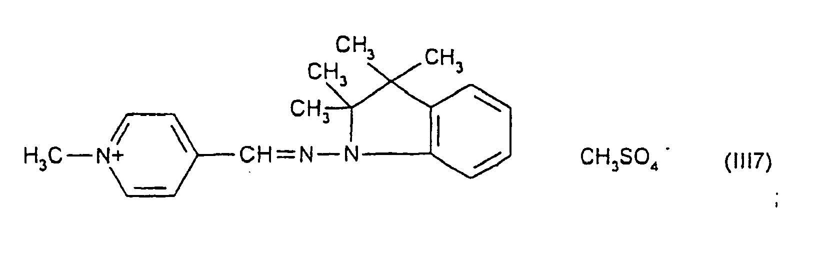 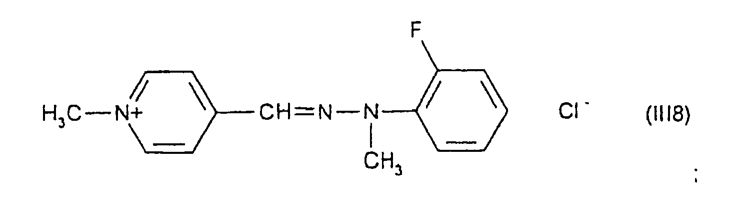 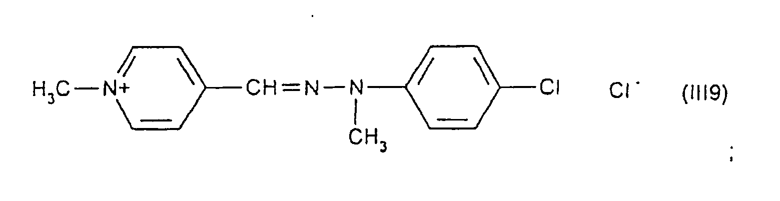 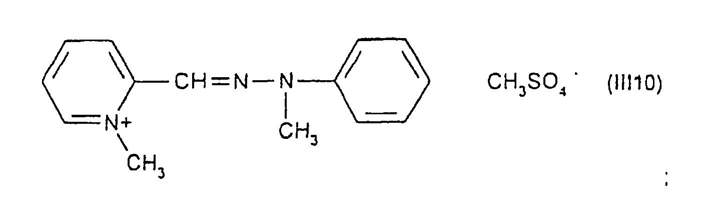 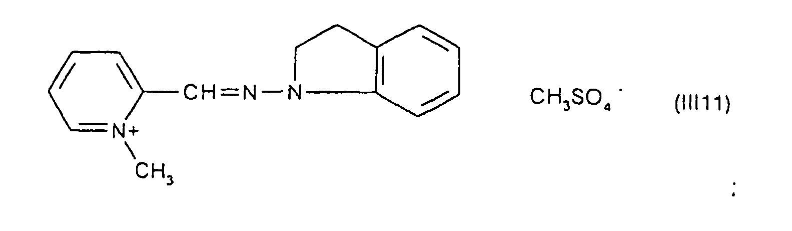 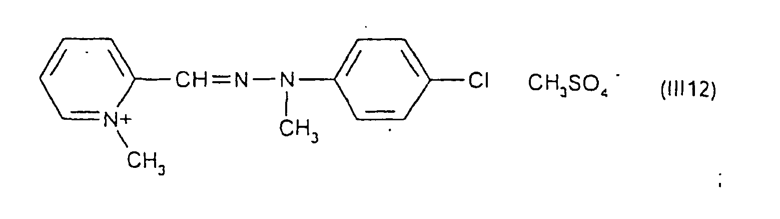 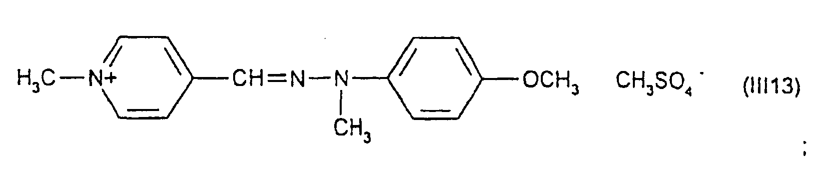 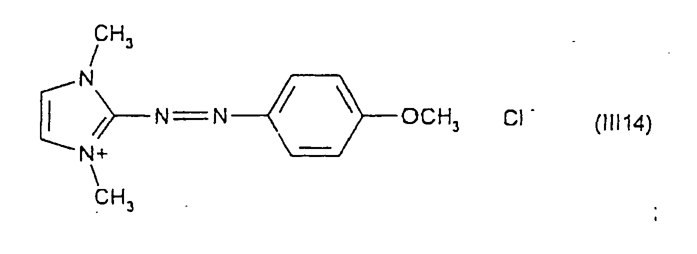 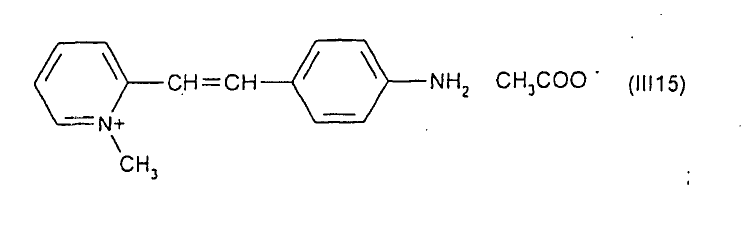 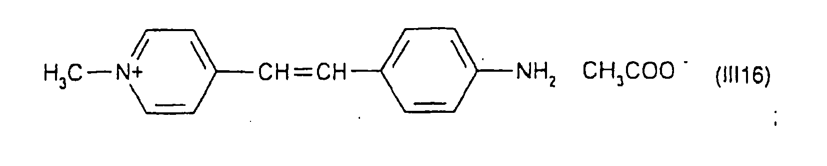 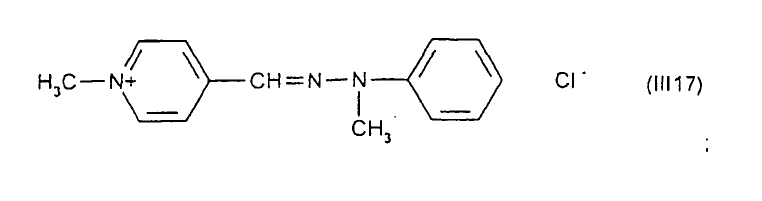 et

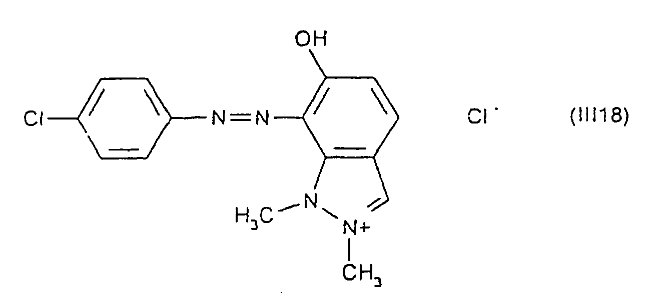

Parmi les composés particuliers de structures (III1) à (III18) décrits ci-dessus, on préfère tout particulièrement les composés répondant aux structures (III4), (III5) et (III13).

Parmi les colorants directs cationiques de formule (III'), utilisables dans les compositions tinctoriales prêtes à l'emploi conformes à l'invention, on peut plus particulièrement citer les composés répondant aux structures (III'1) à (III'3) suivantes : 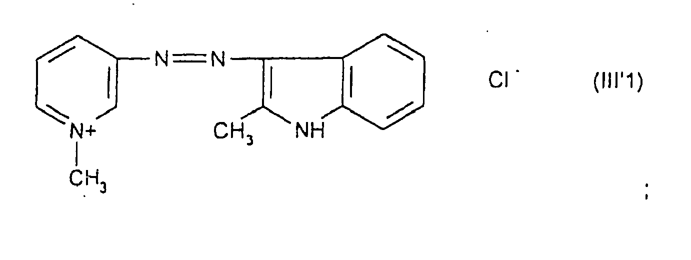 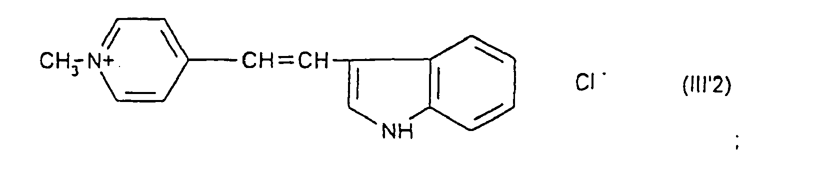 et

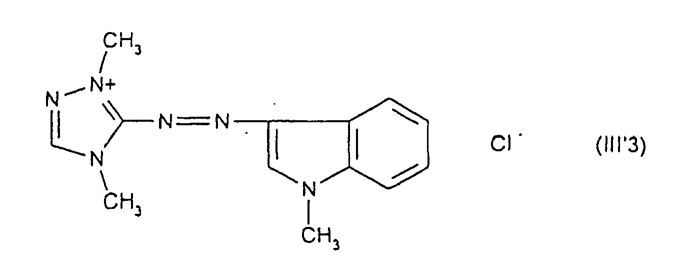

Le colorant direct cationique suivant 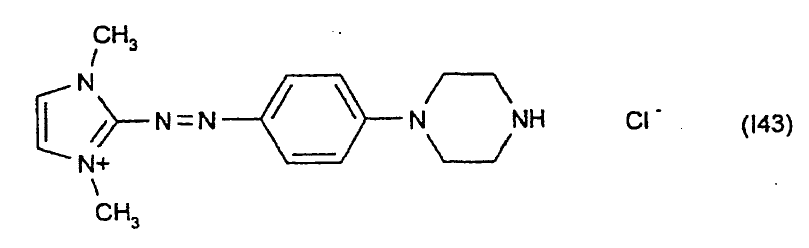 peut également être utilisé.

Le ou les colorants directs cationiques utilisés selon l'invention, représentent de préférence de 0,001 à 10 % en poids environ du poids total de la composition tinctoriale prête à l'emploi et encore plus préférentiellement de 0,05 à 5 % en poids environ de ce poids.

La nature du ou des colorants auto-oxydables utilisés dans la composition tinctoriale prête à l'emploi n'est pas critique.

Parmi les colorants auto-oxydables benzéniques utilisables dans la composition tinctoriale conforme à l'invention, on peut notamment citer les composés de formule (IV) suivante et leurs sels d'addition avec un acide : 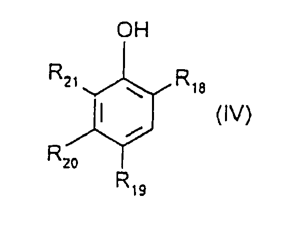 dans laquelle :
- R₁₈ représente un atome d'hydrogène, un radical alkyle en C₁-C₄ ou un radical amino,
- R₁₉ représente un radical alkyle en C₁-C₄, hydroxyle, amino, monoalkyl(C₁-C₄)amino ou dialkyl(C₁-C₄)amino,
- R₂₀ représente un atome d'hydrogène, un radical hydroxyle ou amino,
- R₂₁ représente un atome d'hydrogène ou un radical amino ;
étant entendu qu'au moins deux des radicaux R₁₉ à R₂₁ représentent, indépendamment l'un de l'autre, un radical hydroxyle, amino, monoalkyl(C₁-C₄)amino ou dialkyl(C₁-C₄)amino.

Parmi les colorants auto-oxydables benzéniques de formule (IV) ci-dessus, on peut plus particulièrement citer le 1,2,4-trihydroxybenzène, le 1-méthyl 2,4,5-trihydroxybenzène, le 2,4-diamino 6-méthyl phénol, le 2-amino 4-méthylamino phénol, le 2,5-diamino 4-méthyl phénol, le 2,6-diamino 4-diéthylamino phénol, le 2,6-diamino 1,4-dihydroxy benzène, et leurs sels d'addition avec un acide.

Parmi les colorants auto-oxydables indoliques utilisables dans la composition tinctoriale conforme à l'invention, on peut notamment citer les composés de formule (V) suivante : 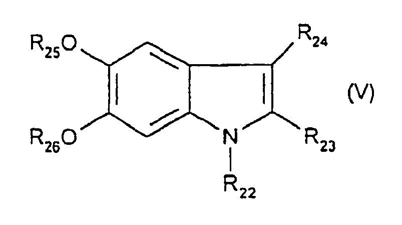 dans laquelle :
- R₂₂, R₂₄, R₂₅ et R₂₆, identiques ou différents, représentent un atome d'hydrogène, un radical alkyle en C₁-C₄ ou acyle en C₁-C₄.
- R₂₃ représente un atome d'hydrogène, un radical alkyle en C₁-C₄ ou un radical carboxyle.

Parmi les colorants auto-oxydables de formule (V) ci-dessus, on peut plus particulièrement citer le 5,6-dihydroxy indole, le 2-méthyl 5,6-dihydroxy indole, le 3-méthyl 5,6-dihydroxy indole, le 1-méthyl 5,6-dihydroxy indole, le 2,3-diméthyl 5,6-dihydroxy indole, le 5-méthoxy 6-hydroxyindole, le 5-acétoxy 6-hydroxy indole, le 5,6-diacétoxy indole, l'acide 5,6-dihydroxy indole 2-carboxylique, et leurs sels d'addition avec un acide.

Le ou les colorants auto-oxydables représentent de préférence de 0,0005 à 12 % en poids environ du poids total de la composition tinctoriale conforme à l'invention, et encore plus préférentiellement de 0,005 à 8 % en poids environ de ce poids.

Afin de faciliter l'oxydation des colorants auto-oxydables, la composition tinctoriale prête à l'emploi conforme à l'invention peut en outre renfermer un ou plusieurs agents oxydants. Ces agents oxydants peuvent notamment être choisis parmi le peroxyde d'hydrogène, le peroxyde d'urée, les bromates de métaux alcalins, les persels tels que les perborates et persulfates, et les enzymes telles que les peroxydases et les oxydo-réductases à deux électrons.

Parmi les oxydo-réductases à 2 électrons pouvant être utilisées à titre d'agent oxydant dans la composition tinctoriale prête à l'emploi conforme à l'invention, on peut plus particulièrement citer les pyranose oxydases, les glucose oxydases, les glycérol oxydases, les lactates oxydases, les pyruvate oxydases, et les uricases.

Selon l'invention, l'utilisation des uricases d'origine animale, microbiologique ou biotechnologique est particulièrement préférée.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

Lorsqu'elles sont utilisées, la ou les oxydo-réductases à 2 électrons conformes à l'invention représentent de préférence de 0,01 à 20 % en poids environ du poids total de la composition tinctoriale prête à l'emploi, et encore plus préférentiellement de 0,1 à 5 % en poids environ de ce poids.

Lorsqu'une enzyme de type oxydo-réductase à 2 électrons est utilisée conformément à l'invention, la composition tinctoriale prête à l'emploi peut en outre renfermer un ou plusieurs donneurs.

Selon l'invention, on entend par donneur, les différents substrats participant au fonctionnement de ladite ou desdites oxydo-réductases à 2 électrons.

La nature du donneur (ou substrat) utilisée varie en fonction de la nature de l'oxydo-réductase à 2 électrons qui est utilisée. Par exemple, à titre de donneur pour les pyranose oxydases, on peut citer le D-glucose, le L-sorbose et le D-xylose ; à titre de donneur pour les glucose oxydases, on peut citer le D-glucose, à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; et enfin à titre de donneur pour les uricases, on peut citer l'acide urique et ses sels.

Lorsqu'ils sont utilisés, le ou les donneurs (ou substrats) utilisés conformément à l'invention représentent de préférence de 0,01 à 20 % en poids environ du poids total de la composition tinctoriale prête à l'emploi conforme à l'invention et encore plus préférentiellement de 0,1 à 5 % en environ de ce poids.

Lorsque la composition tinctoriale prête à l'emploi conforme à l'invention renferme un agent oxydant, elle peut en outre renfermer une ou plusieurs bases d'oxydation et/ou un ou plusieurs coupleurs. Ces bases d'oxydation peuvent notamment être choisies parmi les paraphénylènediamines, les para-aminophénols, les orthophénylènediamines et les bases hétérocycliques telles que par exemple les dérivés pyridiniques, les dérivés pyrimidiniques, les dérivés pyrazoliques, et les dérivés pyrazolo-pyrimidiniques. Les coupleurs peuvent notamment être choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

Lorsqu'elles sont présentes, la ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids.

Lorsqu'ils sont présents, le ou les coupleurs représentent de préférence de 0,0001 à 10 % en poids environ du poids total de la composition tinctoriale prête à l'emploi et encore plus préférentiellement de 0,005 à 8 % en poids environ de ce poids.

D'une manière générale, les sels d'addition avec un acide utilisables dans le cadre des compositions tinctoriales de l'invention (colorants auto-oxydables, bases d'oxydation et coupleurs) sont notamment choisis parmi les chlorhydrates, les bromhydrates, les sulfates, les tartrates, les lactates et les acétates.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol , ainsi que les alcools aromatiques comme l'alcool benzylique ou le phénoxyéthanol, les produits analogues et leurs mélanges.

Le pH de la composition prête à l'emploi conforme à l'invention est généralement compris entre 5 et 11 environ, et de préférence entre 6,5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide colorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl-2-amino-1-propanol ainsi que leurs dérivés, les hydroxydes de sodium ou de potassium et les composés de formule (VII) suivante : 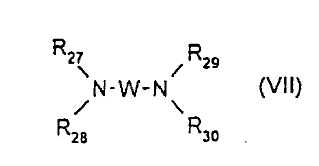 dans laquelle W est un reste propylène éventuellement substitué par un groupement hydroxyle ou un radical alkyle en C₁-C₄ ; R₂₇, R₂₈, R₂₉ et R₃₀, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que par exemple des agents antioxydants, des agents de pénétration, des agents séquestrants, des parfums, des tampons, des agents dispersants, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains. Elle doit être exempte d'oxygène gazeux, de manière à éviter toute oxydation prématurée du ou des colorants auto-oxydables.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une forme de réalisation particulière de l'invention, et lorsque la composition prête à l'emploi conforme à l'invention renferme un agent oxydant, le procédé comporte une étape préliminaire consistant à stocker sous forme séparée, d'une part, une composition (A) comprenant, dans un milieu approprié pour la teinture, au moins un colorant direct cationique, et au moins un colorant auto-oxydable benzénique ou indolique et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLES 1 et 2 DE TEINTURE

On a préparé les compositions tinctoriales prêtes à l'emploi suivantes (teneurs en grammes) :

| **COMPOSITION** | **1** | **2** |
|---|---|---|
| 5,6-dihydroxy indole (colorant auto-oxydable) | 0,5 | - |
| 1,2,4-trihydroxy benzène (colorant auto-oxydable) | - | 1,2 |
| Colorant direct cationique Basic Red 76 (Arianor Madder Red) | - | - |
| Colorant direct cationique orangé de structure (I4) | 0,07 | - |
| Colorant direct cationique rouge de structure (I1) | - | 0,05 |
| Support de teinture commun (*) | (*) | (*) |
| Eau déminéralisée q.s.p. | 100 g | 100 g |

| | | |
|---|---|---|
| (*) : Support de teinture commun : - Ethanol 20,0 g - Nonyl phénol oxyéthyléné par 9 moles d'oxyde d'éthylène vendu sous la dénomination IGEPAL NP 9 OR par la société RHODIA CHEMIE 8,0 g - 2-amino-2-méthyl-1-propanol q.s. pH = 8,0 | | |

Chacune des compositions tinctoriales prêtes à l'emploi décrites ci-dessus a été appliquée sur des mèches de cheveux gris naturels à 90 % de blancs pendant 30 minutes. Les cheveux ont ensuite été rincés, lavés avec un shampooing standard, puis séchés.

Les cheveux ont été teints dans les nuances figurant dans le tableau ci-après :

| **EXEMPLE** | **Nuance obtenue** |
|---|---|
| 1 | Blond cuivré |
| 2 | Blond acajou |

## Revendications

1. Composition prête à l'emploi, pour la teinture des fibres kératiniques et en particulier des fibres kératiniques humaines telles que les cheveux, **caractérisée par le fait qu'**elle comprend, dans un milieu approprié pour la teinture :
- au moins un colorant direct cationique,
- et au moins un colorant auto-oxydable benzénique ou indolique.

2. Composition selon la revendication 1, **caractérisée par le fait que** le ou les colorants directs cationiques sont choisis parmi les amino-anthraquinones cationiques, les mono- ou di-azoïques cationiques, les naphtoquinones cationiques.

3. Composition selon la revendication 2, **caractérisée par le fait que** le ou les colorants directs cationiques sont choisis parmi le chlorure de [8-[(p-aminophényl)azo]-7-hydroxy-2-naphtyl]triméthylammonium, le chlorure de 3-[(4-amino-6-bromo-5,8-dihydro-1-hydroxy-8-imino-5-oxo-2-naphtalényl)-amino]-N,N,N-triméthyl-benzénaminium, le chlorure de 7-hydroxy-8-[(2-méthoxyphényl)azo]-N,N,N-triméthyl-2-naphtalènaminium, le chlorure de [8-[(4-amino-2-nitrophényl)azo]-7-hydroxy-2-naphtyl]triméthylammonium et le chlorure de 3-[(4,5-dihydro-3-méthyl-5-oxo-1-phényl-1 H-pyrazol-4-yl)azo]-N,N,N-triméthylbenzènaminium.

4. Composition selon la revendication 1, **caractérisée par le fait que** le ou les colorants directs cationiques sont choisis parmi :
a) les composés de formule (I) suivante : 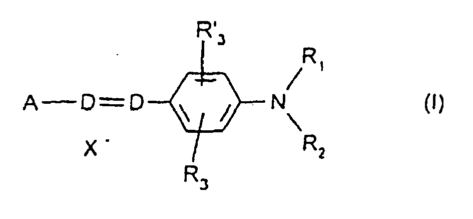 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ; ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alcoxy en C₁-C₄ ou acétyloxy,
X⁻ représente un anion de préférence choisi parmi le chlorure, le méthyl sulfate et l'acétate,
A représente un groupement choisi par les structures A1 à A18 suivantes : 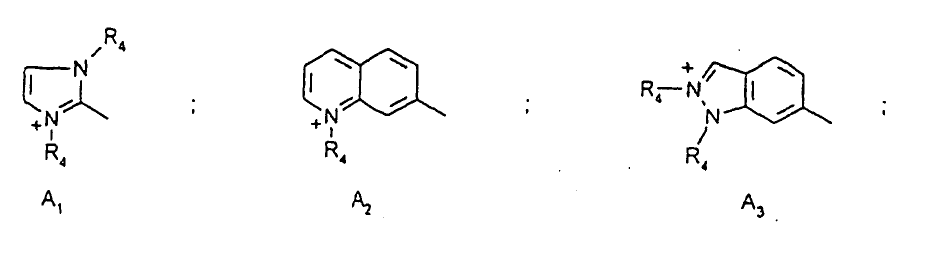 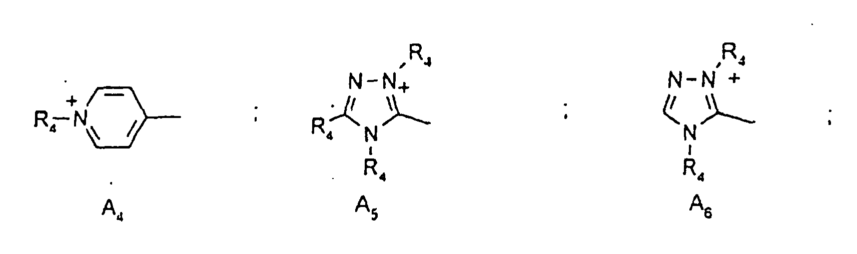 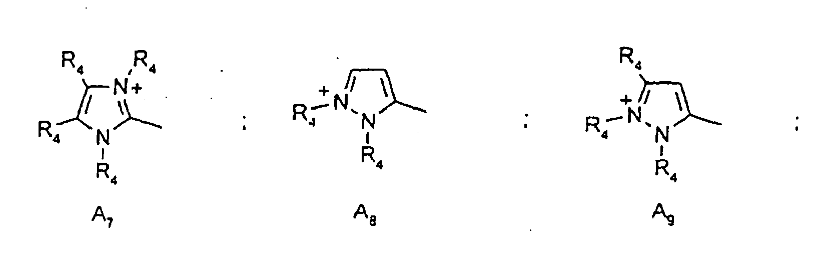 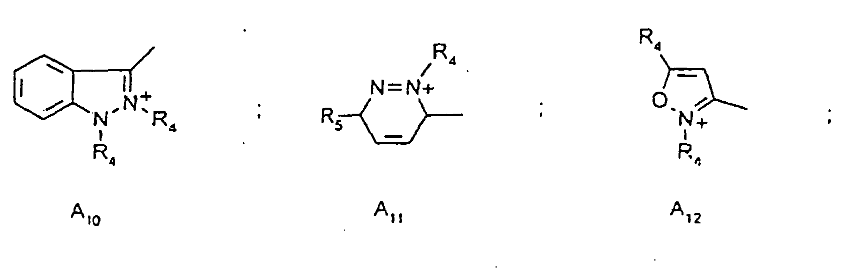 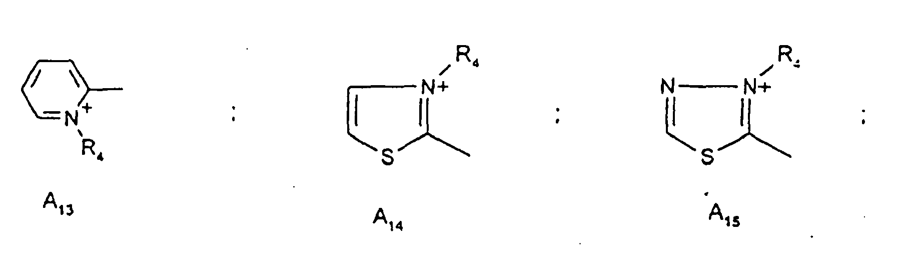 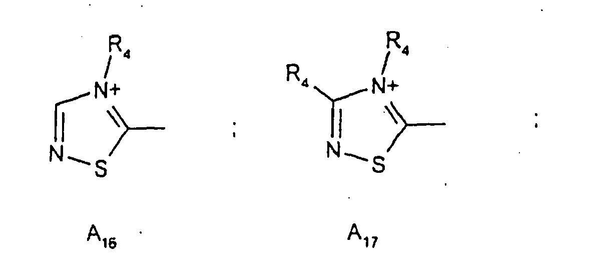 et 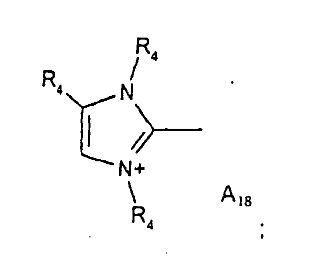 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
b) les composés de formule (II) suivante : 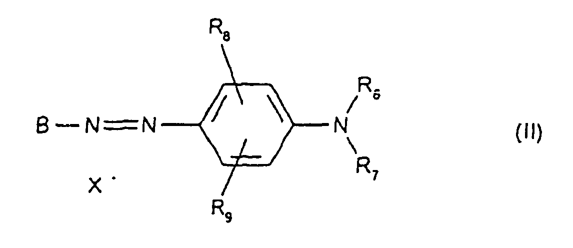 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à 86 suivantes : 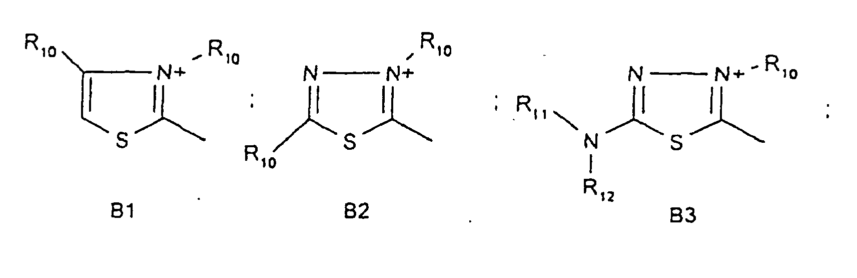 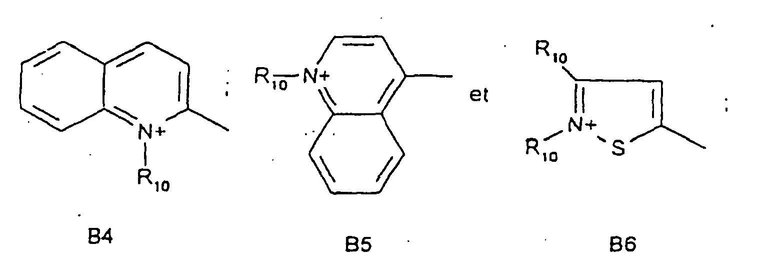 dans lesquelles R₁₀ représente un radical alkyle en C₁-C₄, R₁₁ et R₁₂, identiques ou différents, représentent un atome d'hydrogène ou un radical alkyle en C₁-C₄ ;
c) les composés de formules (III) et (III') suivantes : 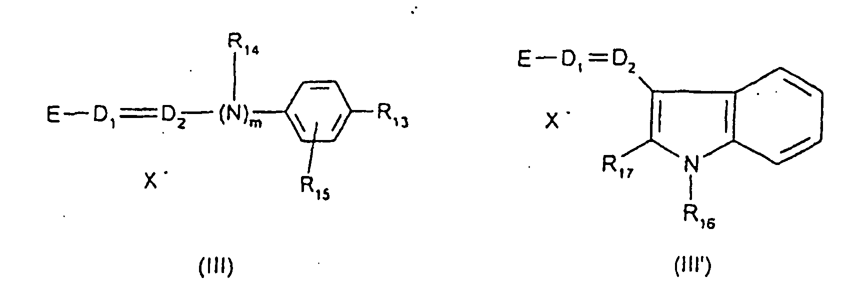 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 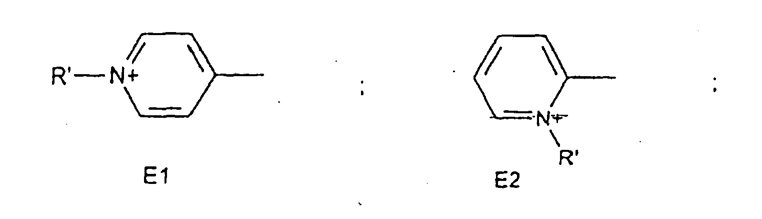 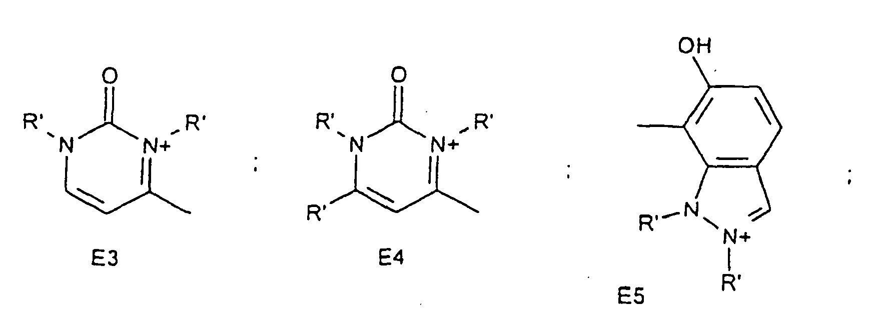 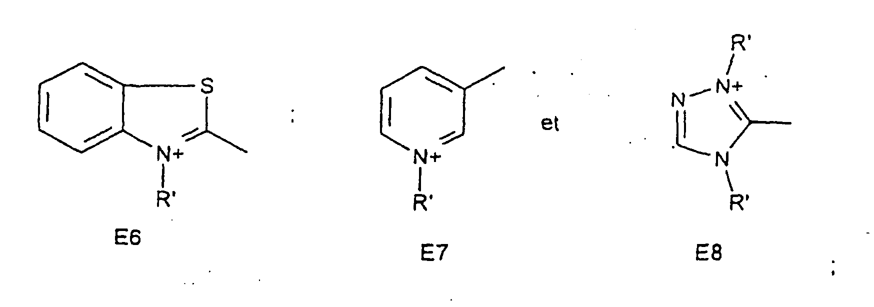 dans lesquelles R' représente un radical alkyle en C₁-C₄ ;
lorsque m = 0 et que D₁ représente un atome d'azote, alors E peut également désigner un groupement de structure E9 suivante : 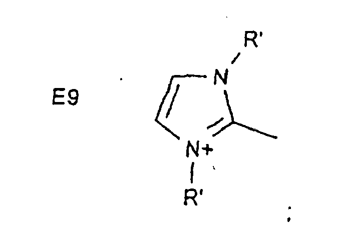 dans laquelle R' représente un radical alkyle en C₁-C₄.

5. composition selon la revendication 4, **caractérisée par le fait que** les colorants directs cationiques de formule (I) sont choisis parmi les composés répondant aux structures (I1) à (I52) suivantes : 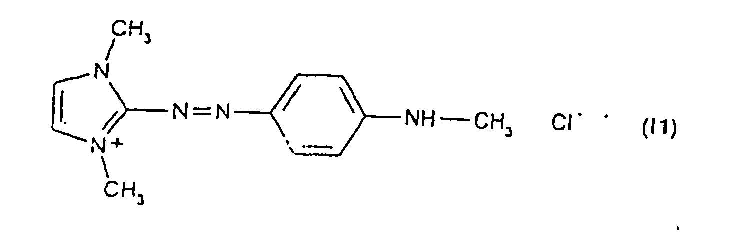 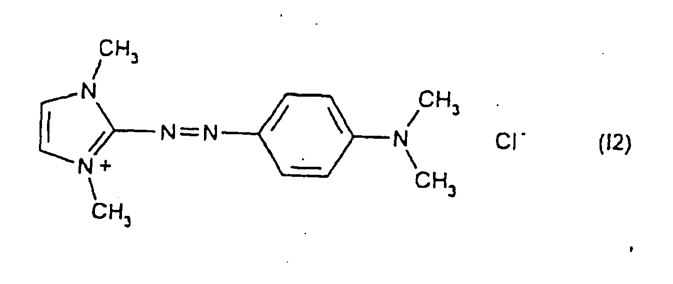 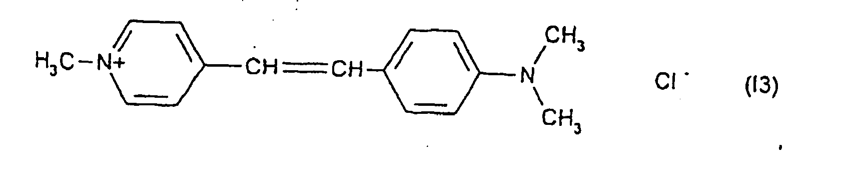 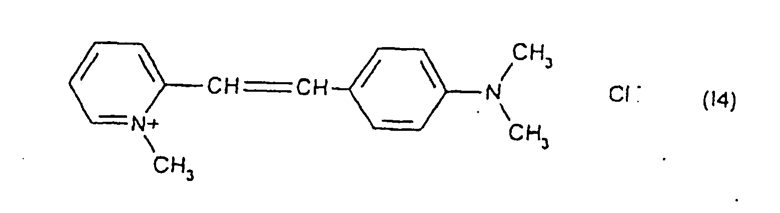 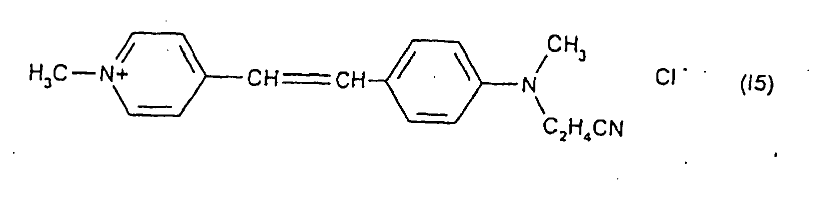 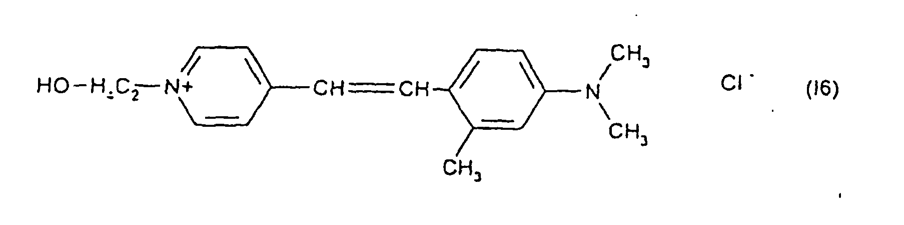 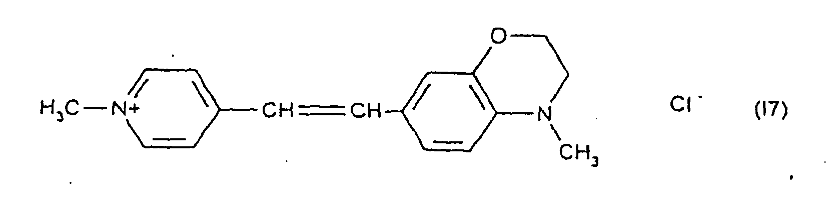 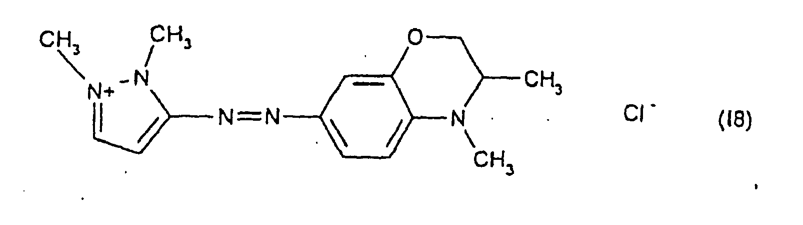 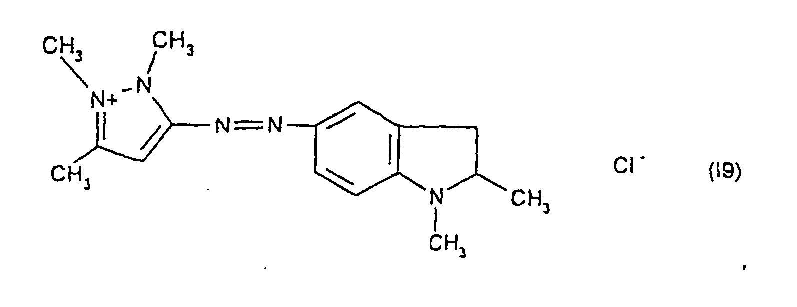 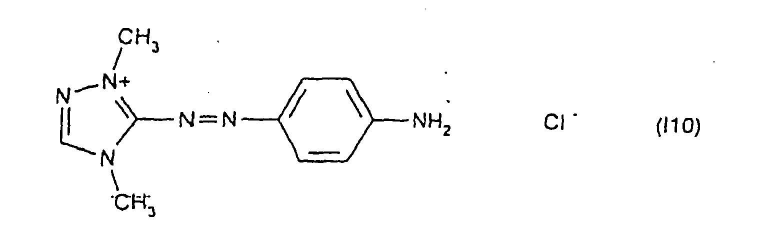 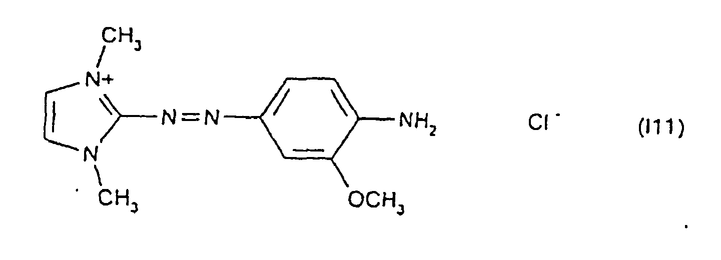 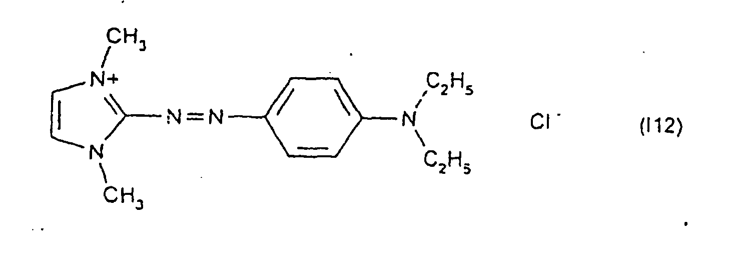 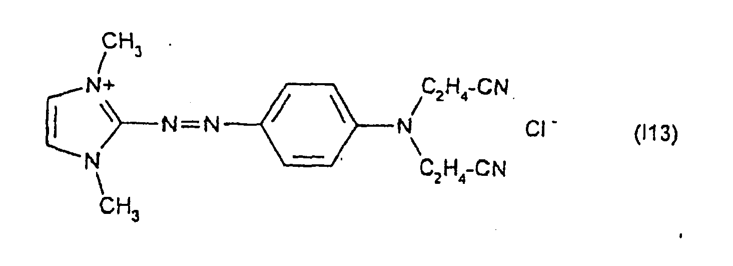 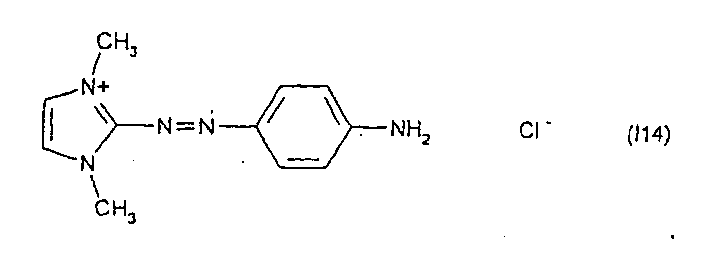 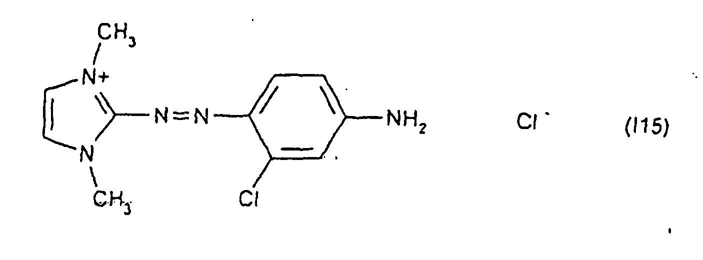 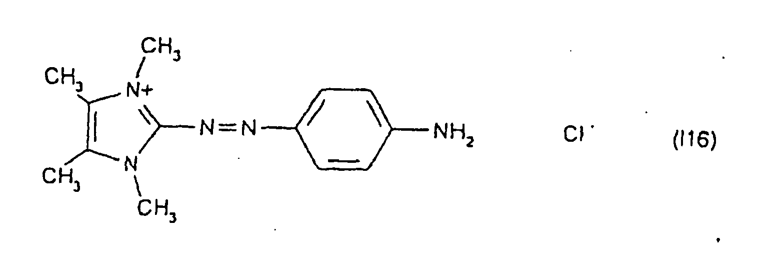 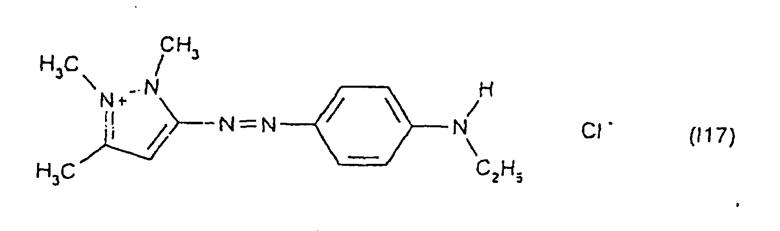 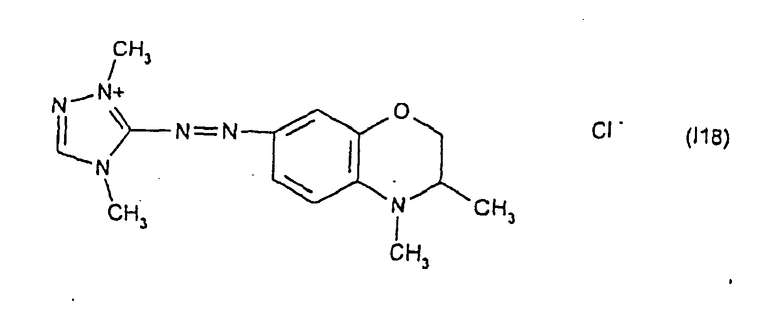 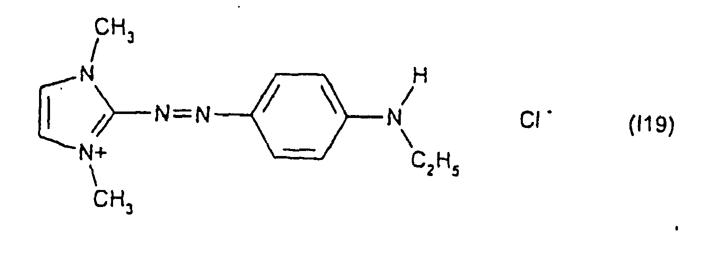 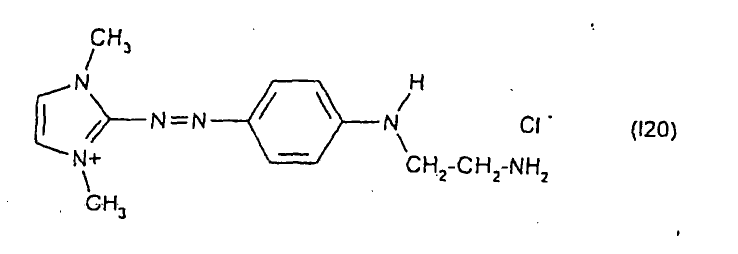 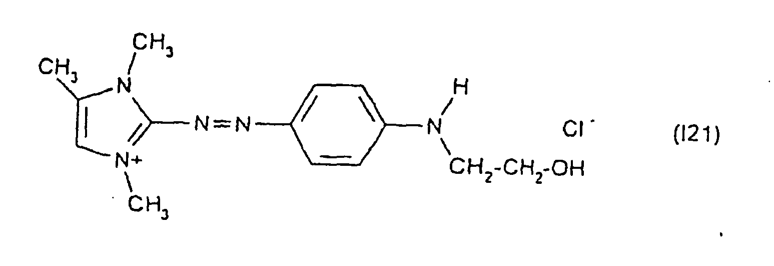 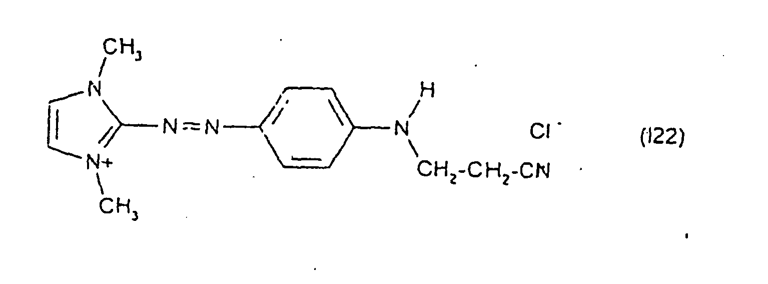 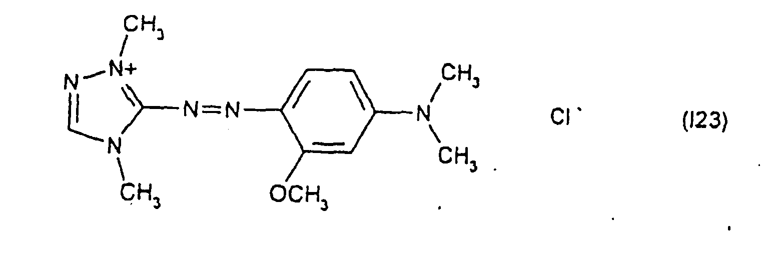 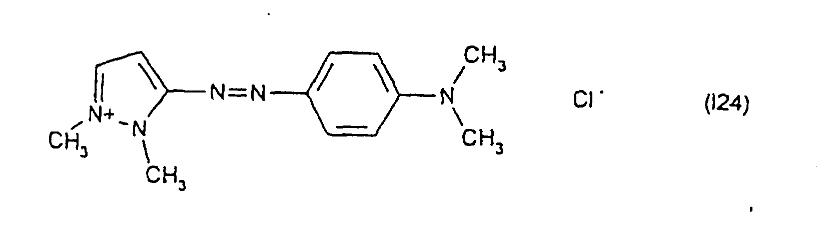 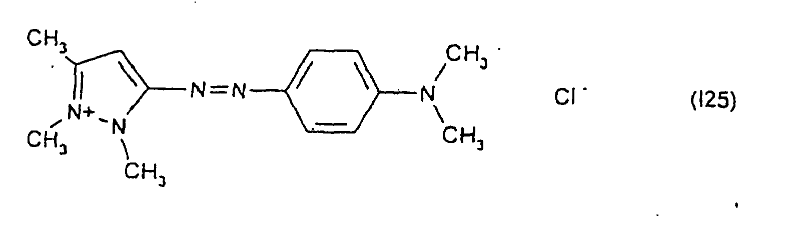 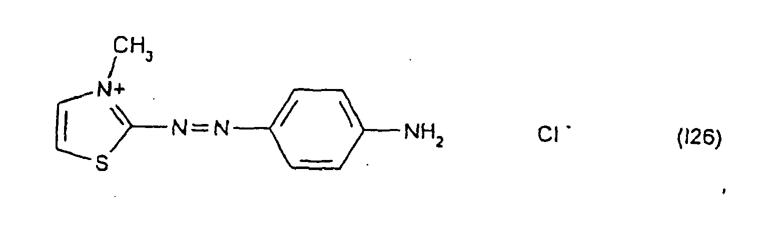 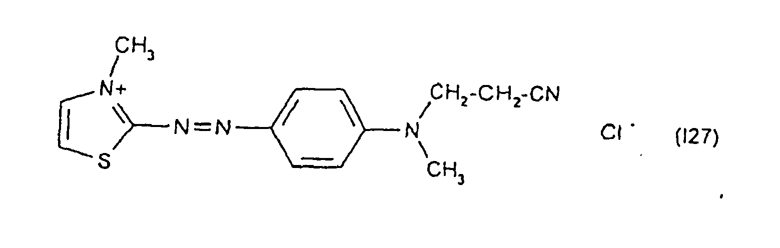 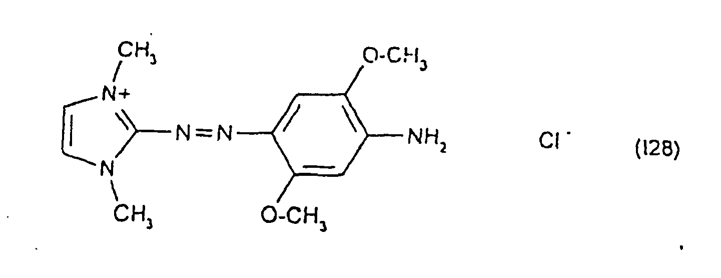 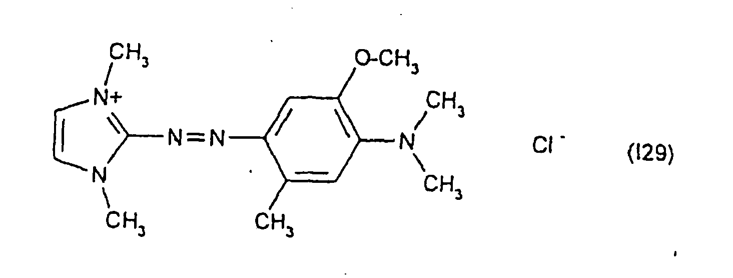 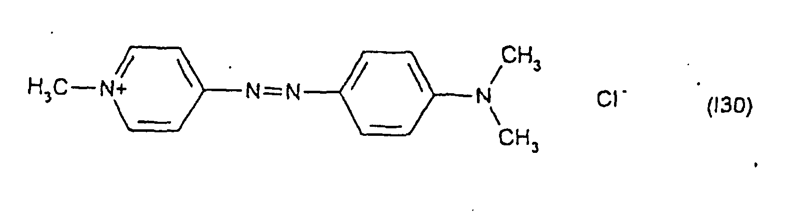 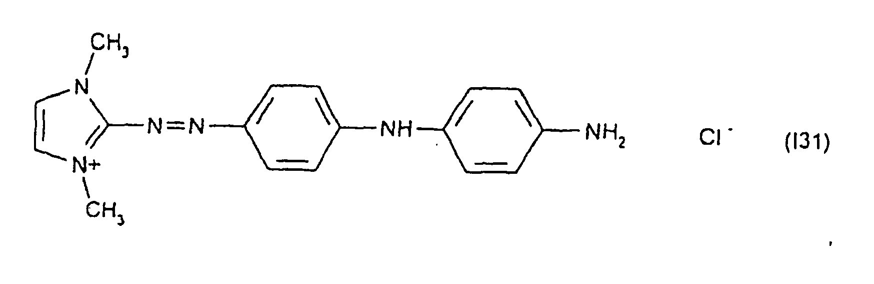 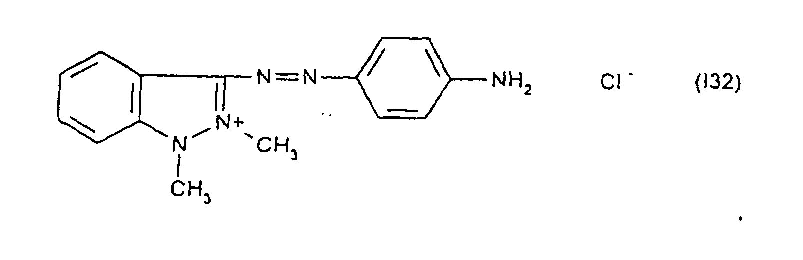 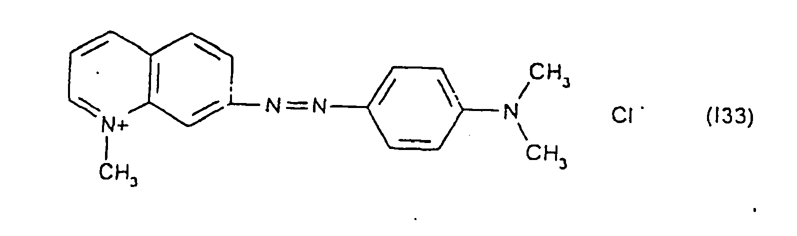 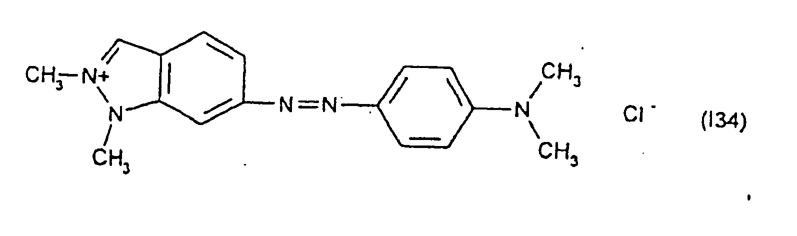 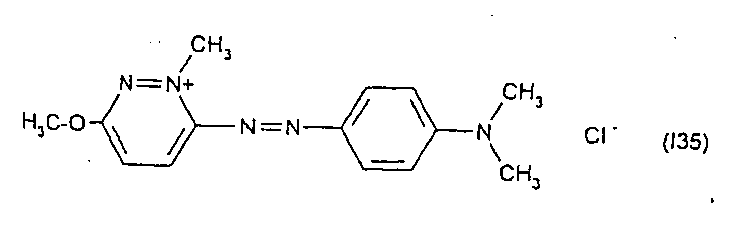 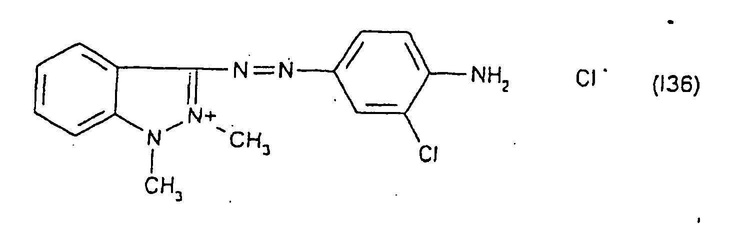 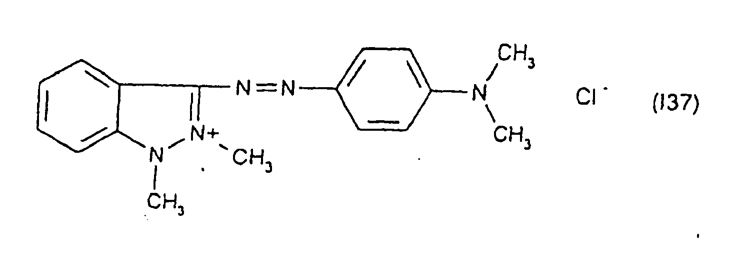 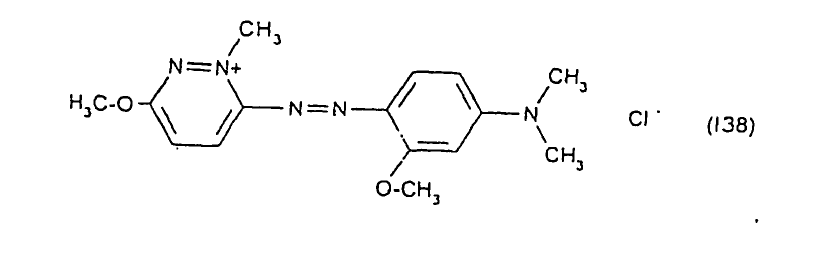 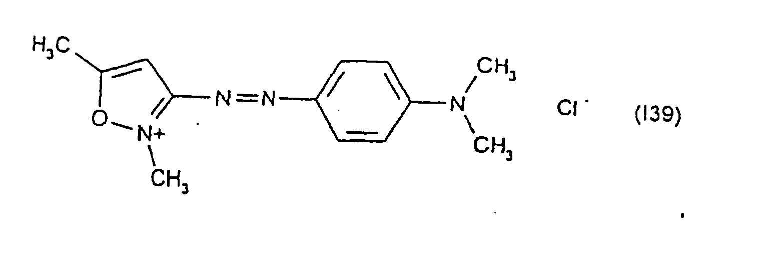 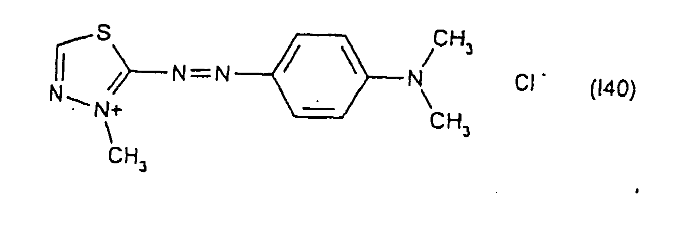 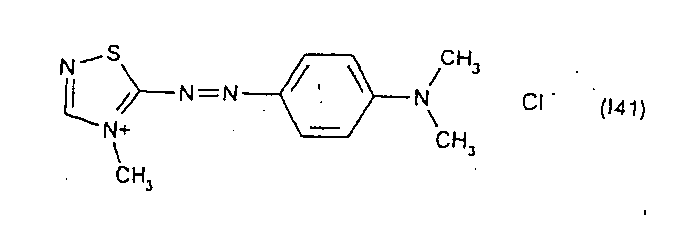 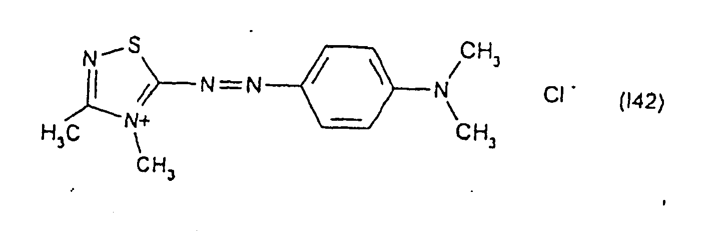 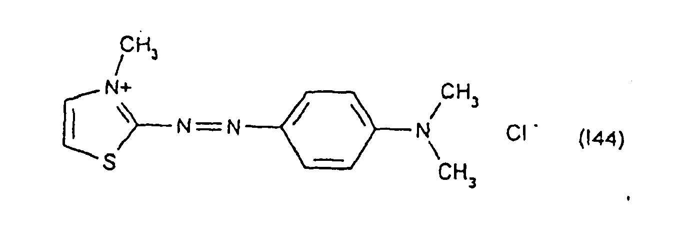 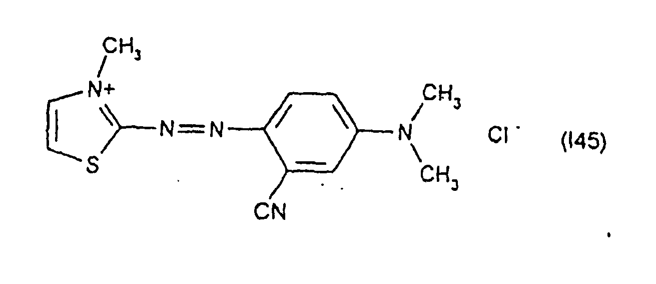 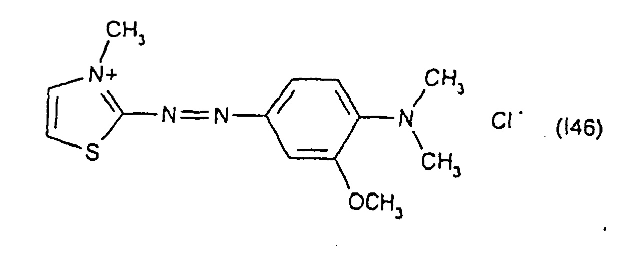 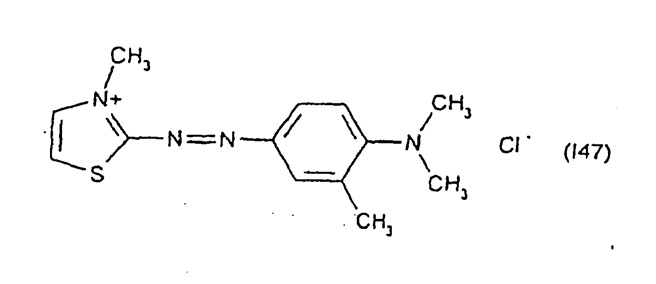 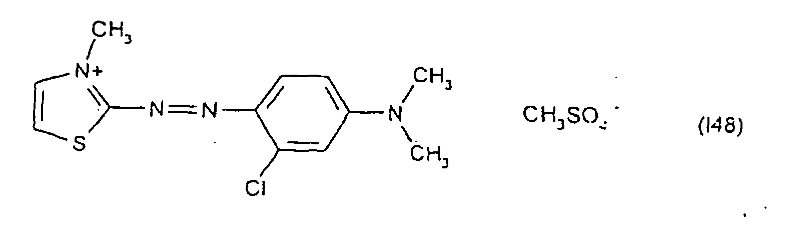 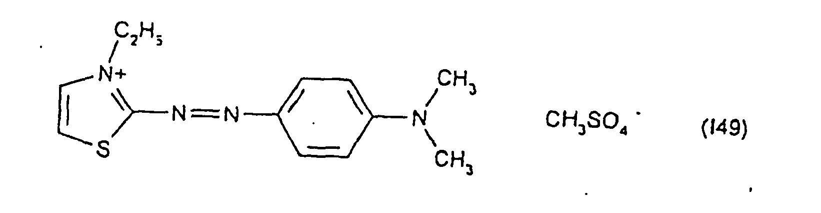 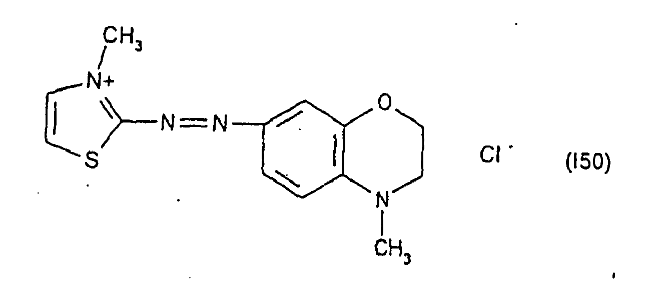 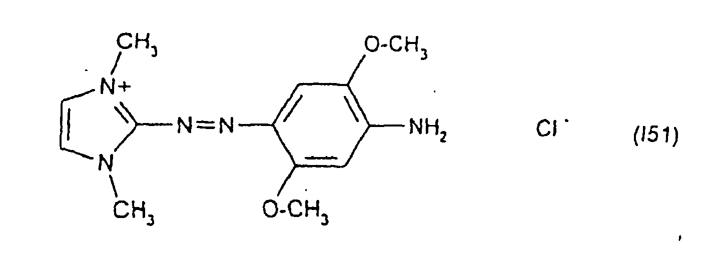 et

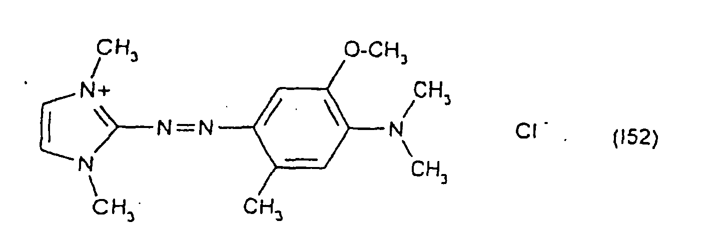

6. Composition selon la revendication 4, **caractérisée par le fait que** les colorants directs cationiques de formule (II) sont choisis parmi les composés répondant aux structures (II1) à (II12) suivantes : 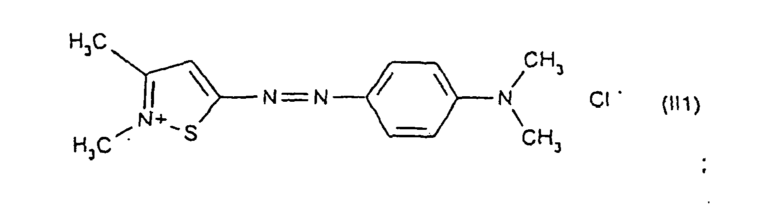 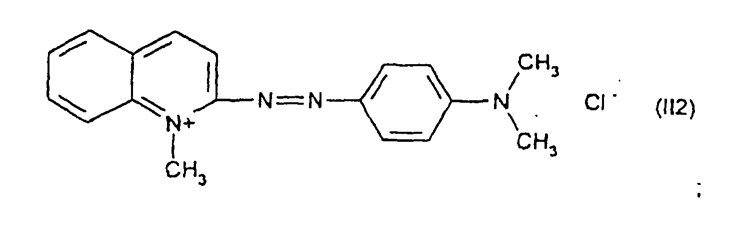 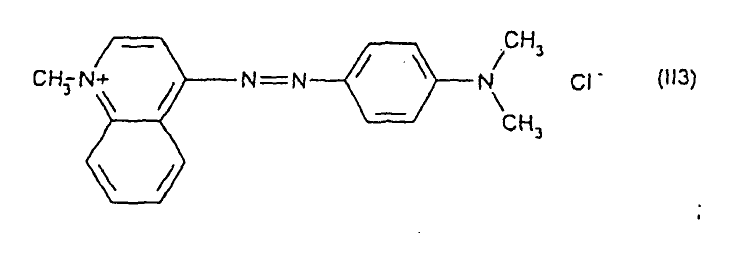 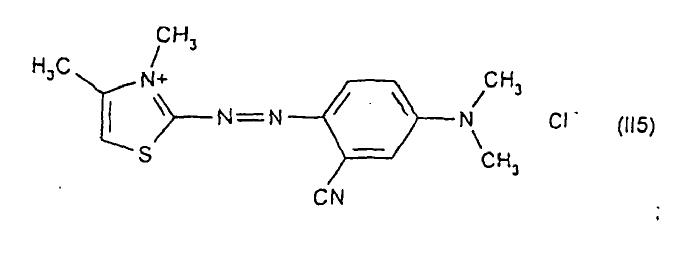 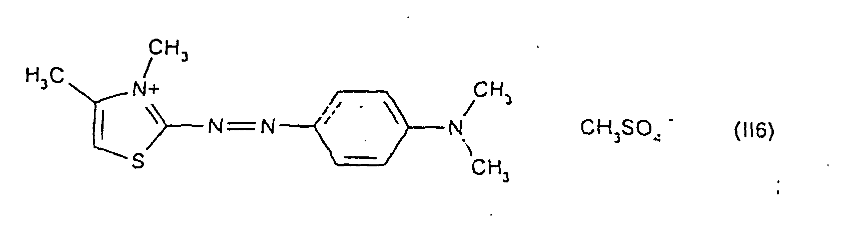 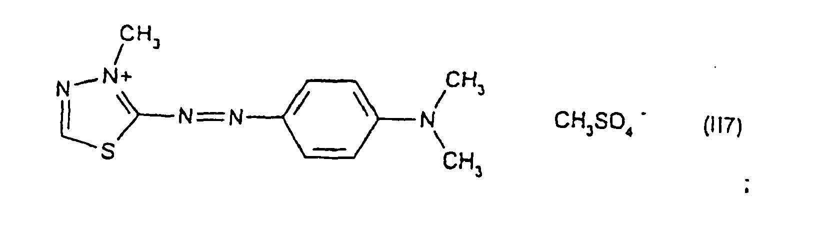 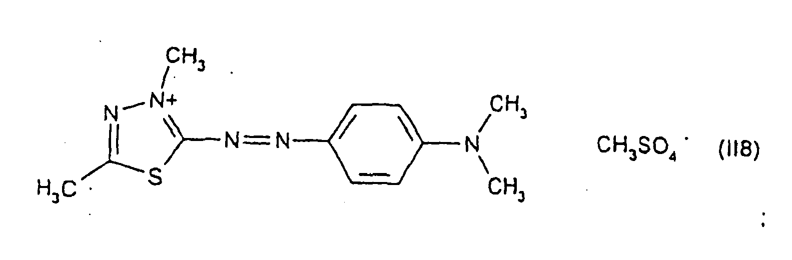 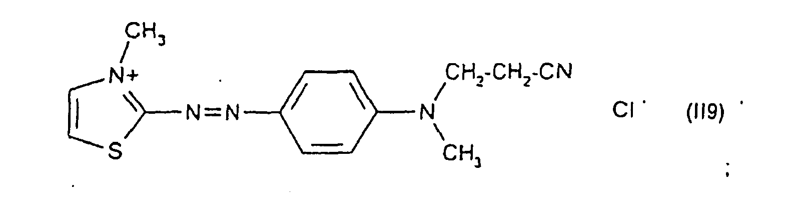 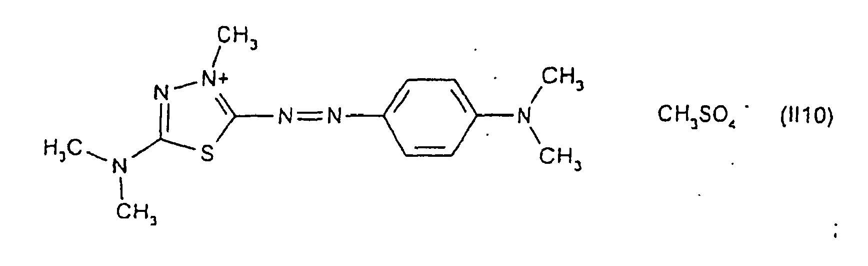 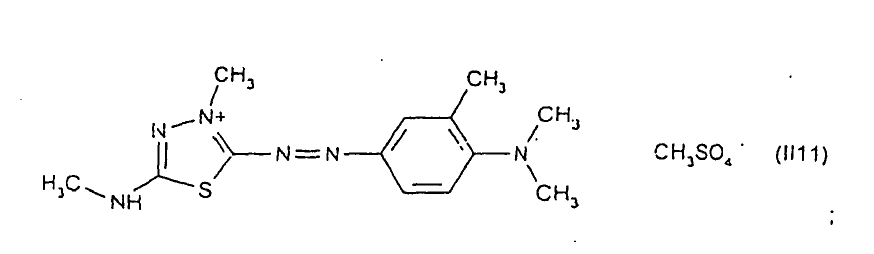 et

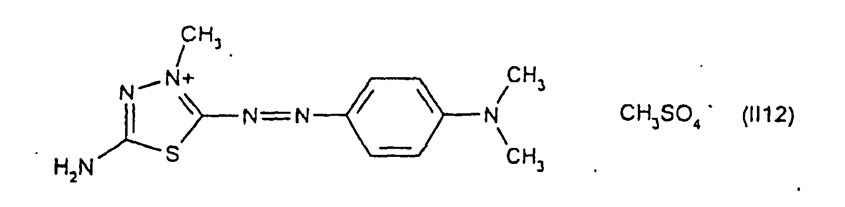

7. Composition selon la revendication 4, **caractérisée par le fait que** les colorants directs cationiques de formule (III) sont choisis parmi les composés répondant aux structures (III1) à (III18) suivantes : 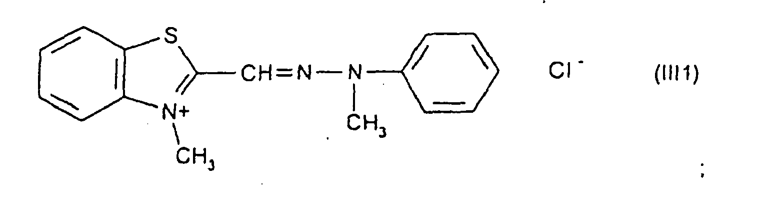 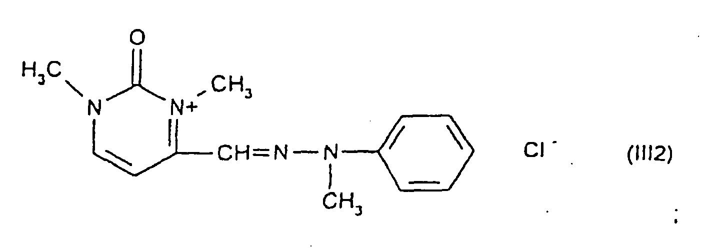 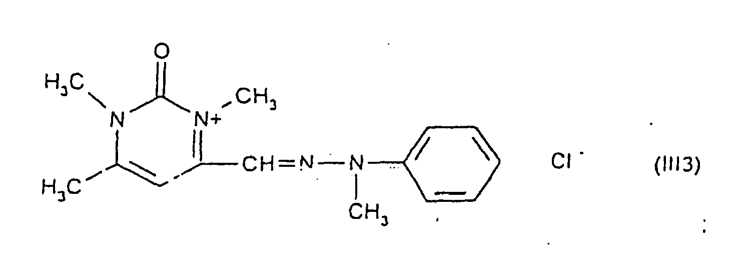 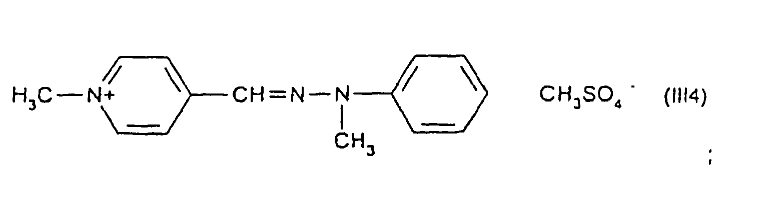 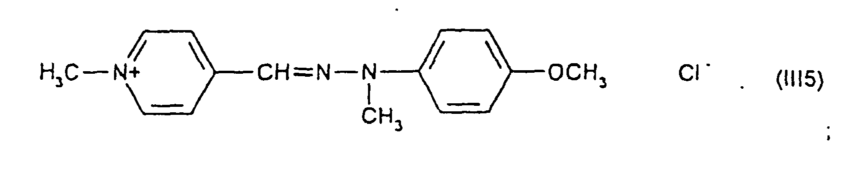 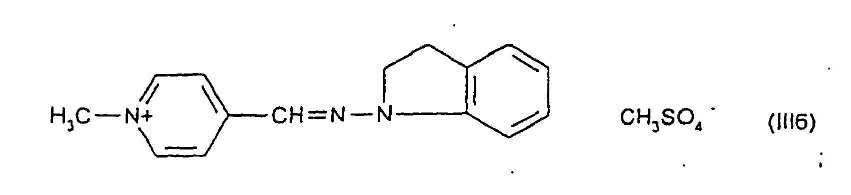 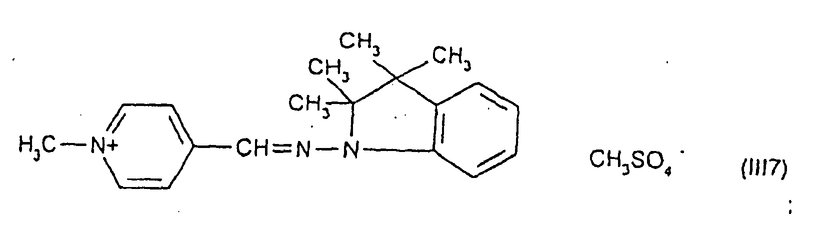 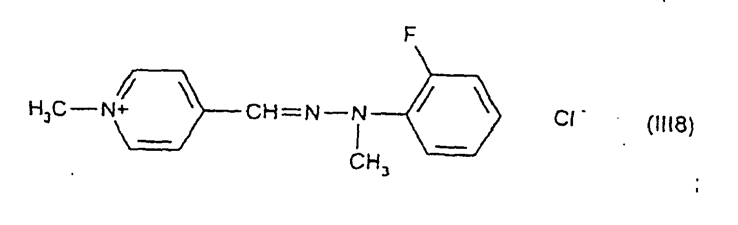 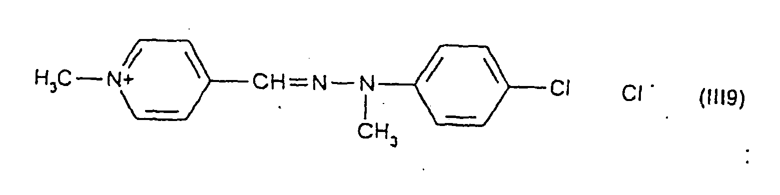 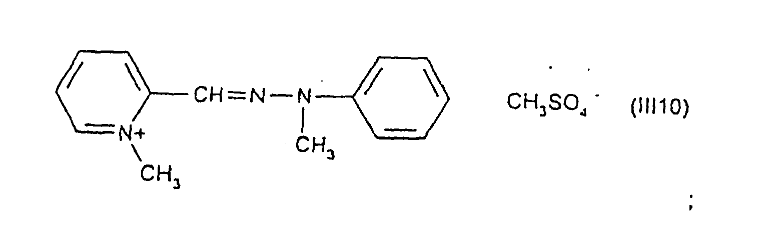 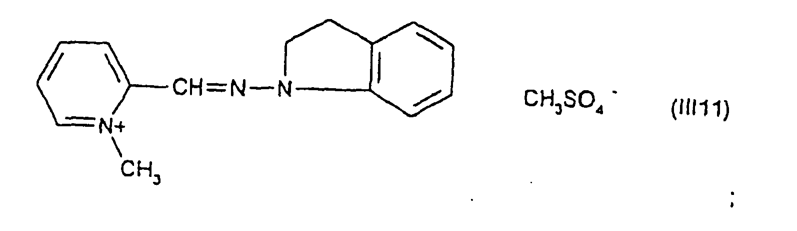 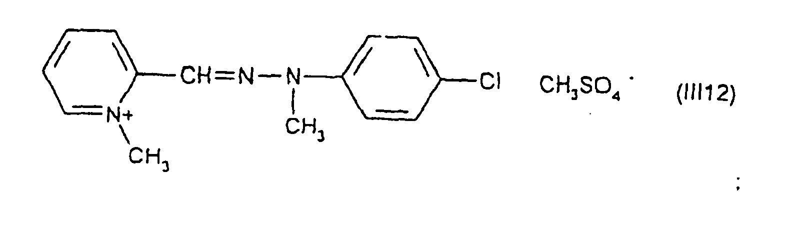 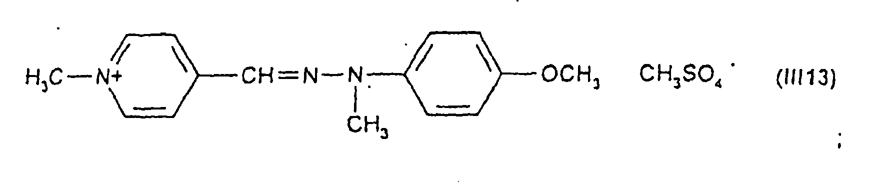 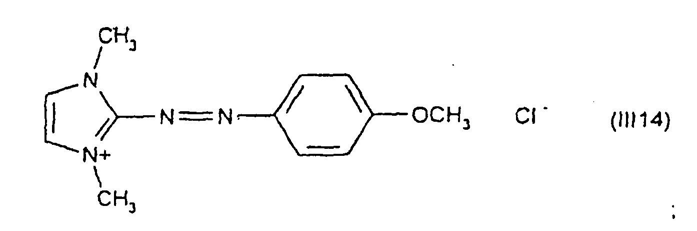 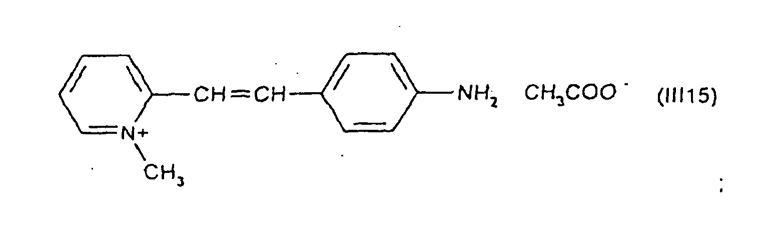 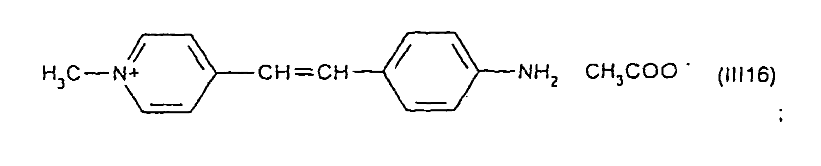 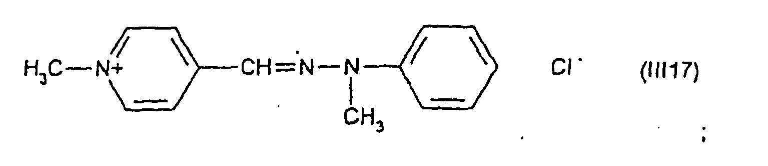 et

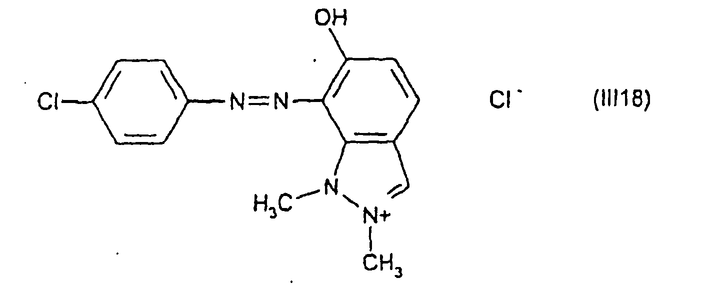

8. Composition selon la revendication 4, **caractérisée par le fait que** les colorants directs cationiques de formule (III') sont choisis parmi les composés répondant aux structures (III'1) à (III'3) suivantes : 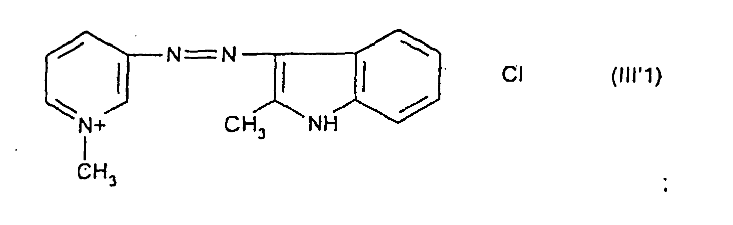 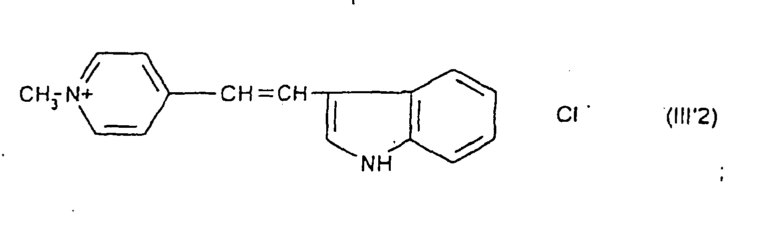 et

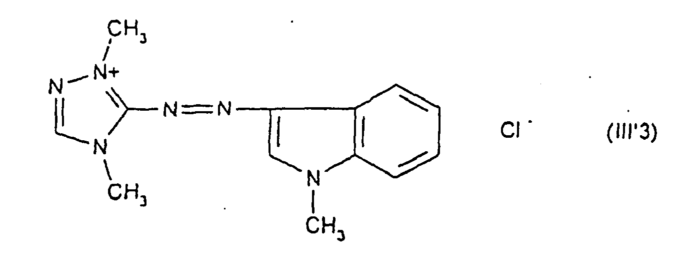

9. Composition selon l'une quelconque des revendications précédentes, **caractérisée par le fait que** le ou les colorants directs cationiques représentent de 0,001 à 10 % en poids du poids total de la composition tinctoriale prête à l'emploi.

10. Composition selon la revendication 9, **caractérisée par le fait que** le ou les colorants directs cationiques représentent de 0,05 à 5 % en poids du poids total de la composition tinctoriale prête à l'emploi.

11. Composition selon l'une quelconque des revendications 1 à 10, **caractérisée par le fait que** les colorants auto-oxydables benzéniques sont choisis parmi les composés de formule (IV) suivante et leurs sels d'addition avec un acide : 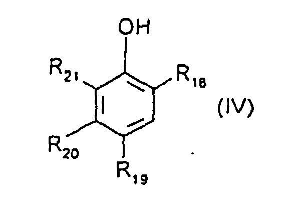 dans laquelle :
- R₁₈ représente un atome d'hydrogène, un radical alkyle en C₁-C₄ ou un radical amino,
- R₁₉ représente un radical alkyle en C₁-C₄, hydroxyle, amino, monoalkyl(C₁-C₄)amino ou dialkyl(C₁-C₄)amino,
- R₂₀ représente un atome d'hydrogène, un radical hydroxyle ou amino.
- R₂₁ représente un atome d'hydrogène ou un radical amino ; étant entendu qu'au moins deux des radicaux R₁₉ à R₂₁ représentent, indépendamment l'un de l'autre, un radical hydroxyle, amino, monoalkyl(C₁-C₄)amino ou dialkyl(C₁-C₄)amino.

12. Composition selon la revendication 11, **caractérisée par le fait que** les colorants auto-oxydables benzéniques de formule (IV) sont choisis parmi le 1,2,4-trihydroxybenzène, le 1-méthyl 2,4,5-trihydroxybenzène, le 2,4-diamino 6-méthyl phénol, le 2-amino 4-méthylamino phénol, le 2,5-diamino 4-méthyl phénol, le 2,6-diamino 4-diéthylamino phénol, le 2,6-diamino 1,4-dihydroxy benzène, et leurs sels d'addition avec un acide.

13. Composition selon l'une quelconque des revendications 1 à 10, **caractérisée par le fait que** les colorants auto-oxydables indoliques sont choisis parmi les composés de formule (V) suivantes : 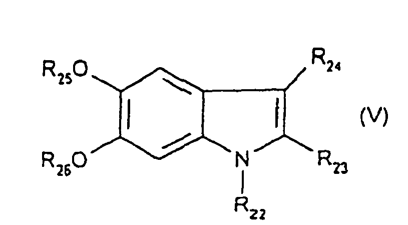 dans laquelle :
- R₂₂, R₂₄, R₂₅ et R₂₆, identiques ou différents, représentent un atome d'hydrogène, un radical alkyle en C₁-C₄ ou acyle en C₁-C₄,
- R₂₃ représente un atome d'hydrogène, un radical alkyle en C₁-C₄ ou un radical carboxyle.

14. Composition selon la revendication 13, **caractérisée par le fait que** les colorants auto-oxydables de formule (V) sont choisis parmi le 5,6-dihydroxy indole, le 2-méthyl 5,6-dihydroxy indole, le 3-méthyl 5,6-dihydroxy indole, le 1-méthyl 5,6-dihydroxy indole, le 2,3-diméthyl 5,6-dihydroxy indole, le 5-méthoxy 6-hydroxyindole, le 5-acétoxy 6-hydroxy indole, le 5,6-diacétoxy indole, l'acide 5,6-dihydroxy indole 2-carboxylique, et leurs sels d'addition avec un acide.

15. Composition selon l'une quelconque des revendications précédentes, **caractérisée par le fait que** le ou les colorants auto-oxydables représentent de 0,0005 à 12 % en poids du poids total de la composition tinctoriale.

16. Composition selon la revendication 15, **caractérisée par le fait que** le ou les colorants auto-oxydables représentent de 0,005 à 8 % en poids du poids total de la composition tinctoriale.

17. Composition selon l'une quelconque des revendications précédentes, **caractérisée par le fait qu'**elle renferme un ou plusieurs agents oxydants choisis parmi le peroxyde d'hydrogène, le peroxyde d'urée, les bromates de métaux alcalins, les persels tels que les perborates et persulfates, et les enzymes.

18. Composition selon la revendication 17, **caractérisée par le fait que** les enzymes sont choisies parmi les peroxydases et les oxydo-réductases à deux électrons.

19. Composition selon la revendication 18, **caractérisée par le fait que** les oxydo-réductases à deux électrons sont choisies parmi les pyranose oxydases, les glucose oxydases, ies glycérol oxydases, les lactates oxydases, les pyruvate oxydases, et les uricases.

20. Composition selon la revendication 18 ou 19, **caractérisée par le fait que** l'oxydo-réductases à 2 électrons est choisie parmi les uricases d'origine animale, microbiologique ou biotechnologique.

21. Composition selon l'une quelconque des revendications 18 à 20, **caractérisée par le fait que caractérisée par le fait que** la ou les oxydo-réductases à 2 électrons représentent de 0,01 à 20 % en poids du poids total de la composition tinctoriale prête à l'emploi.

22. Composition selon la revendication 21, **caractérisée par le fait que** la ou les oxydo-réductases à 2 électrons représentent de 0,1 à 5 % en poids du poids total de la composition tinctoriale prête à l'emploi.

23. Composition selon l'une quelconque des revendications 20 à 22, **caractérisée par le fait qu'**elle renferme un donneur (ou substrat) pour ladite oxydo-réductase à 2 électrons, choisi parmi l'acide urique et ses sels.

24. Composition selon l'une quelconque des revendications 17 à 23, **caractérisée par le fait qu'**elle renferme une ou plusieurs bases d'oxydation choisies parmi les paraphénylénediamines, les para-aminophénols, les orthophénylènediamines et les bases hétérocycliques et/ou un ou plusieurs coupleurs choisis parmi les méta-phénylènediamines, les méta-aminophénols, les métadiphénols, les coupleurs hétérocycliques tels que par exemple les dérivés indoliques, les dérivés indoliniques, les dérivés de benzimidazole, les dérivés de benzomorpholine, les dérivés de sésamol, les dérivés pyridiniques, pyrimidiniques et pyrazoliques, et leurs sels d'addition avec un acide.

25. Composition selon la revendication 24, **caractérisée par le fait que** la ou les bases d'oxydation représentent de 0,0005 à 12 % en poids du poids total de la composition tinctoriale prête à l'emploi et que le ou les coupleurs représentent de 0,0001 à 10 % en poids du poids total de la composition tinctoriale prête à l'emploi.

26. Composition selon la revendication 25, **caractérisée par le fait que** la ou les bases d'oxydation représentent de 0,005 à 8 % en poids du poids total de la composition tinctoriale prête à l'emploi et que le ou les coupleurs représentent de 0,005 à 8 % en poids du poids total de la composition tinctoriale prête à l'emploi.

27. Composition selon l'une quelconque des revendications 11, 12, 14 à 16, et 24 à 26, **caractérisée par le fait que** les sels d'addition avec un acide sont choisis parmi les chlorhydrates, les bromhydrates, les sulfates, les tartrates, les lactates et les acétates.

28. Composition selon l'une quelconque des revendications précédentes, **caractérisée par le fait que** le milieu approprié pour la teinture est constitué par de l'eau ou par un mélange d'eau et d'au moins un solvant organique.

29. Composition selon l'une quelconque des revendications précédentes, **caractérisée par le fait qu'**elle présente un pH compris 5 et 11.

30. Composition selon l'une quelconque des revendications 1 et 9 à 29, **caractérisée en ce que** le colorant direct cationique est de formule suivante :

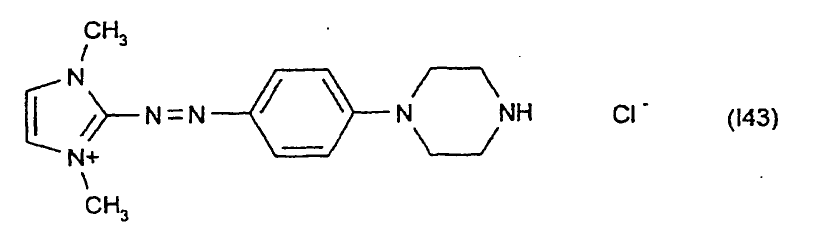

31. Procédé de teinture des fibres kératiniques et en particulier des fibres kératiniques humaines telles que les cheveux, **caractérisé par le fait qu'**on applique sur lesdites fibres au moins une composition tinctoriale prête à l'emploi telle que définie dans l'une quelconque des revendications précédentes, pendant un temps suffisant pour développer la coloration désirée.

32. Procédé selon la revendication 31, **caractérisé par le fait qu'**il comporte une étape préliminaire consistant à stocker sous forme séparée, d'une part, une composition (A) comprenant, dans un milieu approprié pour la teinture, au moins un colorant direct cationique, et au moins un colorant auto-oxydable et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques.

33. Dispositif à plusieurs compartiments ou "kit" de teinture, **caractérisé par le fait qu'**il comporte un premier compartiment renfermant la composition (A) telle que définie dans la revendication 32 et un second compartiment renfermant la composition (B) telle que définie dans la revendication 32.

## Claims

1. Ready-to-use composition for dyeing keratin fibres, and in particular human keratin fibres such as the hair, **characterized in that** it comprises, in a medium which is suitable for dyeing:
- at least one cationic direct dye and
- at least one benzene or indole auto-oxidizable dye.

2. Composition according to Claim 1, **characterized in that** the cationic direct dye(s) is (are) chosen from cationic aminoanthraquinone dyes, cationic monoazo or diazo dyes and cationic naphthoquinone dyes.

3. Composition according to Claim 2, **characterized in that** the cationic direct dye(s) is (are) chosen from [8-[(p-aminophenyl)azo]-7-hydroxy-2-naphthyl]trimethylammonium chloride, 3-[(4-amino-6-bromo-5,8-dihydro-1-hydroxy-8-imino-5-oxo-2-naphthalenyl)amino]-N,N,N-trimethylbenzenaminium chloride, 7-hydroxy-8-[(2-methoxyphenyl)azo]-N,N,N-trimethyl-2-naphthalenaminium chloride, [8-[(4-amino-2-nitrophenyl)azo]-7-hydroxy-2-naphthyl]-trimethylammonium chloride and 3-[(4,5-dihydro-3-methyl-5-oxo-1-phenyl-1H-pyrazol-4-yl)azo]-N,N,N-trimethylbenzenaminium chloride.

4. Composition according to Claim 1, **characterized in that** the cationic direct dye(s) is (are) chosen from:
a) the compounds of formula (I) below: 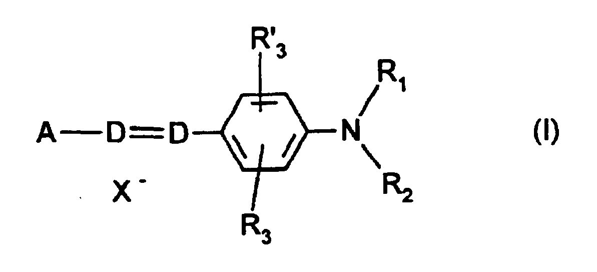 in which:
D represents a nitrogen atom or a -CH group,
R₁ and R₂, which may be identical or different, represent a hydrogen atom; a C₁-C₄ alkyl radical which can be substituted with a -CN, -OH or -NH₂ radical; or form, with a carbon atom of the benzene ring, an optionally oxygenated or nitrogenous heterocycle, which can be substituted with one or more C₁-C₄ alkyl radicals; a 4'-aminophenyl radical,
R₃ and R'₃, which may be identical or different, represent a hydrogen or halogen atom chosen from chlorine, bromine, iodine and fluorine, or a cyano, C₁-C₄ alkoxy or acetyloxy radical,
X⁻ represents an anion preferably chosen from chloride, methyl sulfate and acetate,
A represents a group chosen by structures A1 to A18 below: 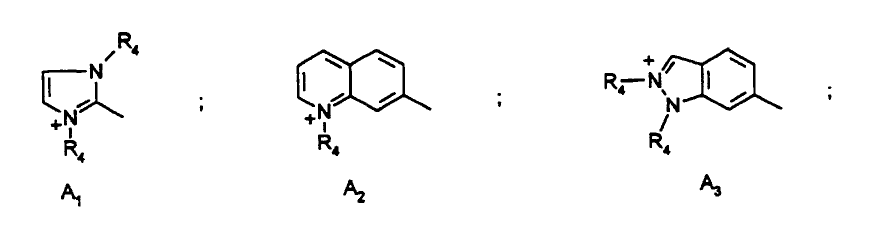 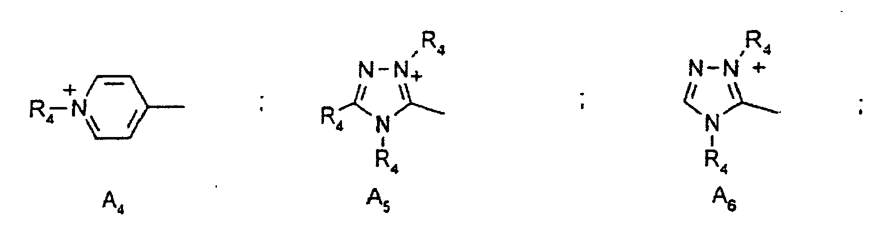 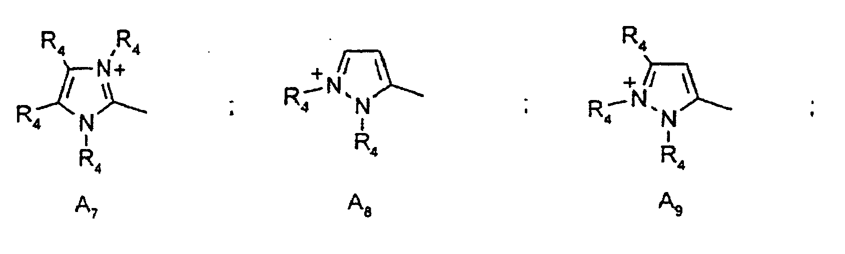 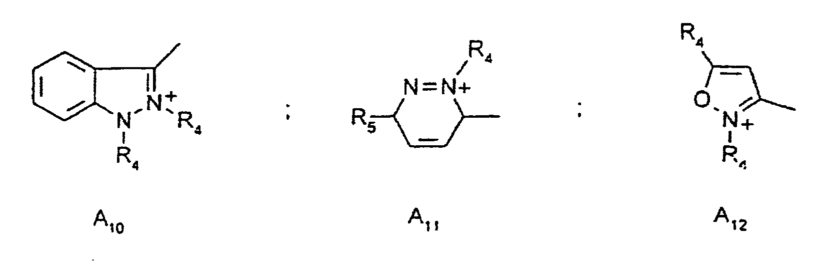 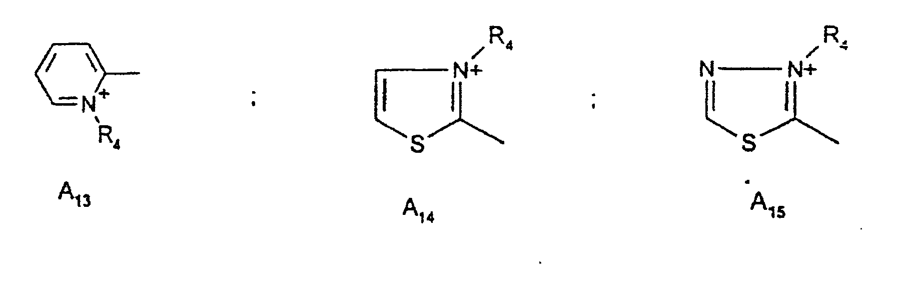 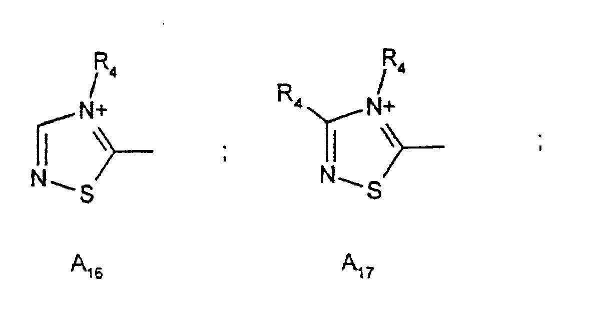 and 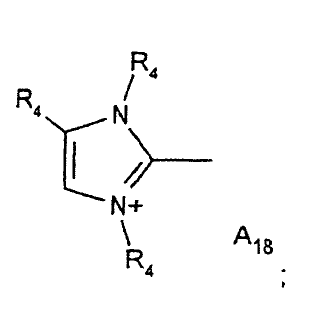
in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom;
b) the compounds of formula (II) below: 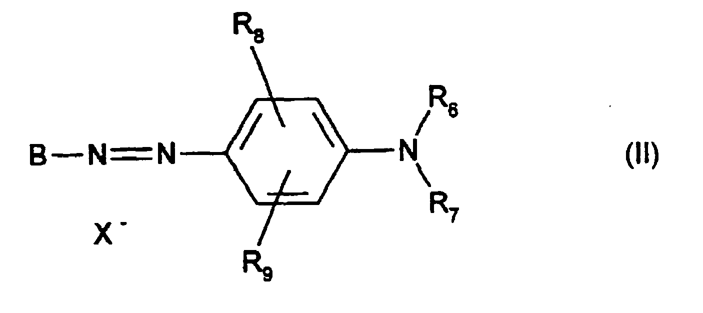 in which:
R₆ represents a hydrogen atom or a C₁-C₄ alkyl radical,
R₇ represents a hydrogen atom, an alkyl radical which can be substituted with a -CN radical or with an amino group, a 4'-aminophenyl radical or forms, with R₆, an optionally oxygenated and/or nitrogenous heterocycle which can be substituted with a C₁-C₄ alkyl radical,
R₈ and R₉, which may be identical or different, represent a hydrogen atom, a halogen atom such as bromine, chlorine, iodine or fluorine, a C₁-C₄ alkyl or C₁-C₄ alkoxy radical or a -CN radical,
X⁻ represents an anion preferably chosen from chloride, methyl sulfate and acetate,
B represents a group chosen by structures B1 to B6 below: 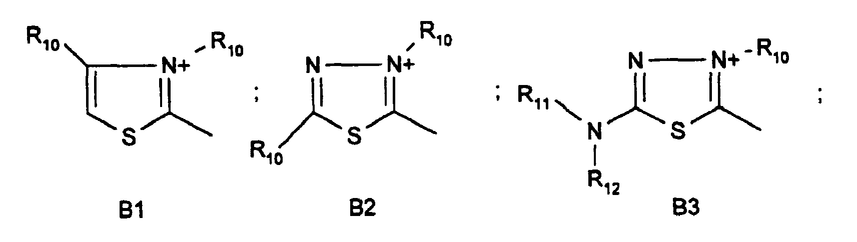 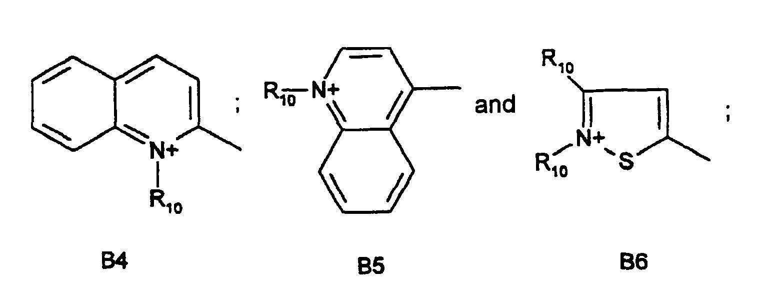
in which R₁₀ represents a C₁-C₄ alkyl radical, R₁₁ and R₁₂, which may be identical or different, represent a hydrogen atom or a C₁-C₄ alkyl radical;
c) the compounds of formulae (III) and (III') below: 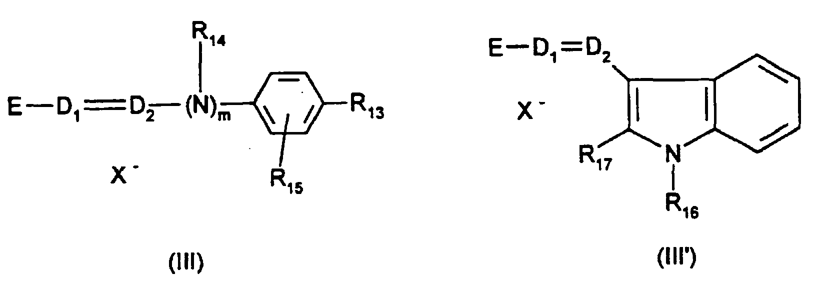 in which:
R₁₃ represents a hydrogen atom, a C₁-C₄ alkoxy radical, a halogen atom such as bromine, chlorine, iodine or fluorine, or an amino radical,
R₁₄ represents a hydrogen atom, a C₁-C₄ alkyl radical or forms, with a carbon atom of the benzene ring, a heterocycle which is optionally oxygenated and/or substituted with one or more C₁-C₄ alkyl groups,
R₁₅ represents a hydrogen atom or a halogen atom such as bromine, chlorine, iodine or fluorine,
R₁₆ and R₁₇, which may be identical or different, represent a hydrogen atom or a C₁-C₄ alkyl radical,
D₁ and D₂, which may be identical or different, represent a nitrogen atom or a -CH group,
m = 0 or 1,
it being understood that when R₁₃ represents an unsubstituted amino group, then D₁ and D₂ simultaneously represent a -CH group and m = 0,
X⁻ represents an anion preferably chosen from chloride, methyl sulfate and acetate,
E represents a group chosen by structures E1 to E8 below: 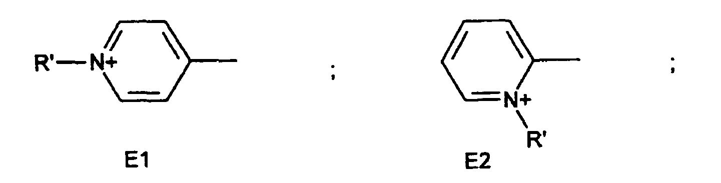 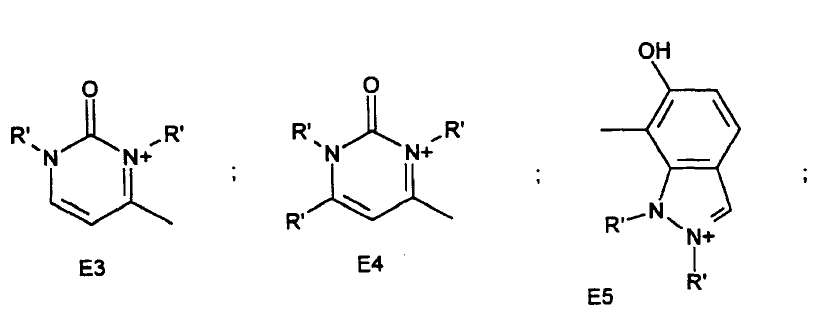 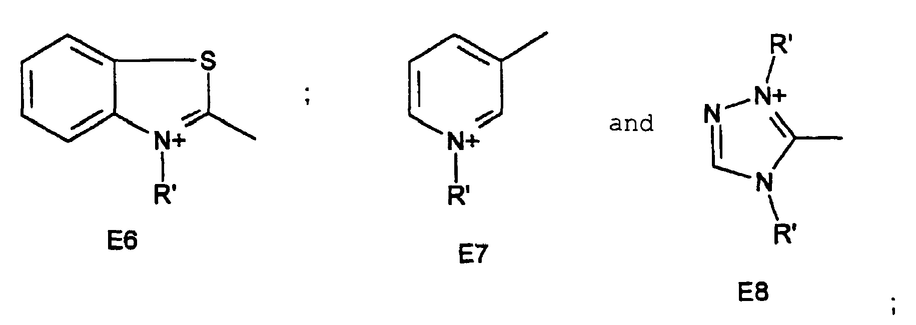 in which R' represents a C₁-C₄ alkyl radical;
when m = 0 and when D₁ represents a nitrogen atom, then E can also denote a group of structure E9 below: 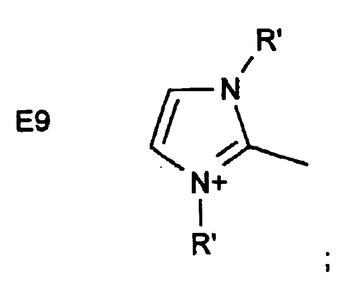 in which R' represents a C₁-C₄ alkyl radical.

5. Composition according to Claim 4, **characterized in that** the cationic direct dyes of formula (I) are chosen from the compounds corresponding to structures (I1) to (I52) below: 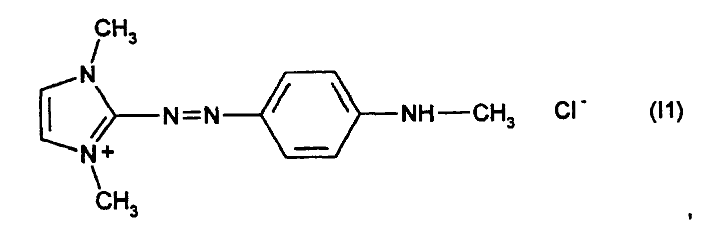 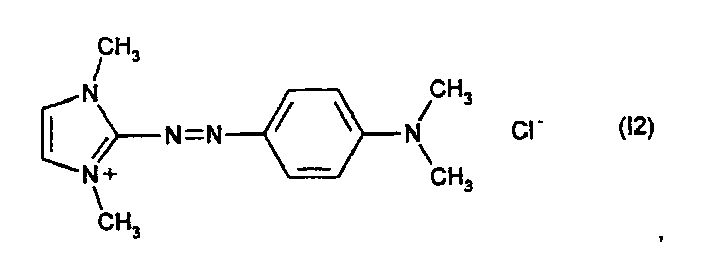 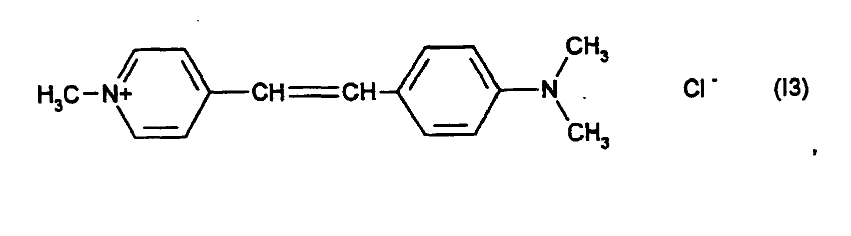 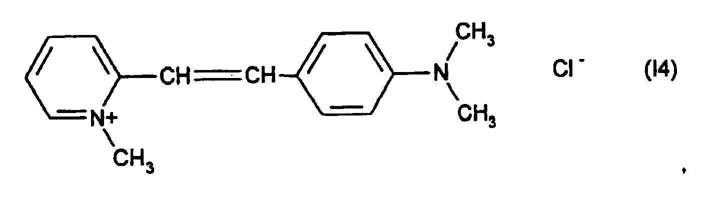 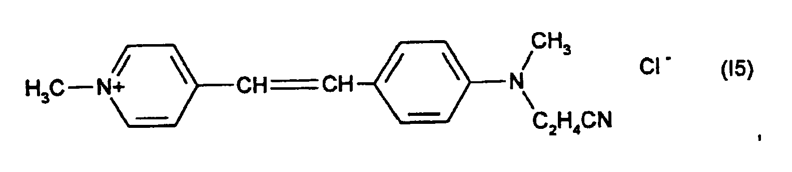 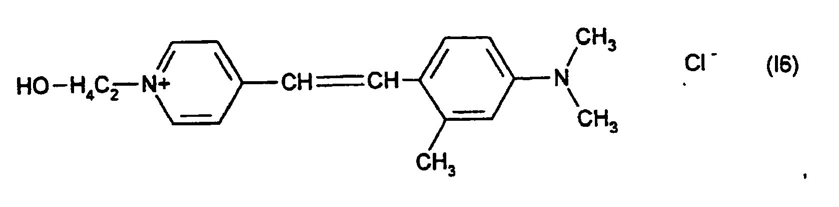 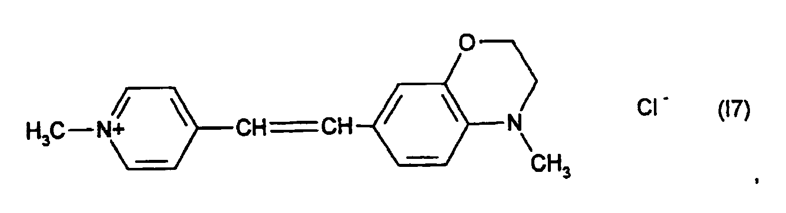 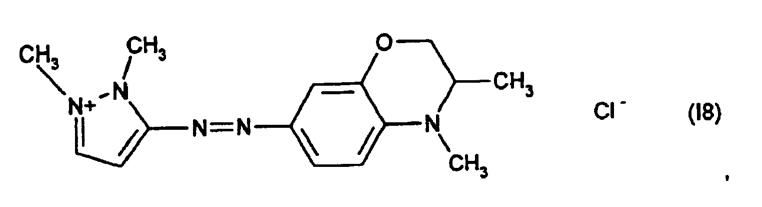 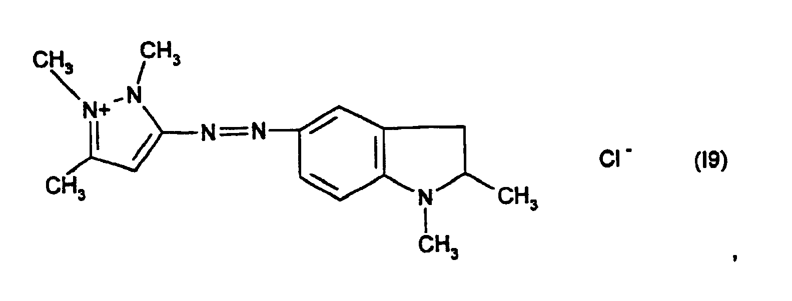 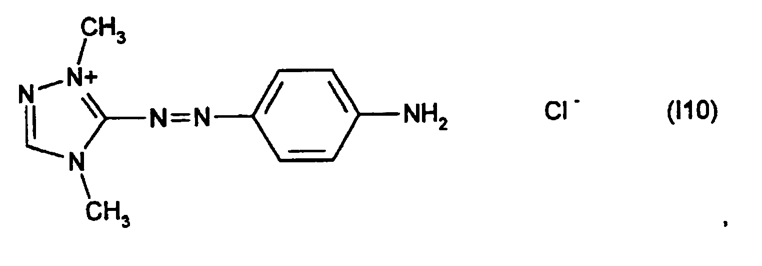 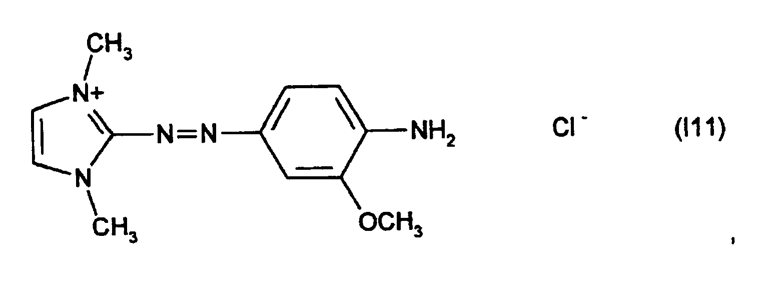 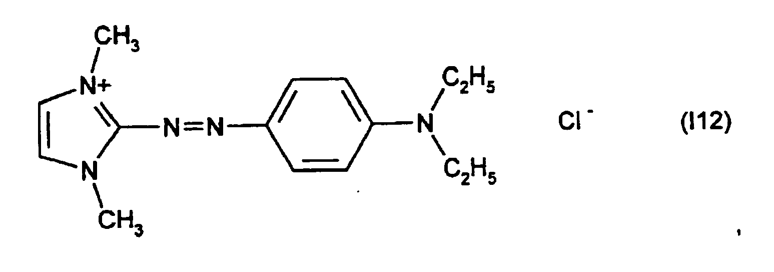 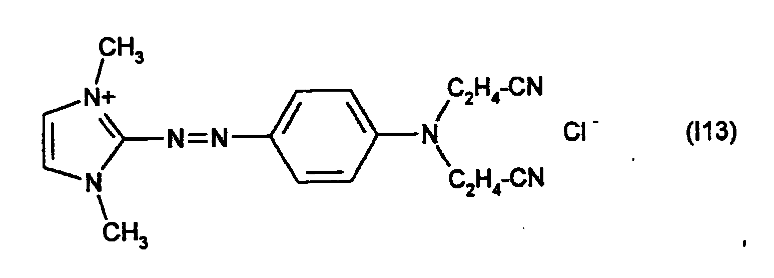 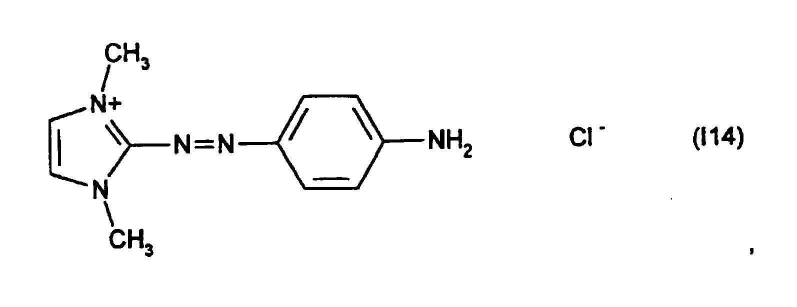 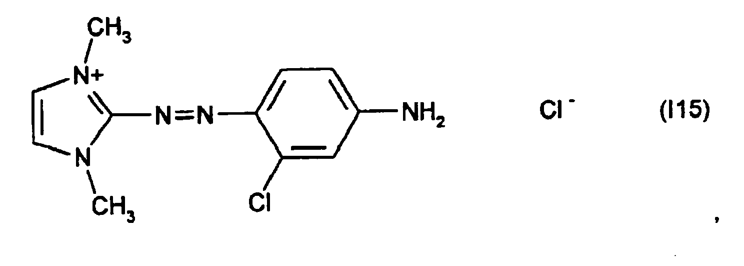 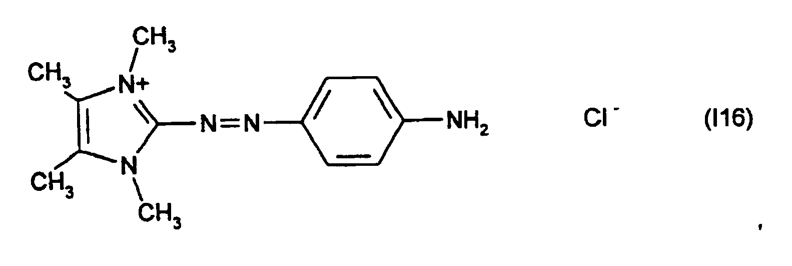 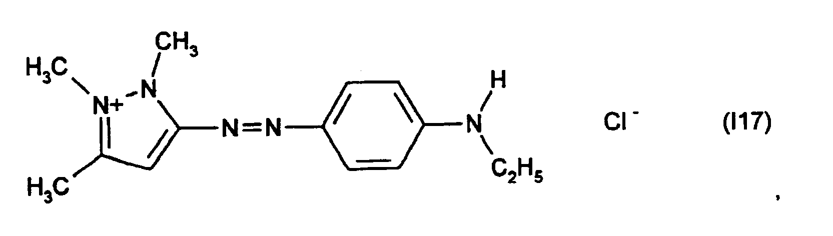 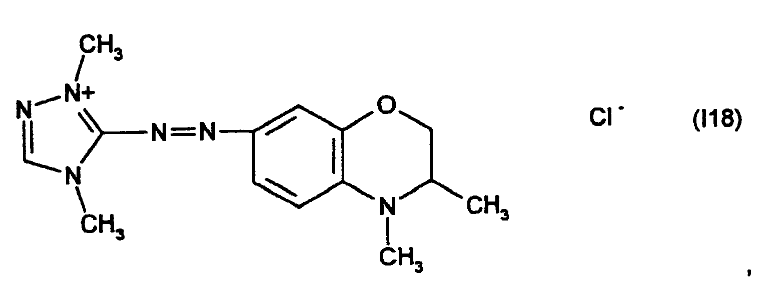 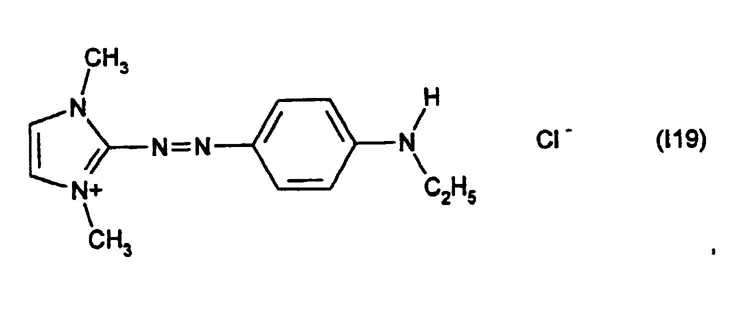 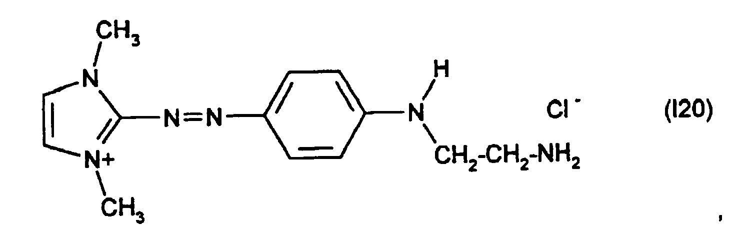 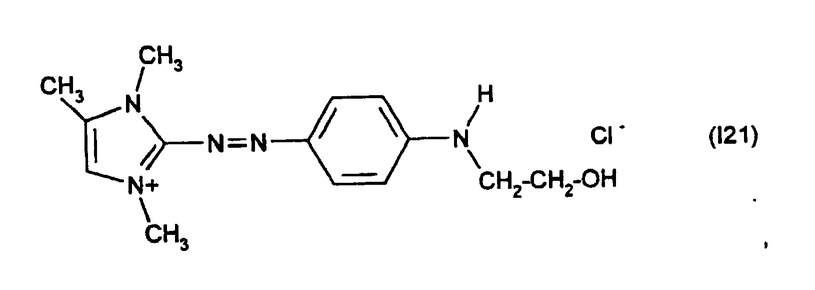 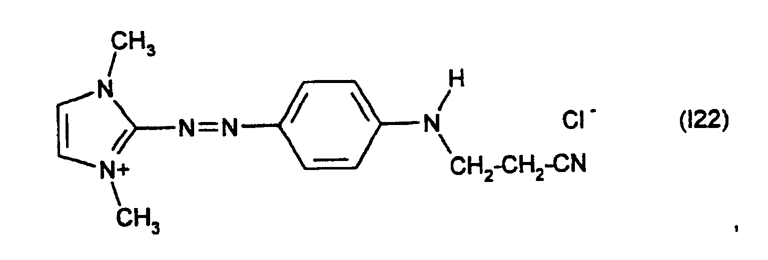 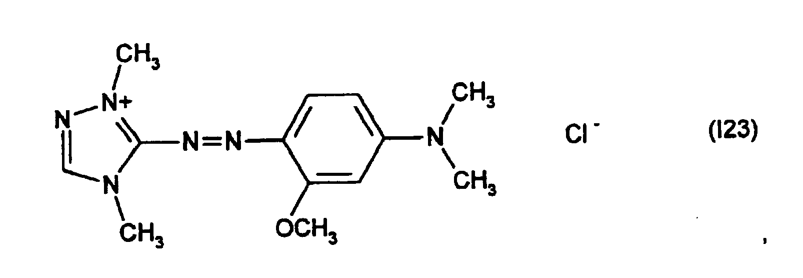 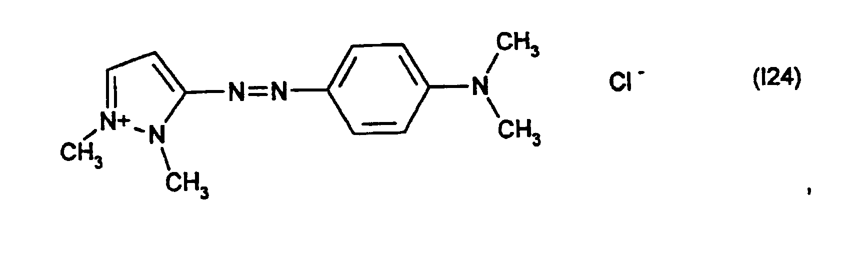 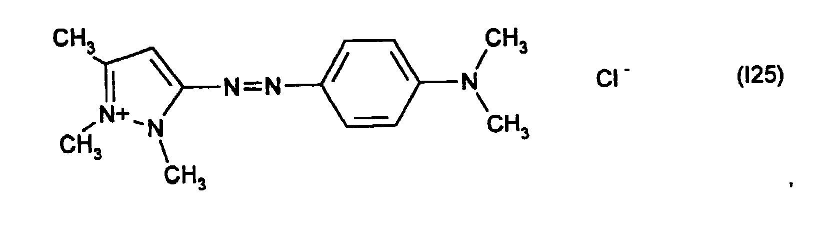 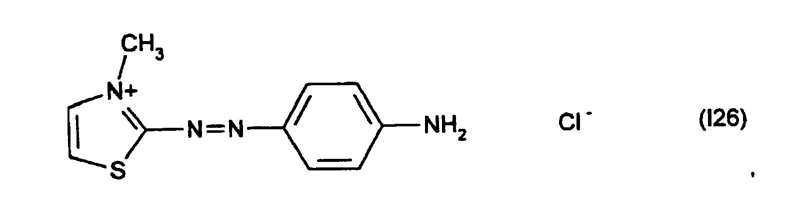 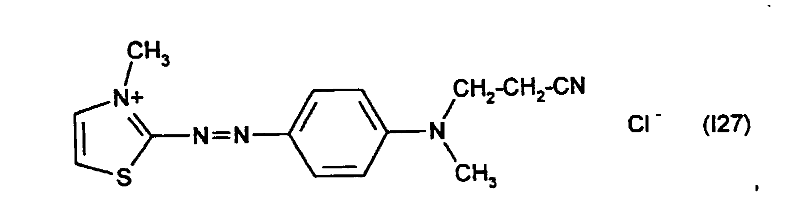 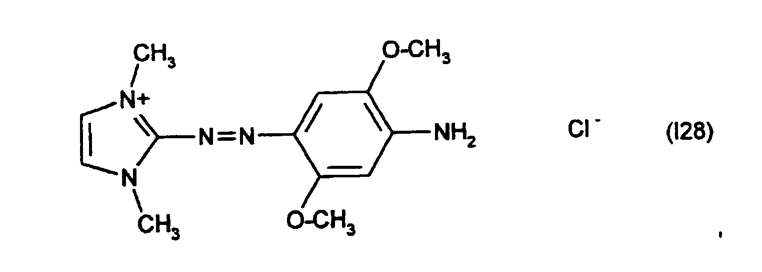 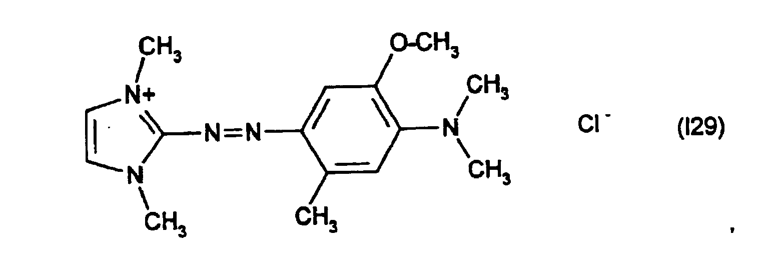 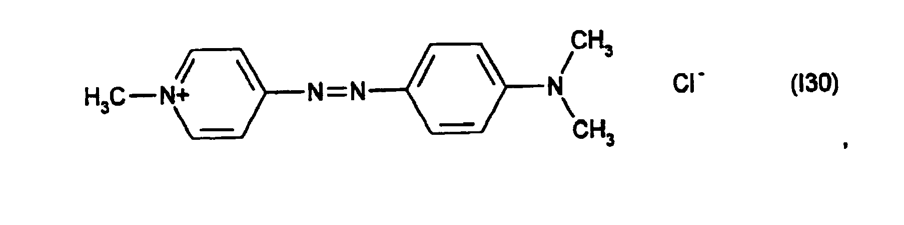 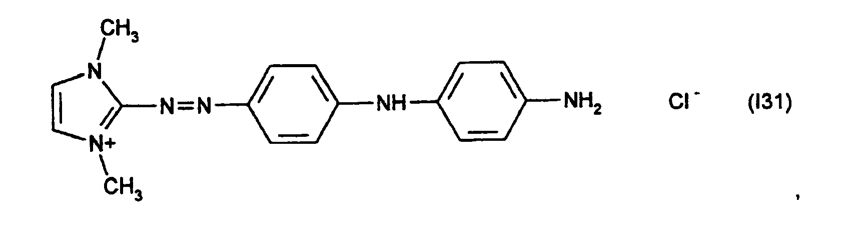 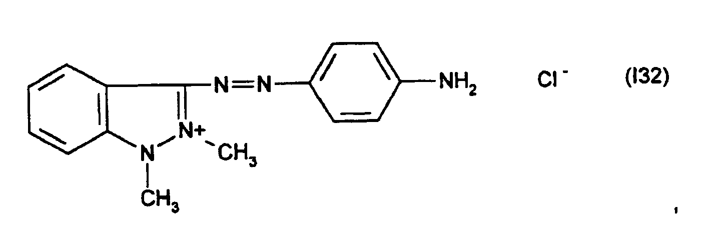 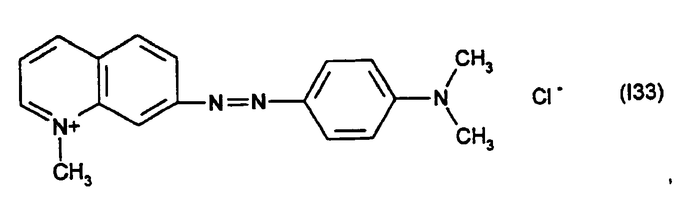 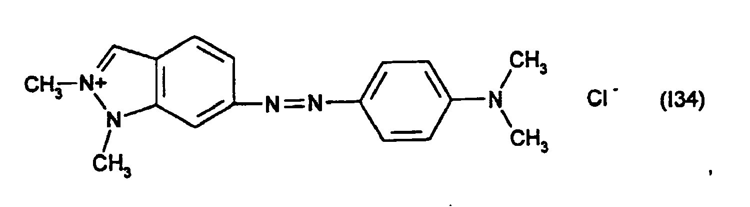 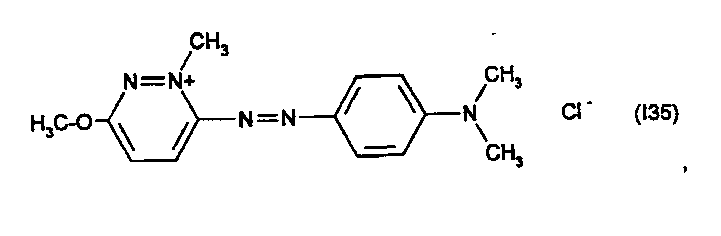 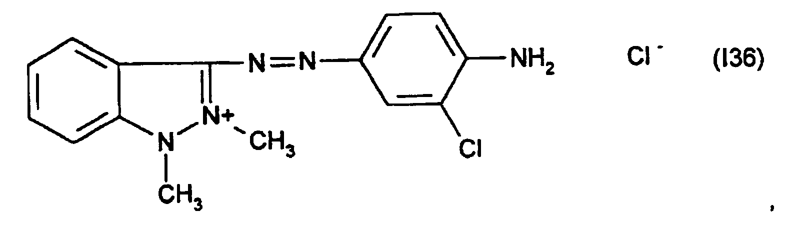 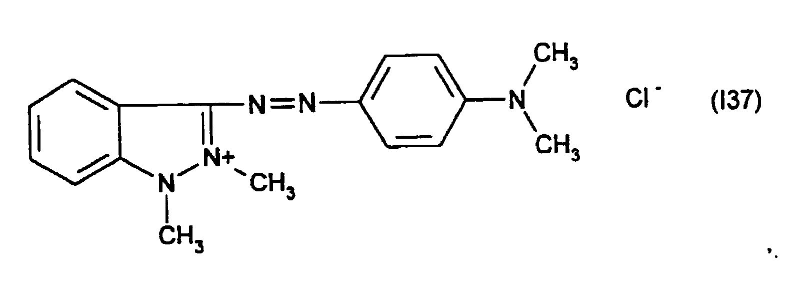 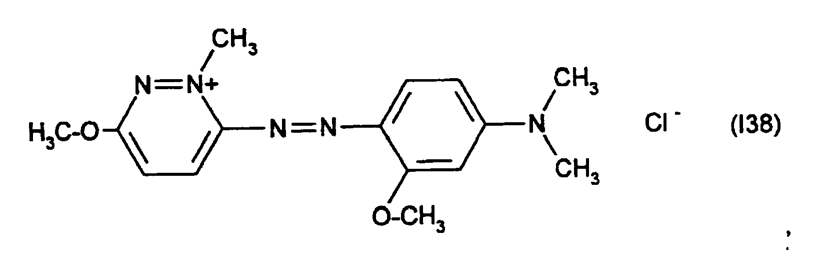 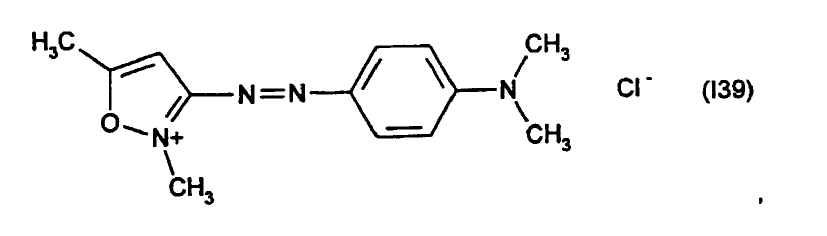 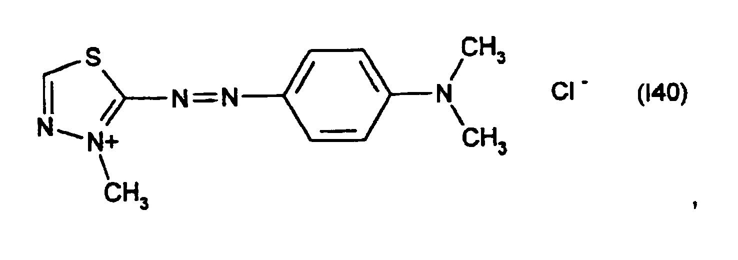 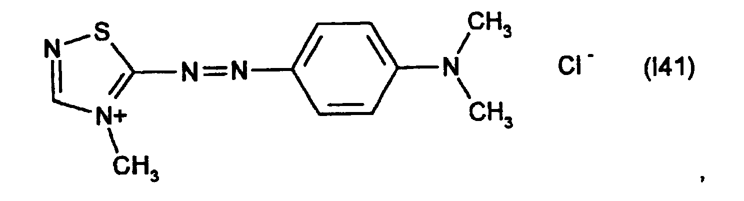 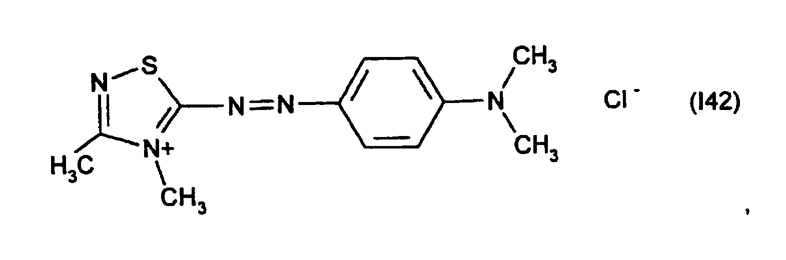 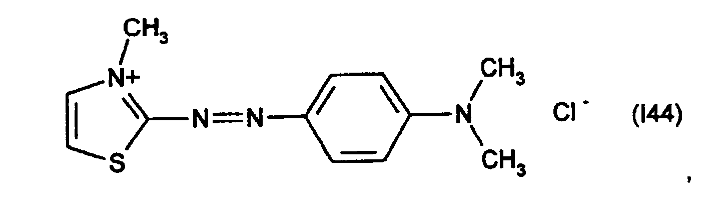 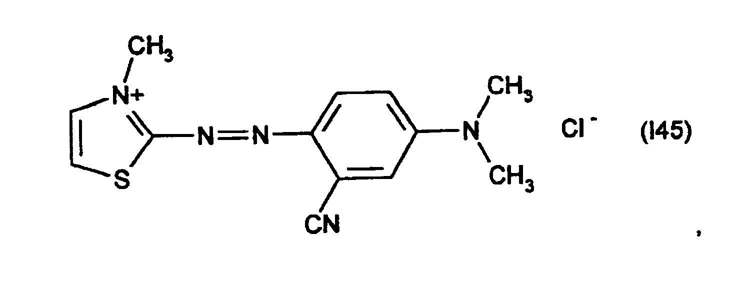 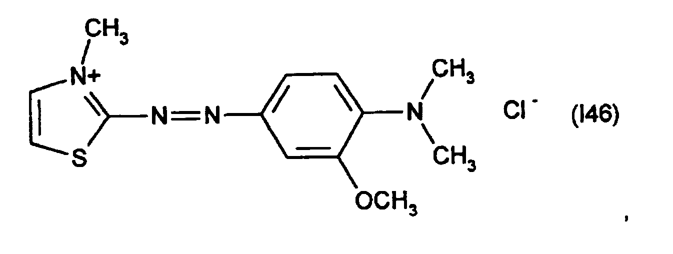 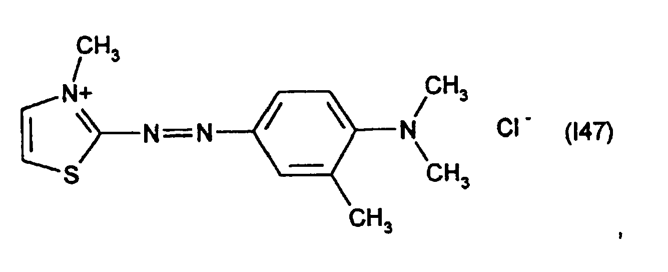 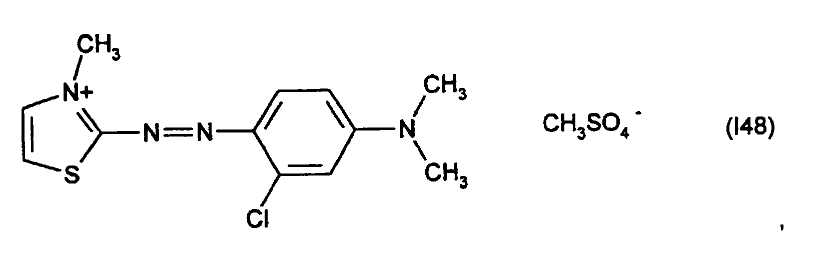 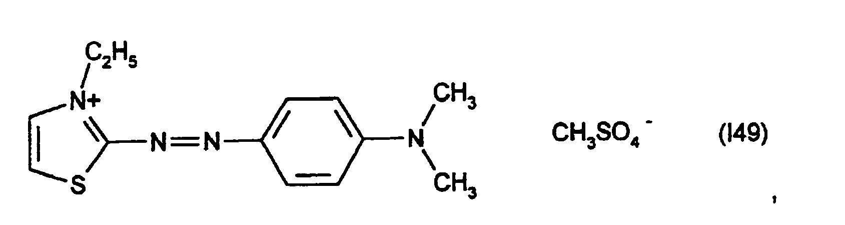 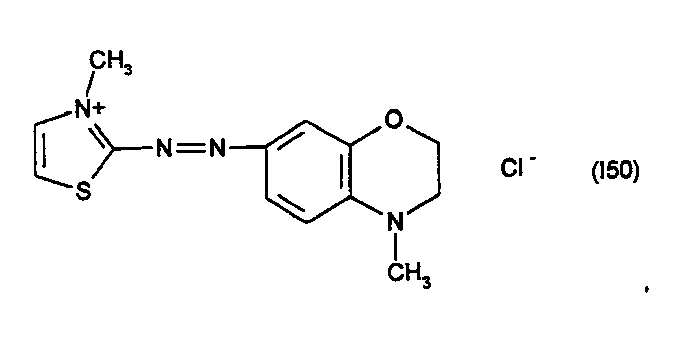 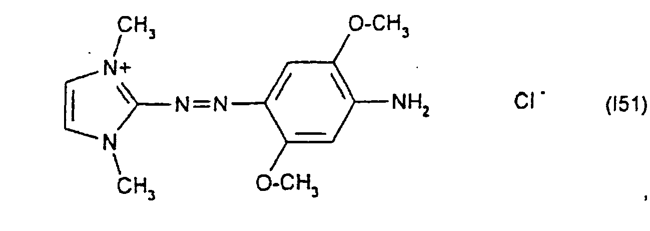 and

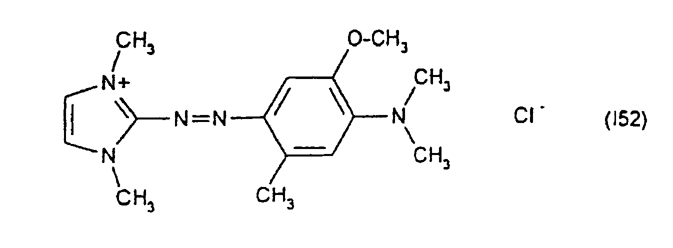

6. Composition according to Claim 4, **characterized in that** the cationic direct dyes of formula (II) are chosen from the compounds corresponding to structures (II1) to (II12) below: 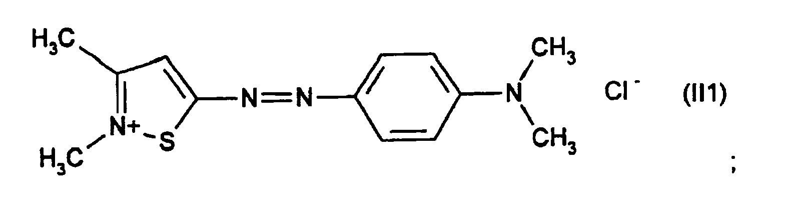 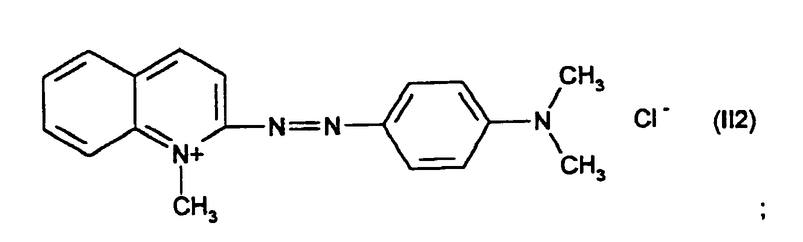 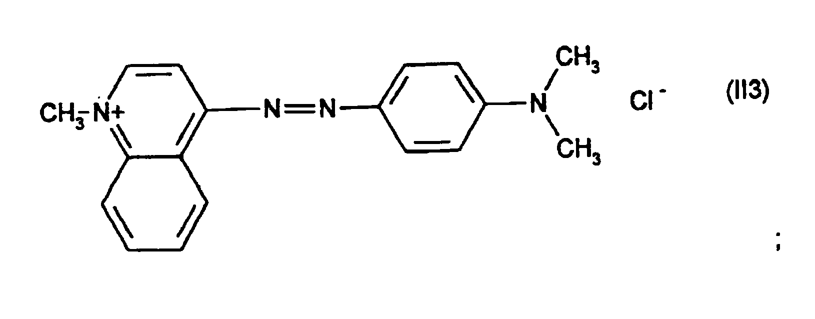 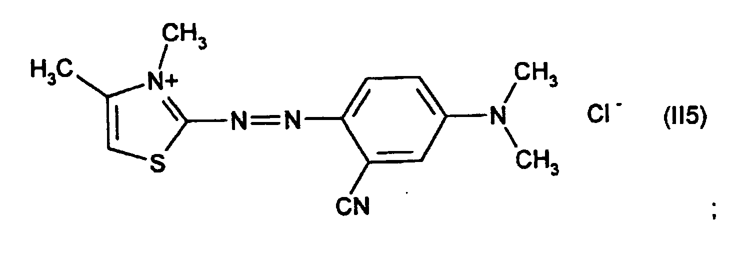 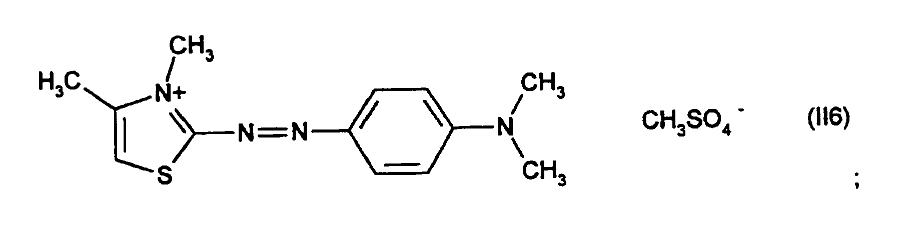 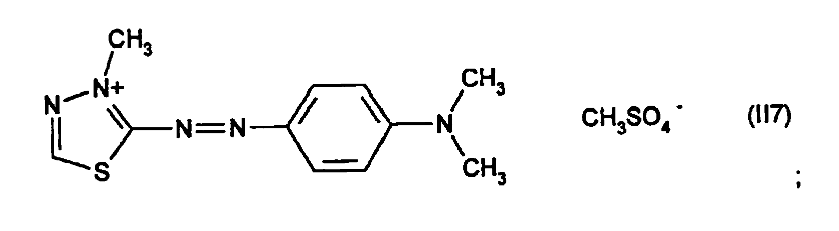 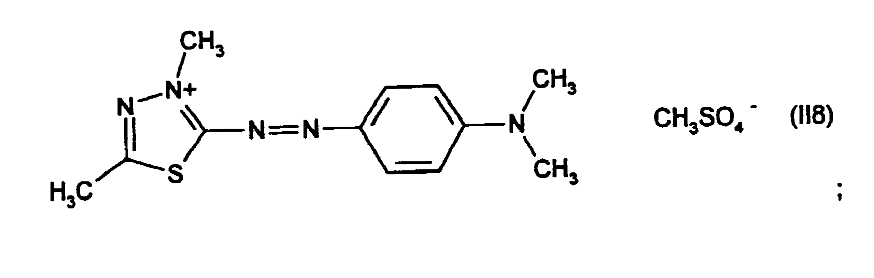 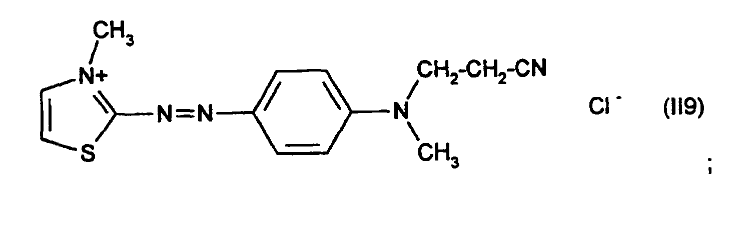 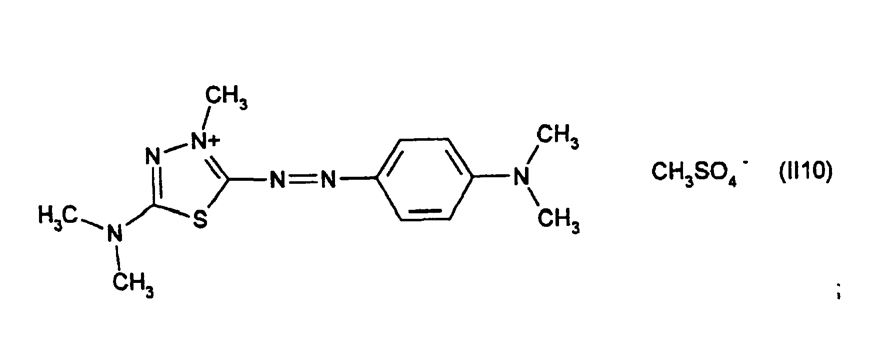 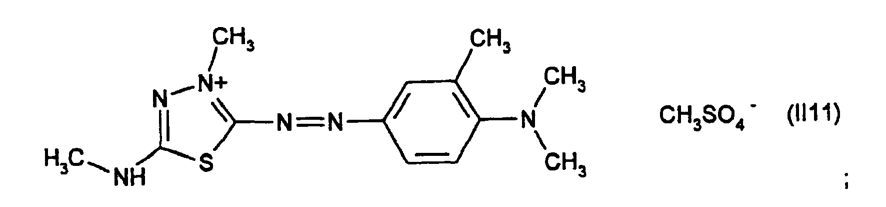 and

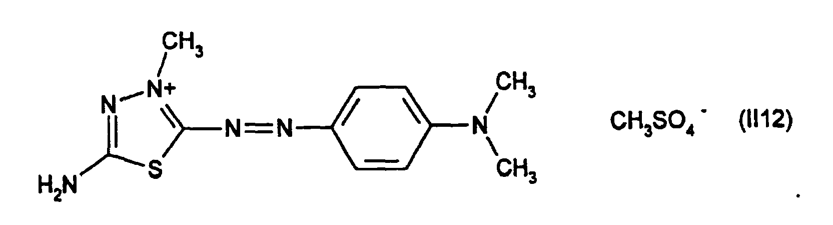

7. Composition according to Claim 4, **characterized in that** the cationic direct dyes of formula (III) are chosen from the compounds corresponding to structures (III1) to (III18) below: 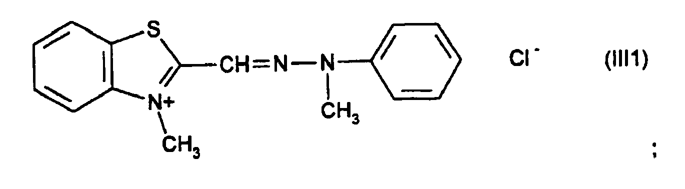 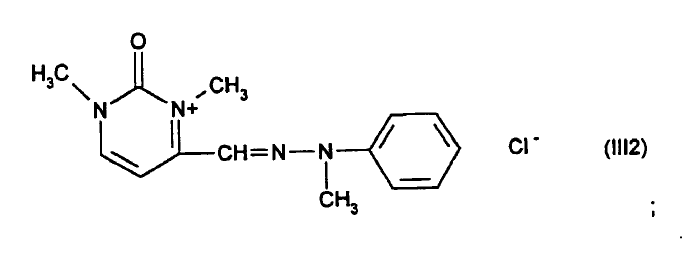 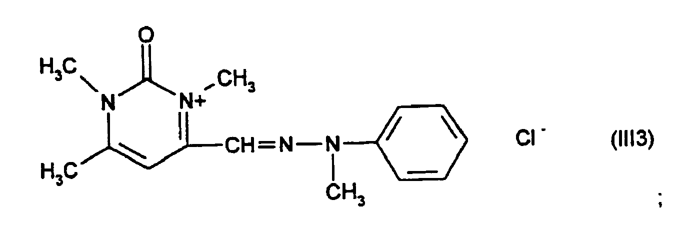 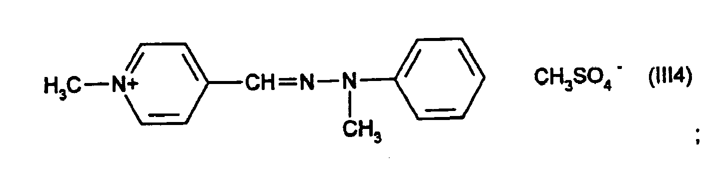 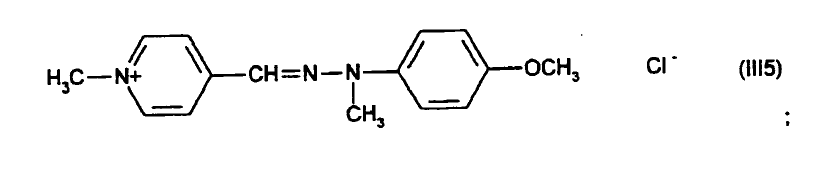 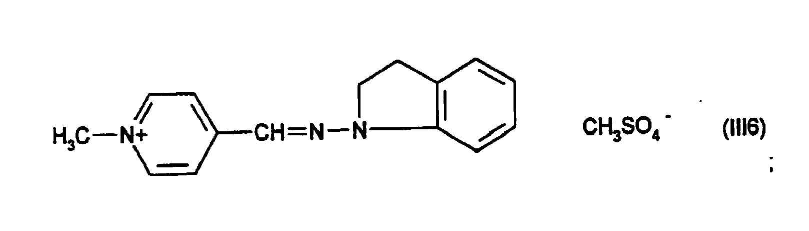 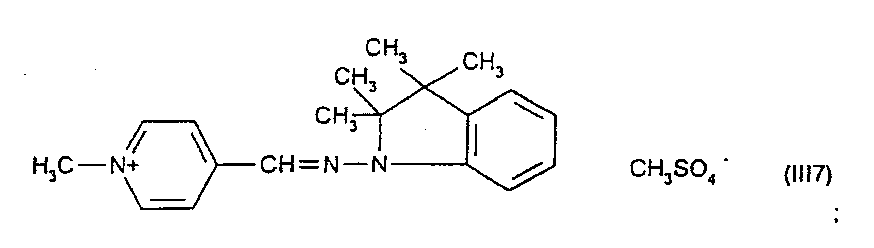 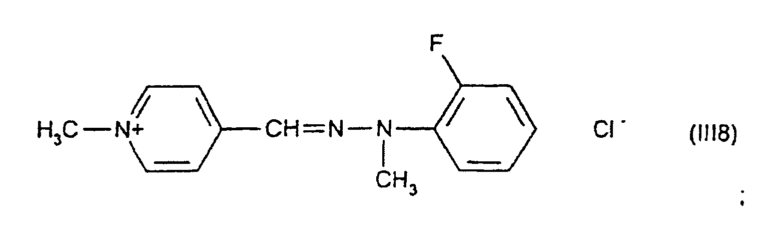 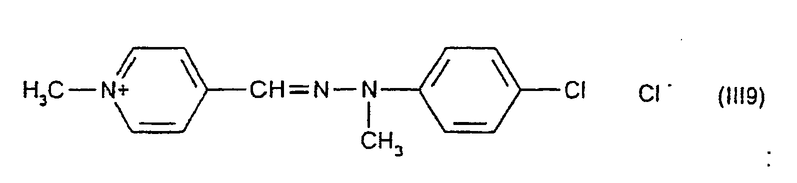 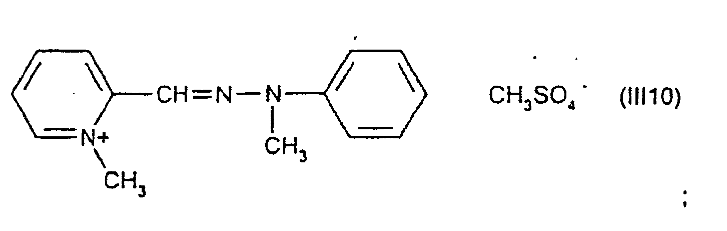 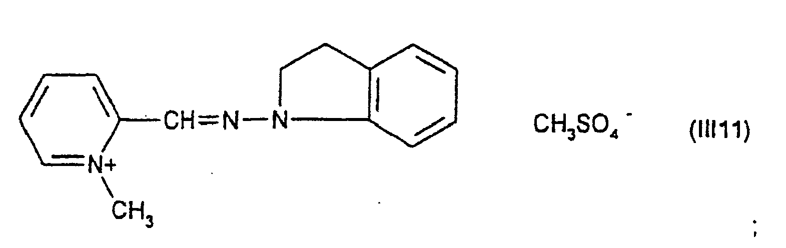 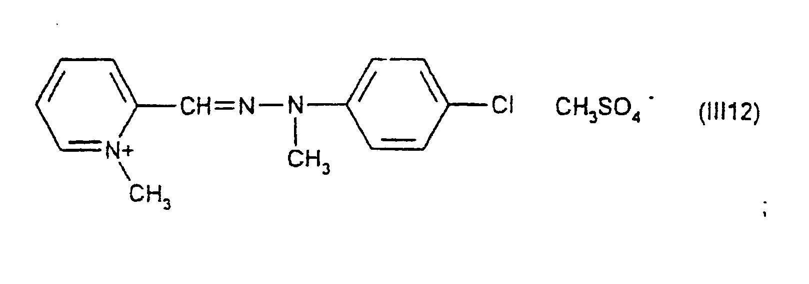 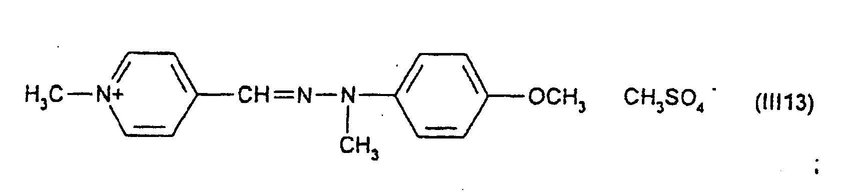 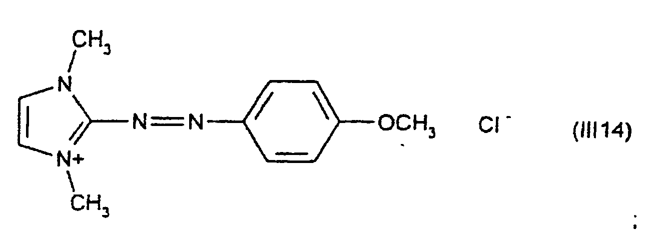 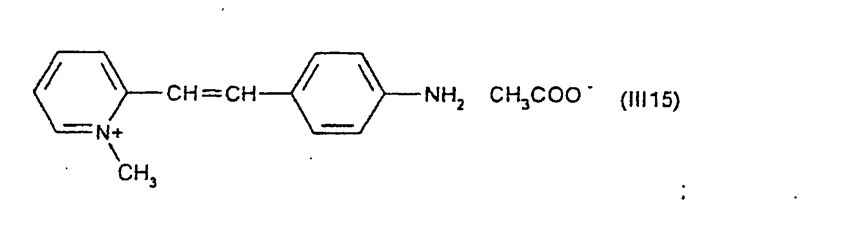 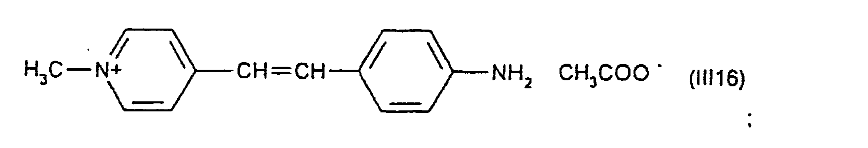 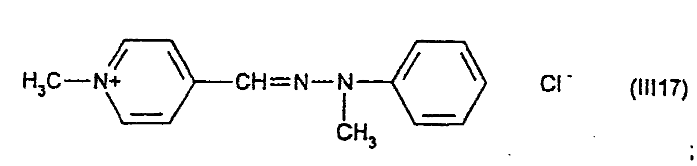 et

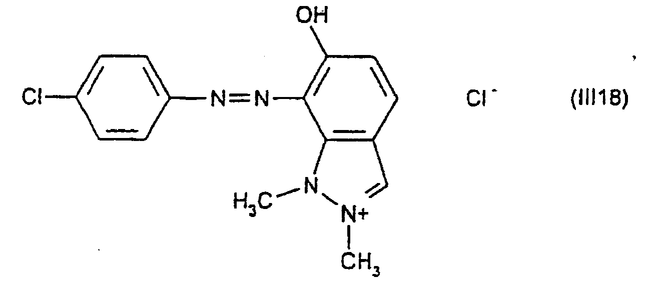

8. Composition according to Claim 4, **characterized in that** the cationic direct dyes of formula (III') are chosen from the compounds corresponding to structures (III'1) to (III'3) below: 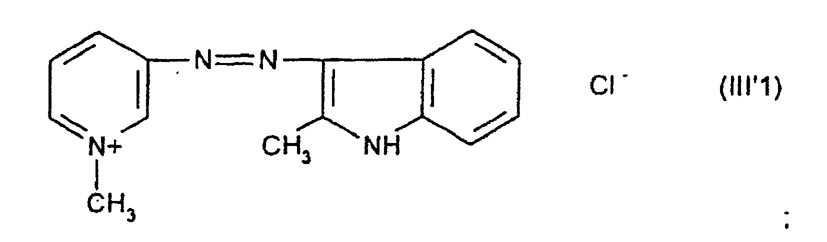 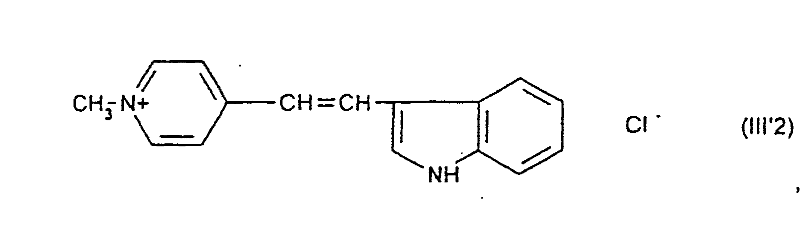 and

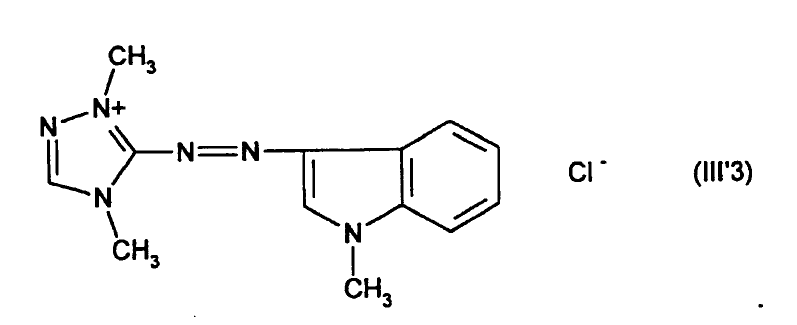

9. Composition according to any one of the preceding claims, **characterized in that** the cationic direct dye(s) represent(s) from 0.001 to 10% by weight relative to the total weight of the ready-to-use dye composition.

10. Composition according to Claim 9, **characterized in that** the cationic direct dye(s) represent(s) from 0.05 to 5% by weight relative to the total weight of the ready-to-use dye composition.

11. Composition according to any one of Claims 1 to 10 **characterized in that** the benzene auto-oxidizable dyes are chosen from the compounds of formula (IV) below, and the addition salts thereof with an acid: 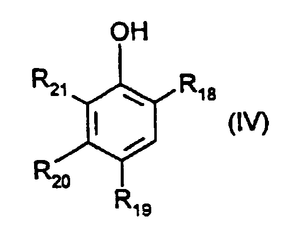 in which:
- R₁₈ represents a hydrogen atom, a C₁-C₄ alkyl radical or an amino radical,
- R₁₉ represents a C₁-C₄ alkyl, hydroxyl, amino, mono(C₁-C₄)alkylamino or di(C₁-C₄)alkylamino radical,
- R₂₀ represents a hydrogen atom or a hydroxyl or amino radical,
- R₂₁ represents a hydrogen atom or an amino radical;
it being understood that at least two of the radicals R₁₉ to R₂₁ represent, independently of each other, a hydroxyl, amino, mono(C₁-C₄)alkylamino or di (C₁-C₄) alkylamino radical.

12. Composition according to Claim 11, **characterized in that** the benzene auto-oxidizable dyes of formula (IV) are chosen from 1,2,4-trihydroxybenzene, 1-methyl-2,4,5-trihydroxybenzene, 2,4-diamino-6-methylphenol, 2-amino-4-methylaminophenol, 2,5-diamino-4-methylphenol, 2,6-diamino-4-diethyl-aminophenol and 2,6-diamino-1,4-dihydroxybenzene, and the addition salts thereof with an acid.

13. Composition according to any one of Claims 1 to 10, **characterized in that** the indole auto-oxidizable dyes are chosen from the compounds of formula (V) below: 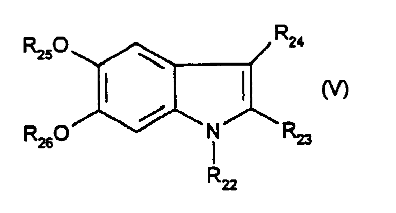 in which:
- R₂₂, R₂₄, R₂₅ and R₂₆, which may be identical or different, represent a hydrogen atom or a C₁-C₄ alkyl or C₁-C₄ acyl radical,
- R₂₃ represents a hydrogen atom, a C₁-C₄ alkyl radical or a carboxyl radical.

14. Composition according to Claim 13, **characterized in that** the auto-oxidizable dyes of formula (V) are chosen from 5,6-dihydroxyindole, 2-methyl-5,6-dihydroxyindole, 3-methyl-5,6-dihydroxyindole, 1-methyl-5,6-dihydroxyindole, 2,3-dimethyl-5,6-dihydroxyindole, 5-methoxy-6-hydroxyindole, 5-acetoxy-6-hydroxyindole, 5,6-diacetoxyindole and 5,6-dihydroxy-2-indolecarboxylic acid, and the addition salts thereof with an acid.

15. Composition according to any one of the preceding claims, **characterized in that** the auto-oxidizable dye(s) represent(s) from 0.0005 to 12% by weight relative to the total weight of the dye composition.

16. Composition according to Claim 15, **characterized in that** the auto-oxidizable dye(s) represent(s) from 0.005 to 8% by weight relative to the total weight of the dye composition.

17. Composition according to any one of the preceding claims, **characterized in that** it contains one or more oxidizing agents chosen from hydrogen peroxide, urea peroxide, alkali metal bromates, persalts such as perborates and persulfates, and enzymes.

18. Composition according to Claim 17, **characterized in that** the enzymes are chosen from peroxidases and two-electron oxidoreductases.

19. Composition according to Claim 18, **characterized in that** the two-electron oxidoreductases are chosen from pyranose oxidases, glucose oxidases, glycerol oxidases, lactate oxidases, pyruvate oxidases and uricases.

20. Composition according to Claim 18 or 19, **characterized in that** the 2-electron oxidoreductases are chosen from uricases of animal, microbiological or biotechnological origin.

21. Composition according to any one of Claims 18 to 20, **characterized in that** the 2-electron oxidoreductase(s) represent(s) from 0.01 to 20% by weight relative to the total weight of the ready-to-use dye composition.

22. Composition according to Claim 21, **characterized in that** the 2-electron oxidoreductase(s) represent(s) from 0.1 to 5% by weight relative to the total weight of the ready-to-use dye composition.

23. Composition according to any one of Claims 20 to 22, **characterized in that** it contains a donor (or substrate) for the said 2-electron oxidoreductase, chosen from uric acid and its salts.

24. Composition according to any one of Claims 17 to 23, **characterized in that** it contains one or more oxidation bases chosen from para-phenylenediamines, para-aminophenols, ortho-phenylenediamines and heterocyclic bases and/or one or more couplers chosen from meta-phenylenediamines, meta-aminophenols, meta-diphenols, heterocyclic couplers such as, for example, indole derivatives, indoline derivatives, benzimidazole derivatives, benzomorpholine derivatives, sesamol derivatives, pyridine, pyrimidine and pyrazole derivatives, and the addition salts thereof with an acid.

25. Composition according to Claim 24, **characterized in that** the oxidation base(s) represent(s) from 0.0005 to 12% by weight relative to the total weight of the ready-to-use dye composition and **in that** the coupler(s) represent(s) from 0.0001 to 10% by weight relative to the total weight of the ready-to-use dye composition.

26. Composition according to Claim 25, **characterized in that** the oxidation base(s) represent(s) from 0.005 to 8% by weight relative to the total weight of the ready-to-use dye composition and **in that** the coupler(s) represent(s) from 0.005 to 8% by weight relative to the total weight of the ready-to-use dye composition.

27. Composition according to Claims 11, 12, 14 to 16 and 24 to 26, **characterized in that** the addition salts with an acid are chosen from the hydrochlorides, hydrobromides, sulfates, tartrates, lactates and acetates.

28. Composition according to any one of the preceding claims, **characterized in that** the medium which is suitable for dyeing consists of water or of a mixture of water and at least one organic solvent.

29. Composition according to any one of the preceding claims, **characterized in that** it has a pH of between 5 and 11.

30. Composition according to any one of Claims 1 and 9 to 29, **characterized in that** the cationic direct dye is of the following formula:

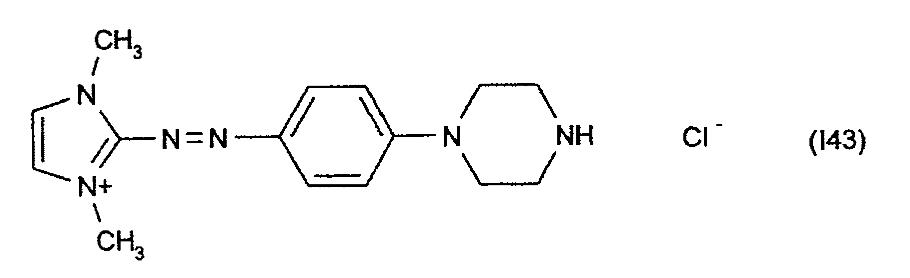

31. Process for dyeing keratin fibres, and in particular human keratin fibres such as the hair, **characterized in that** at least one ready-to-use dye composition as defined in any one of the preceding claims is applied to the said fibres for a period which is sufficient to develop the desired coloration.

32. Process according to Claim 31, **characterized in that** it includes a preliminary step which consists in separately storing, on the one hand, a composition (A) comprising, in a medium which is suitable for dyeing, at least one cationic direct dye, and at least one auto-oxidizable dye, and, on the other hand, a composition (B) containing, in a medium which is suitable for dyeing, at least one oxidizing agent, and then in mixing them together at the time of use, before applying this mixture to the keratin fibres.

33. Multi-compartment dyeing device or "kit", **characterized in that** it includes a first compartment containing composition (A) as defined in Claim 32 and a second compartment containing composition (B) as defined in Claim 32.

## Patentansprüche

1. Gebrauchsfertige Zusammensetzung für die Färbung von Keratinfasern und insbesondere von menschlichen Keratinfasern, wie den Haaren, **dadurch gekennzeichnet, dass** sie in einem für die Färbung geeigneten Medium enthält:
- mindestens einen kationischen direktziehenden Farbstoff und
- mindestens einen selbstoxidierenden Benzol- oder Indolfarbstoff.

2. Zusammensetzung nach Anspruch 1, **dadurch gekennzeichnet, dass** der oder die kationischen direktziehenden Farbstoffe unter den kationischen Aminoanthrachinonfarbstoffen, den kationischen Mono- oder Diazofarbstoffen, den kationischen Naphthochinonen ausgewählt sind.

3. Zusammensetzung nach Anspruch 2, **dadurch gekennzeichnet, dass** der oder die kationischen direktziehenden Farbstoffe ausgewählt sind unter: [8-[(p-Aminophenyl)-azo]-7-hydroxy-2-naphthyl]-trimethylammoniumchlorid, 3-[(4-Amino-6-brom-5,8-dihydro-1-hydroxy-8-imino-5-oxo-2-naphthalinyl)-amino]-N,N,N-trimethylbenzolaminiumchlorid, 7-Hydroxy-8-[(2-methoxyphenyl)-azo]-N,N,N-trimethyl-2-naphthalinaminiumchlorid, [8-[(4-Amino-2-nitrophenyl)-azo]-7-hydroxy-2-naphthyl]-trimethylammoniumchlorid und 3-[(4,5-Dihydro-3-methyl-5-oxo-1-phenyl-1H-pyrazol-4-yl)-azo]-N,N,N-trimethylbenzolaminiumchlorid.

4. Zusammensetzung nach Anspruch 1, **dadurch gekennzeichnet, dass** der oder die kationischen direktziehenden Farbstoffe ausgewählt sind unter
a) den Verbindungen der folgenden Formel (I): 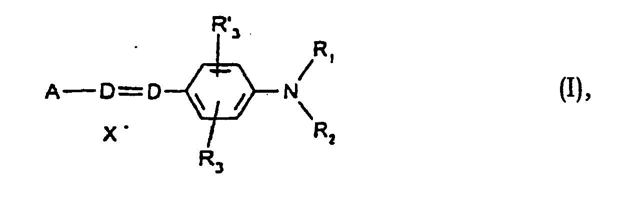 in der bedeuten:
- D ein Stickstoffatom oder die Gruppe -CH,
- R₁ und R₂, gleich oder verschieden, Wasserstoff, C₁₋₄-Alkyl, das mit einem Rest -CN, -OH oder NH₂ substituiert sein kann; oder Reste, die mit einem Kohlenstoffatom des Benzolrings einen Heterocyclus bilden, der gegebenenfalls Sauerstoff oder Stickstoff enthält, der mit einem oder mehreren C₁₋₄-Alkylresten substituiert sein kann; 4'-Aminophenyl,
- R₃ und R'₃, gleich oder verschieden, Wasserstoff oder ein Halogenatom, das unter Chlor, Brom, Iod und Flor ausgewählt ist, Cyano, C₁₋₄-Alkoxy oder Acetyloxy,
- X⁻ ein Anion, das vorzugsweise unter Chlorid, Methylsulfat und Acetat ausgewählt ist,
- A eine Gruppe, die unter den folgenden Strukturen A1 bis A 18 ausgewählt ist: 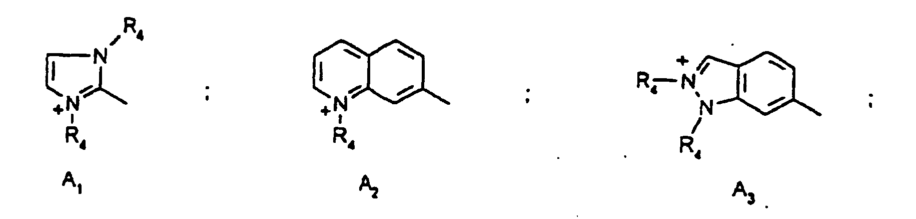 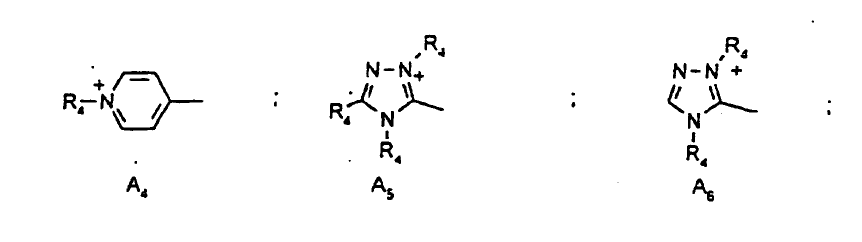 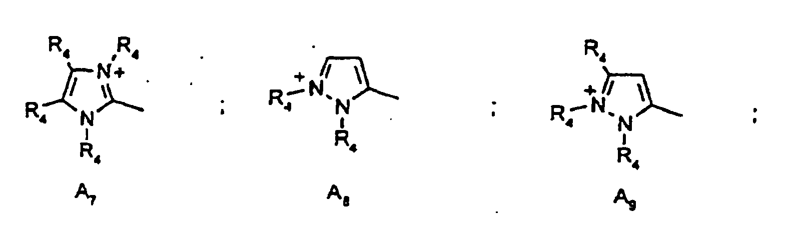 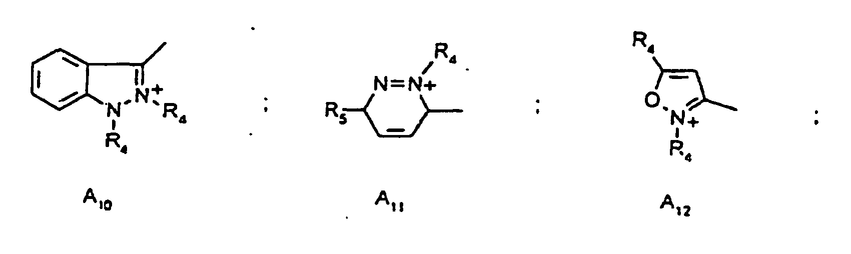 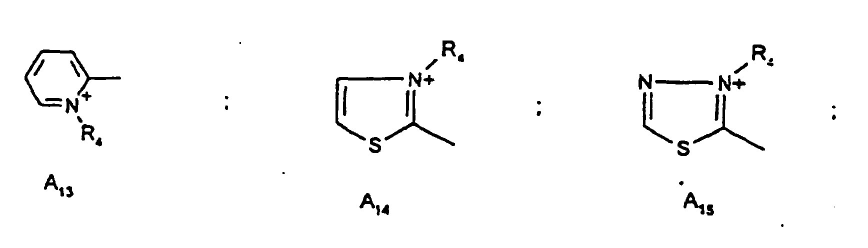 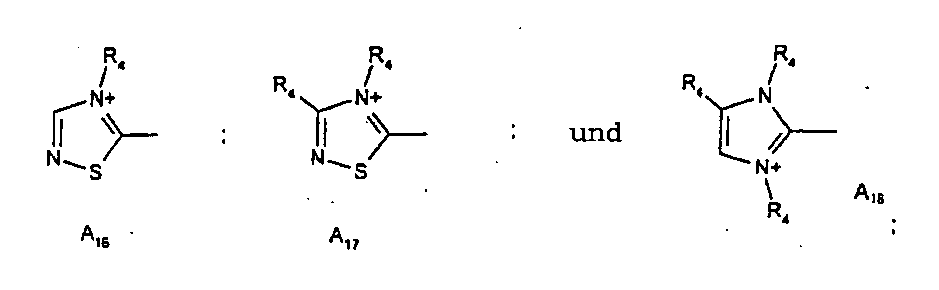 in denen R₄ C₁₋₄-Alkyl, das mit einer Hydroxygruppe substituiert sein kann, bedeutet und R₅ C₁₋₄-Alkoxy bedeutet,
mit der Maßgabe, dass R₁ und R₂ nicht gleichzeitig Wasserstoff bedeuten, wenn D -CH bedeutet, A A₄ oder A₁₃ bedeutet und R₃ verschieden von Alkoxy ist;
b) den Verbindungen der folgenden Formel (II) 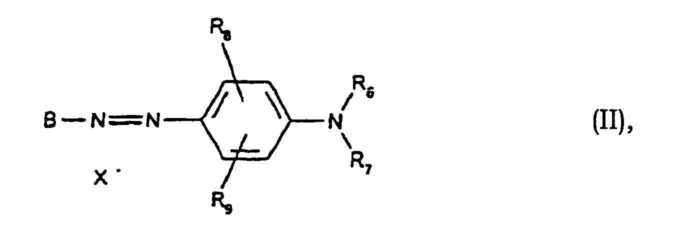 in der bedeuten:
- R₆ Wasserstoff oder C₁₋₄-Alkyl,
- R₇ Wasserstoff, Alkyl, das mit einer Gruppe -CN oder einer Aminogruppe substituiert sein kann, 4'-Aminophenyl oder einen Rest, der mit R₆ einen Heterocyclus bildet, der gegebenenfalls Sauerstoff und/oder Stickstoff enthält, der mit C₁₋₄-Alkyl substituiert sein kann,
- R₈ und R₉, gleich oder verschieden, Wasserstoff, ein Halogenatom, wie Brom, Chlor, Iod oder Fluor, C₁₋₄-Alkyl oder C₁₋₄-Alkoxy, eine Gruppe -CN,
- X⁻ ein Anion, das vorzugsweise unter Chlorid, Methylsulfat und Acetat ausgewählt ist,
- B eine Gruppe, die unter den folgenden Strukturen B1 bis B6 ausgewählt ist: 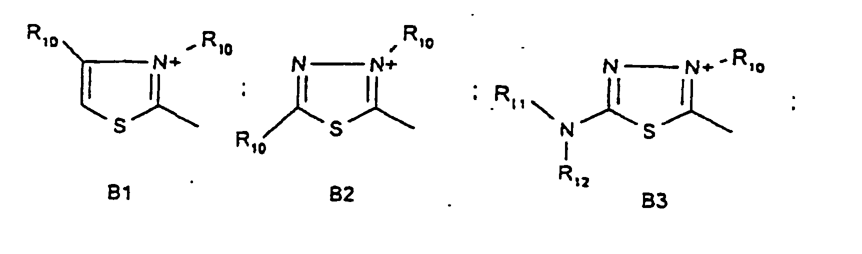 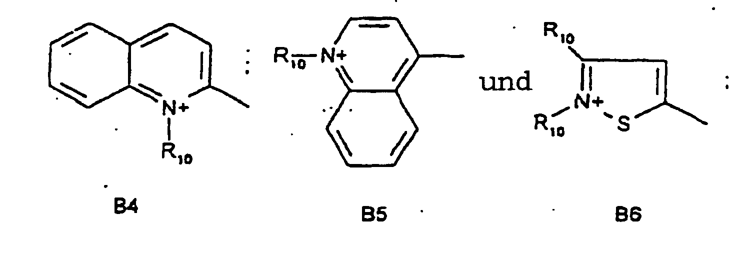 in denen R₁₀ C₁₋₄-Alkyl bedeutet, R₁₁ und R₁₂, gleich oder verschieden, Wasserstoff oder C₁₋₄-Alkyl bedeuten;
c) den Verbindungen der folgenden Formeln (III) und (III'): 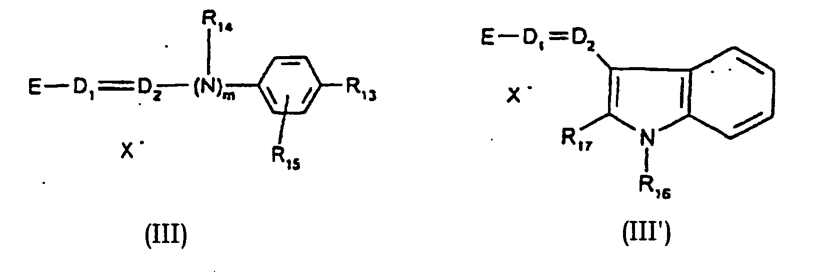 in denen bedeuten:
- R₁₃ Wasserstoff, C₁₋₄-Alkoxy, ein Halogenatom, wie Brom, Chlor, Iod oder Fluor, eine Aminogruppe,
- R₁₄ Wasserstoff, C₁₋₄-Alkyl oder einen Rest, der mit einem der Kohlenstoffatome des Benzolrings einen Heterocyclus bildet, der gegebenenfalls Sauerstoff enthält und/oder gegebenenfalls mit einer oder mehren C₁₋₄-Alkylgruppen substituiert ist;
- R₁₅ Wasserstoff oder ein Halogenatom, wie Brom, Chlor, Iod oder Fluor,
- R₁₆ und R₁₇, gleich oder verschieden, Wasserstoff oder C₁₋₄-Alkyl,
- D₁ und D₂, gleich oder verschieden, ein Stickstoffatom oder die Gruppe -CH,
- m = 0 oder 1,
mit der Maßgabe, dass D₁ und D₂ gleichzeitig eine Gruppe -CH darstellen und m gleich 0 ist, wenn R₁₃ eine nicht-substituierte Aminogruppe darstellt,
- X⁻ ein Anion, das vorzugsweise unter Chlorid, Methylsulfat und Acetat ausgewählt ist,
- E eine Gruppe, die unter den folgenden Strukturen E1 bis E8 ausgewählt ist 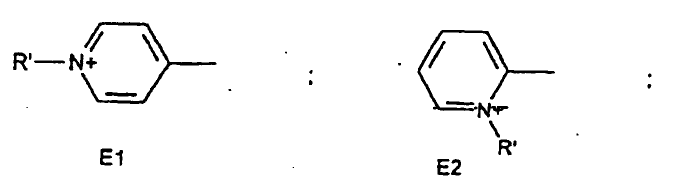 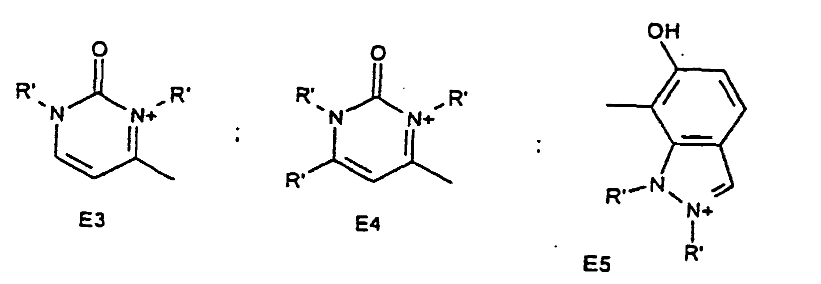 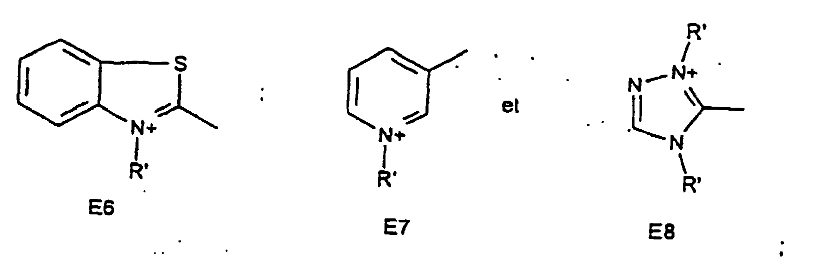 in denen R' C₁₋₄-Alkyl bedeutet;
und wenn m = 0 ist und D₁ ein Stickstoffatom bedeutet, kann E außerdem eine Gruppe der folgenden Struktur E9 darstellen: 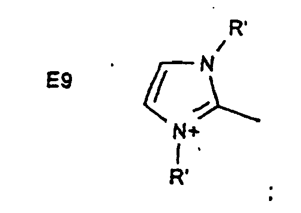 in der R' C₁₋₄-Alkyl bedeutet.

5. Zusammensetzung nach Anspruch 4, **dadurch gekennzeichnet, dass** die kationischen direktziehenden Farbstoffe der Formel (I) unter den Verbindungen ausgewählt sind, die den folgenden Strukturen (I1) bis (I52) entsprechen:

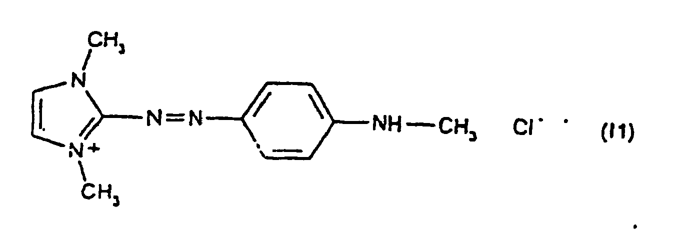

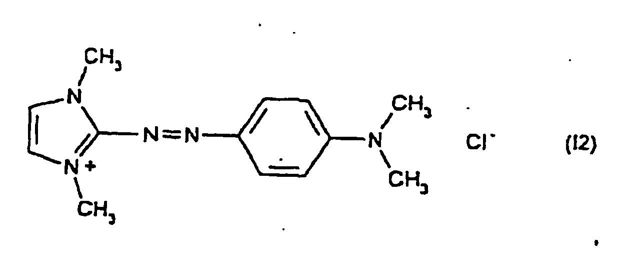

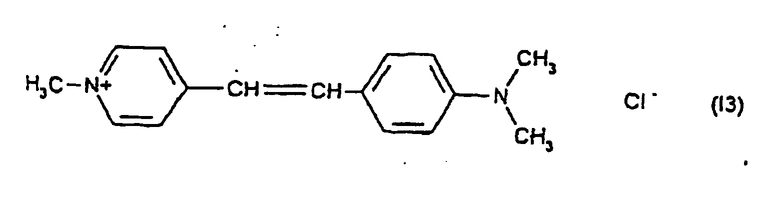

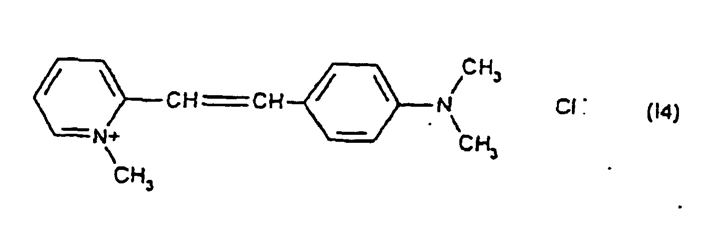

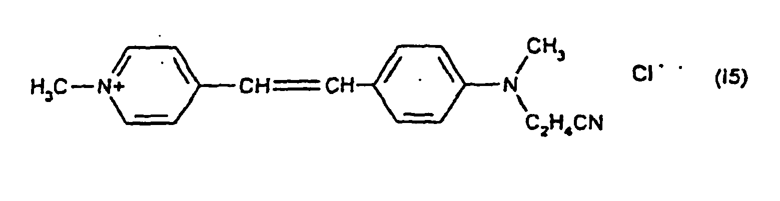

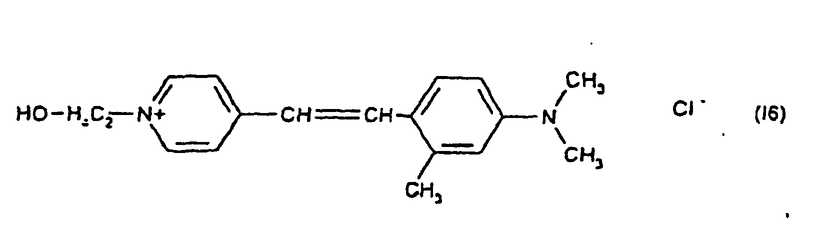

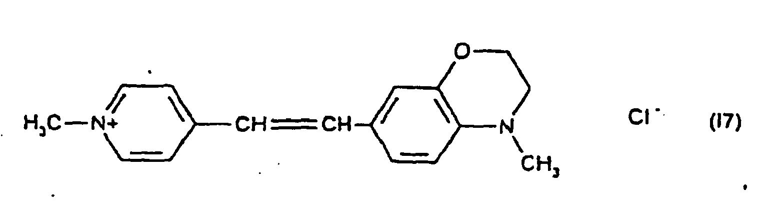

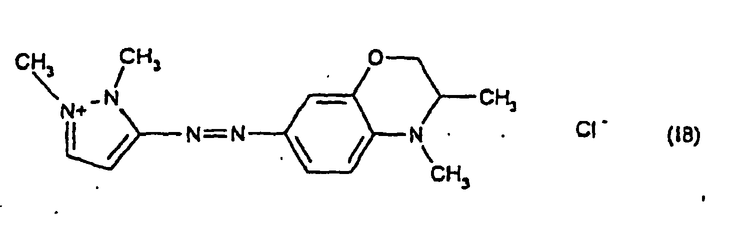

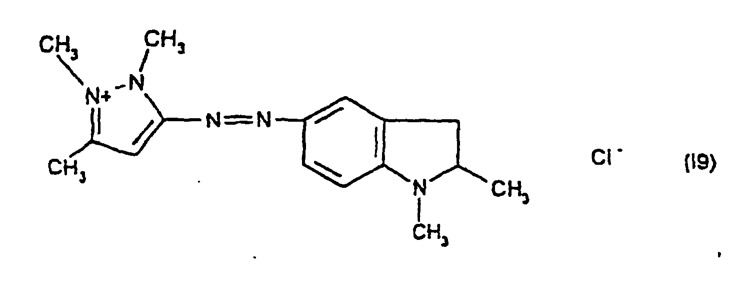

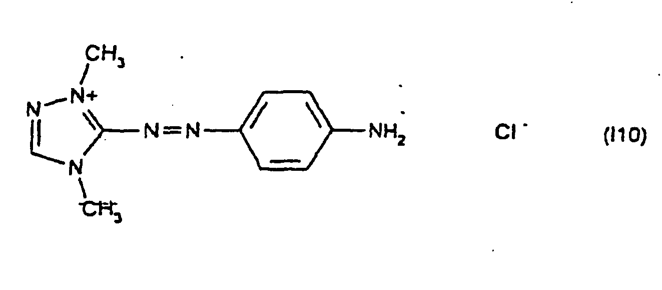

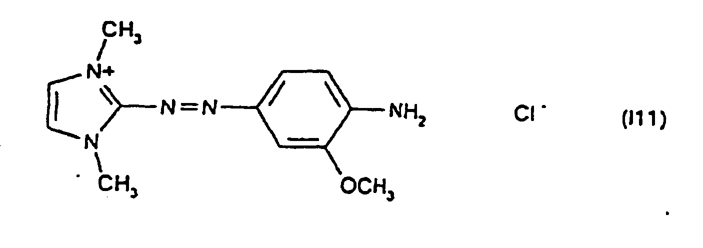

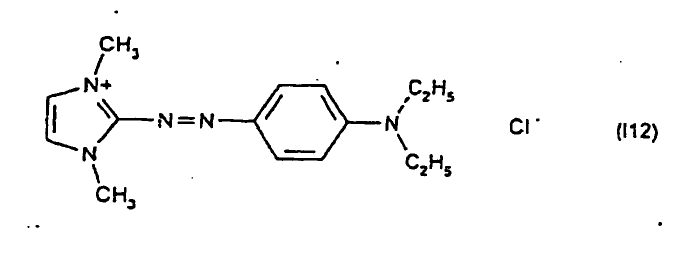

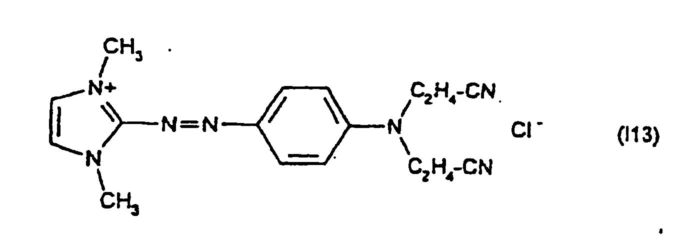

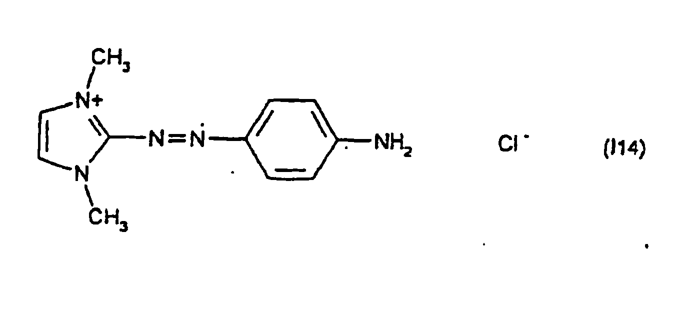

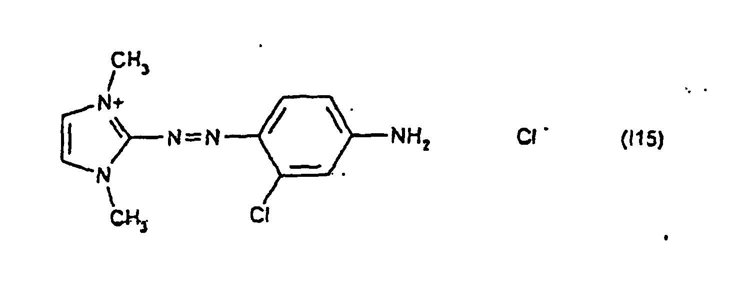

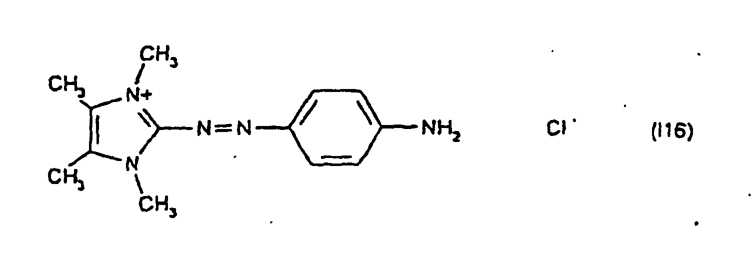

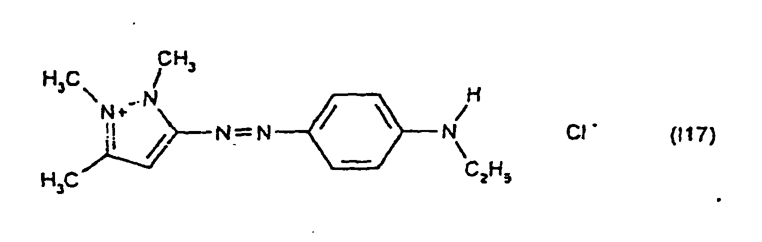

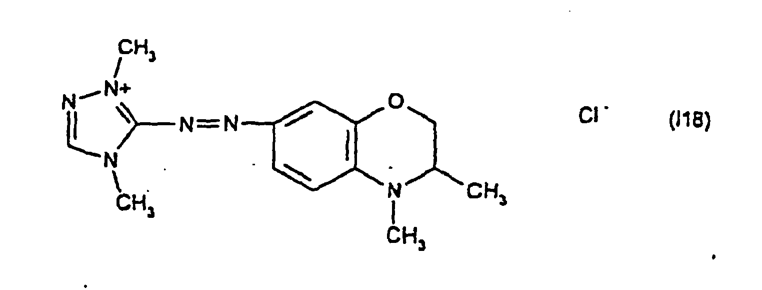

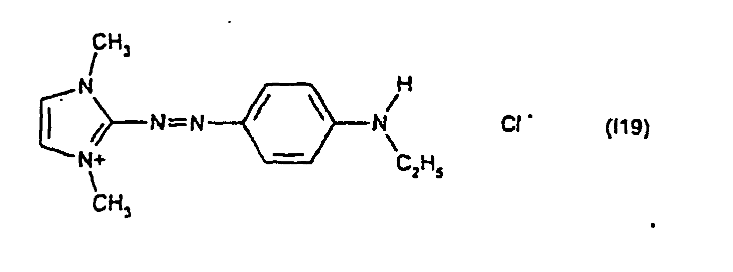

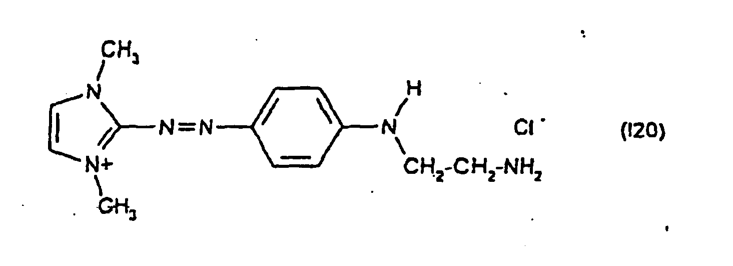

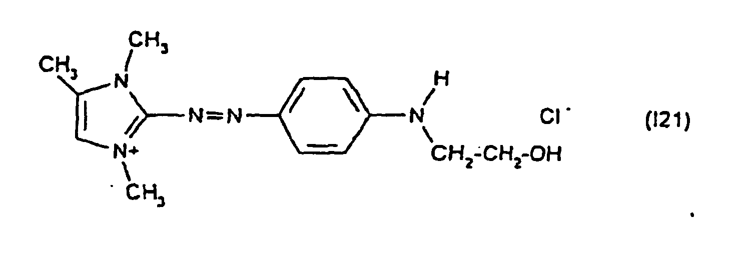

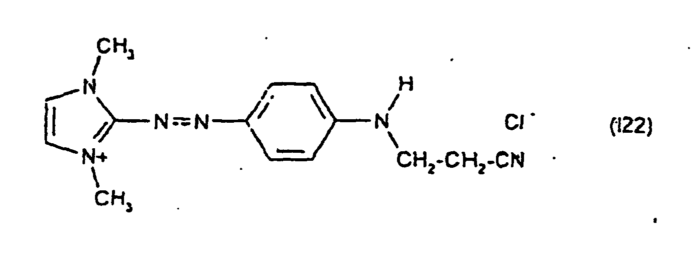

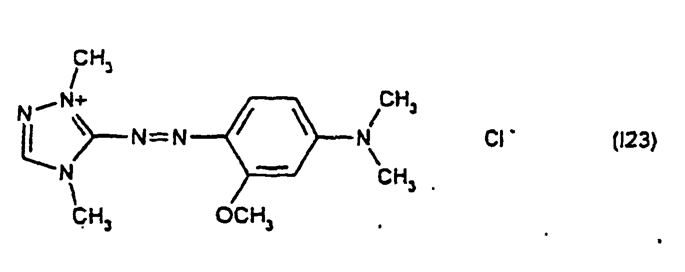

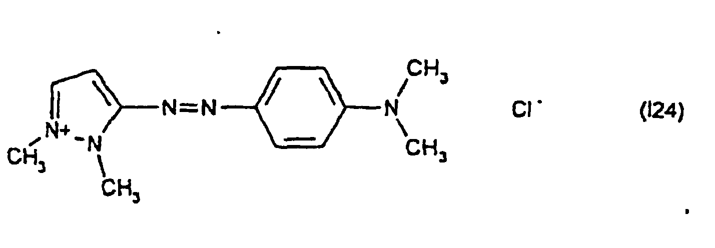

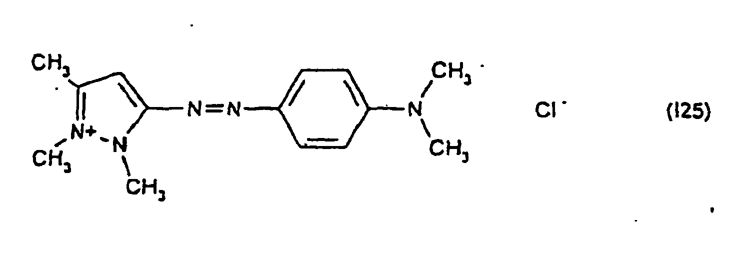

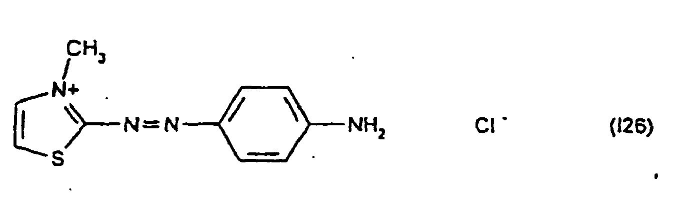

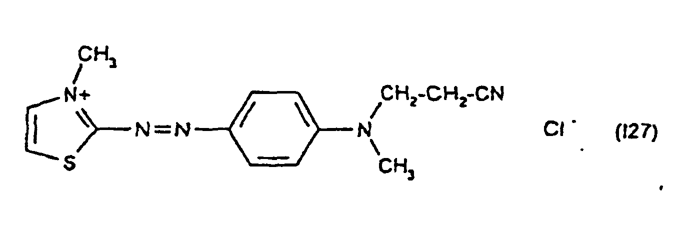

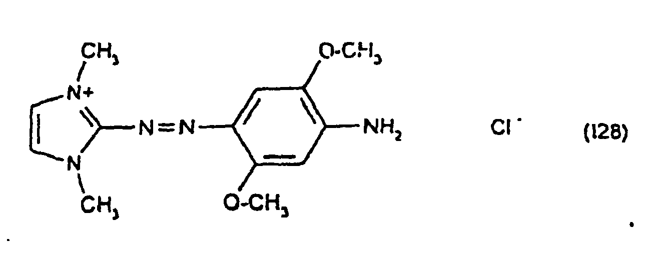

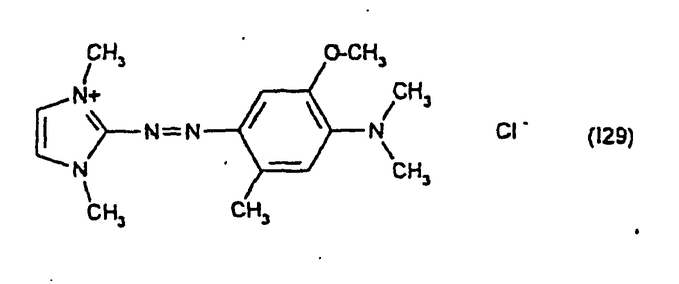

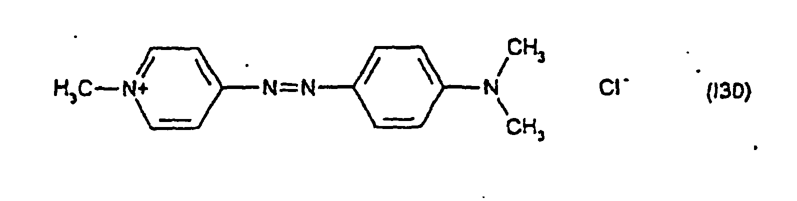

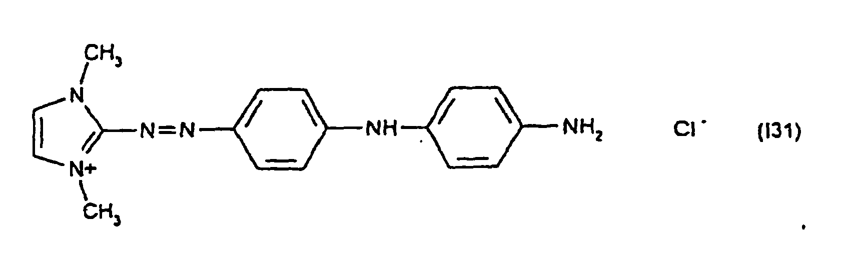

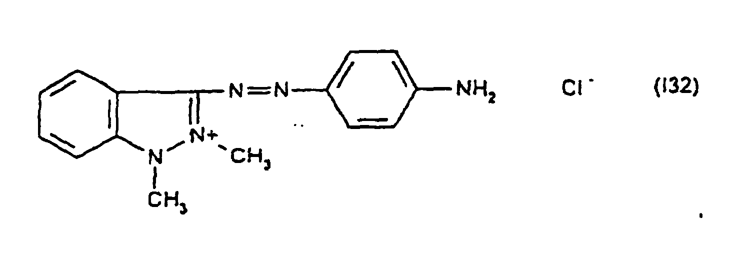

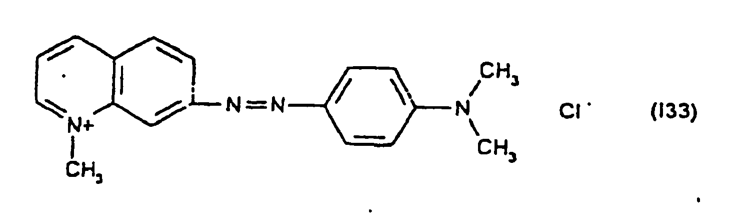

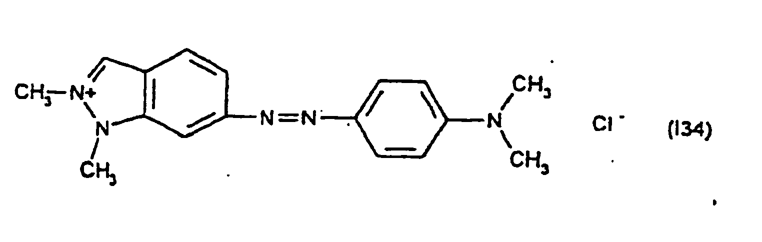

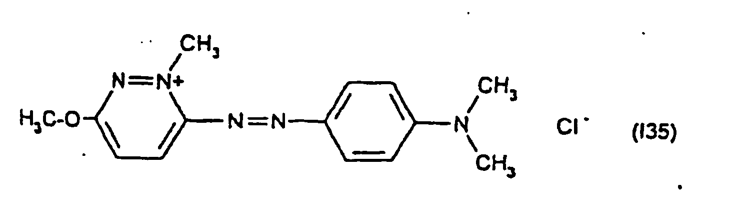

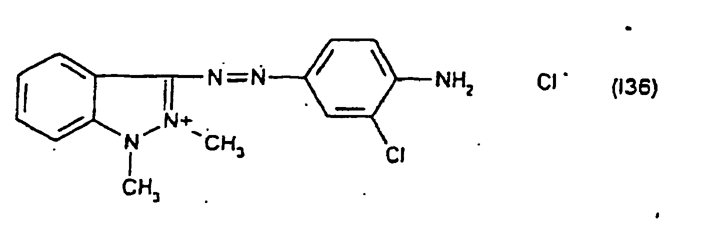

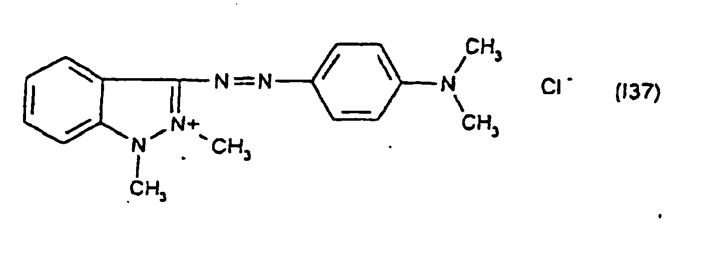

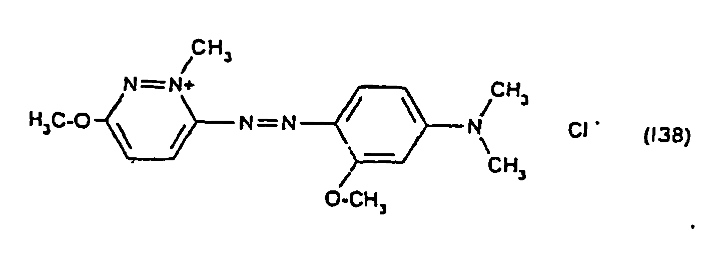

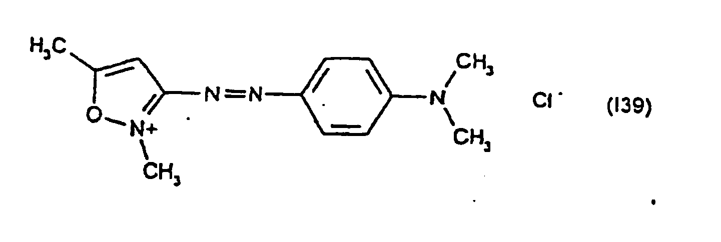

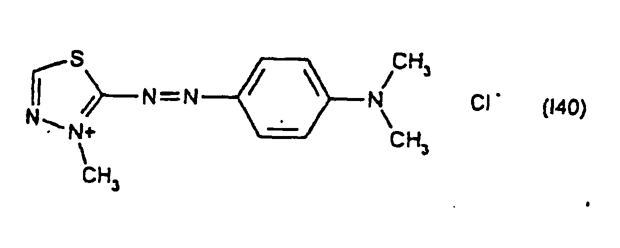

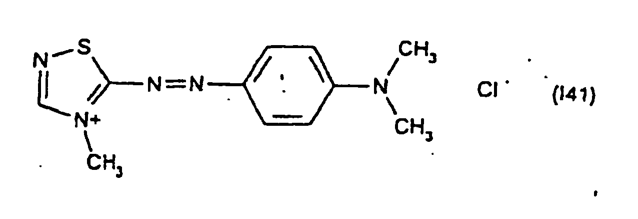

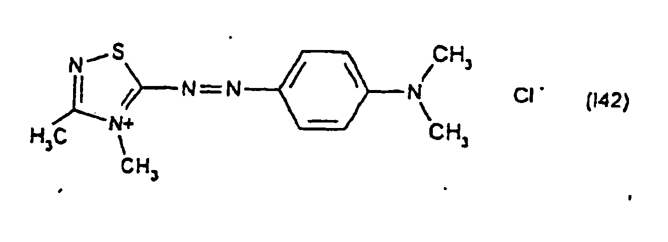

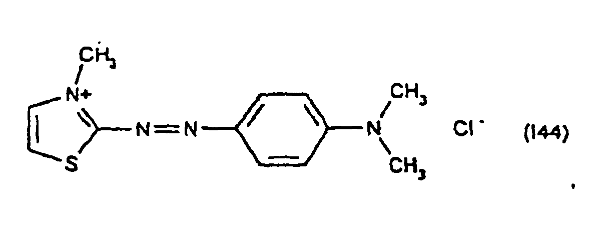

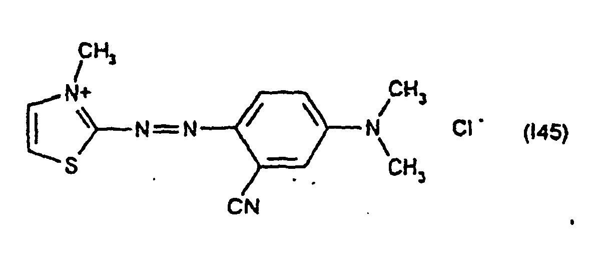

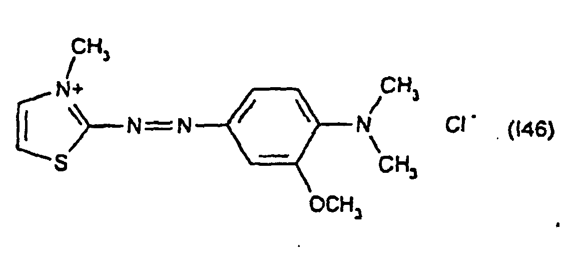

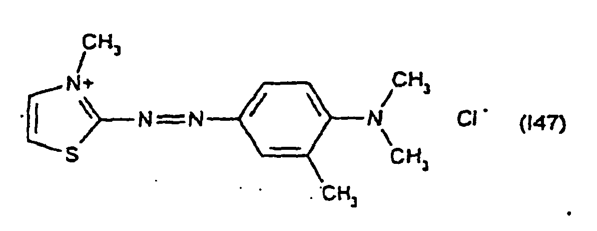

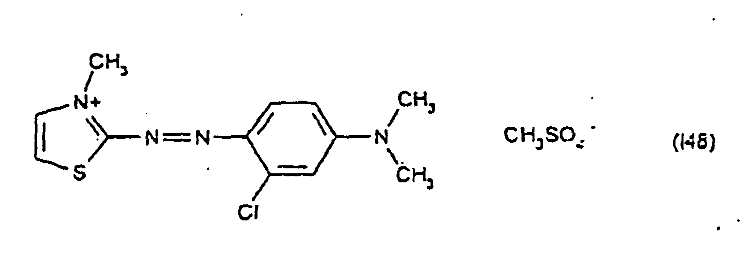

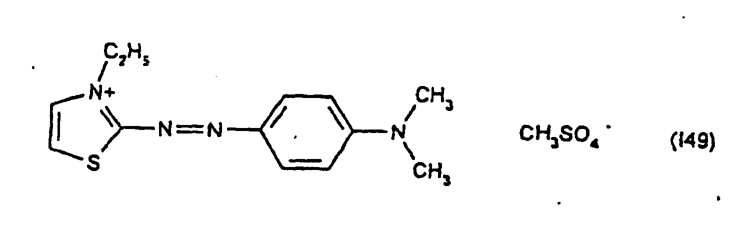

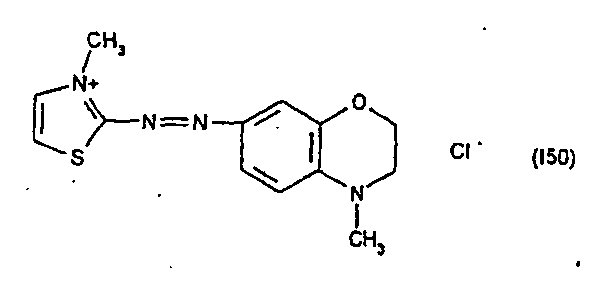

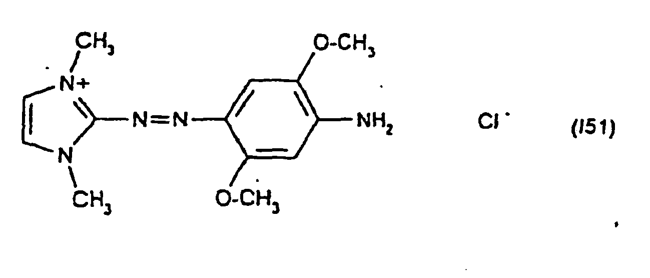

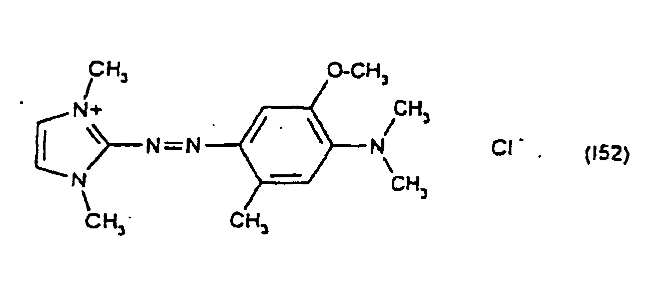

6. Zusammensetzung nach Anspruch 4, **dadurch gekennzeichnet, dass** die kationischen direktziehenden Farbstoffe der Formel (II) unter den Verbindungen ausgewählt sind, die den folgenden Strukturen (II1) bis (II12) entsprechen: 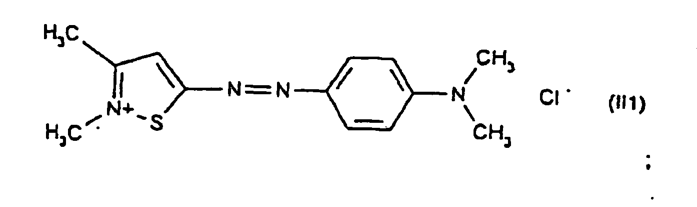 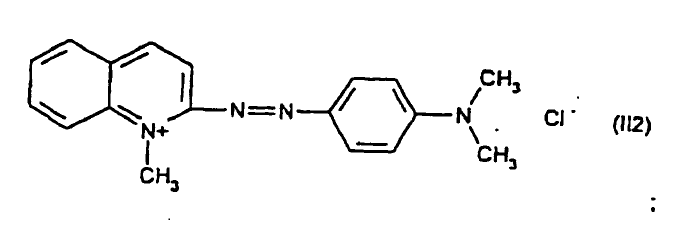 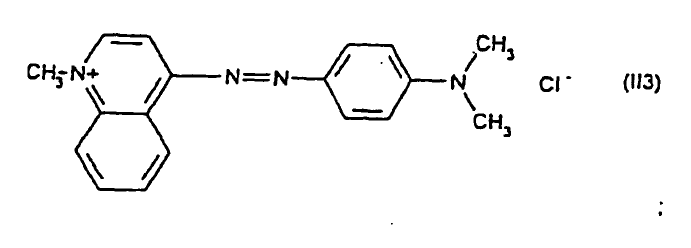 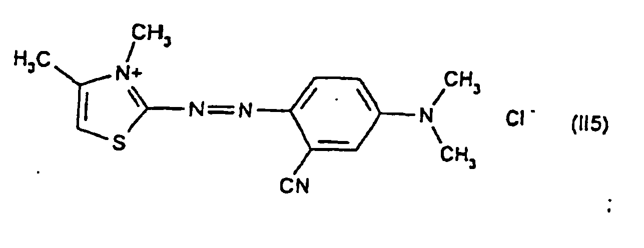 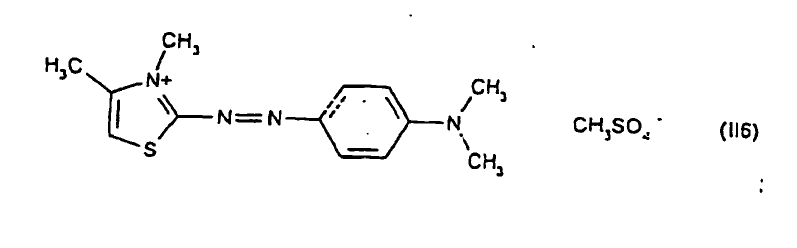 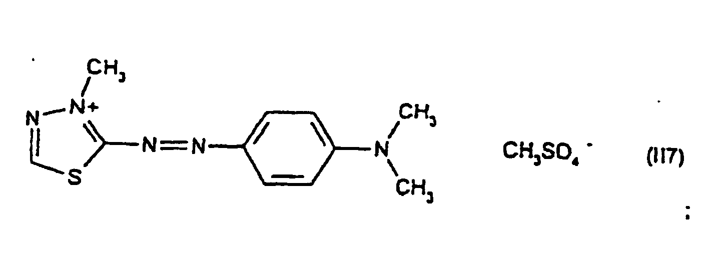 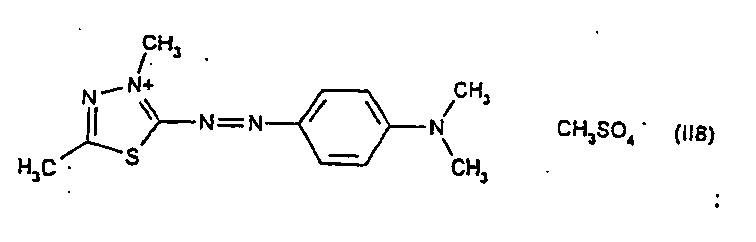 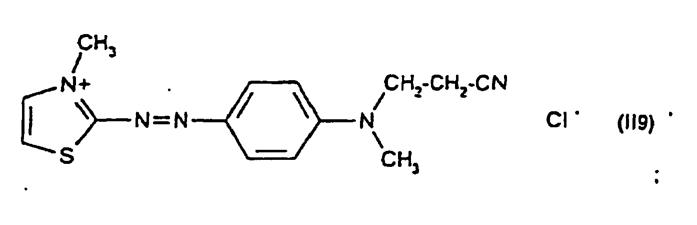 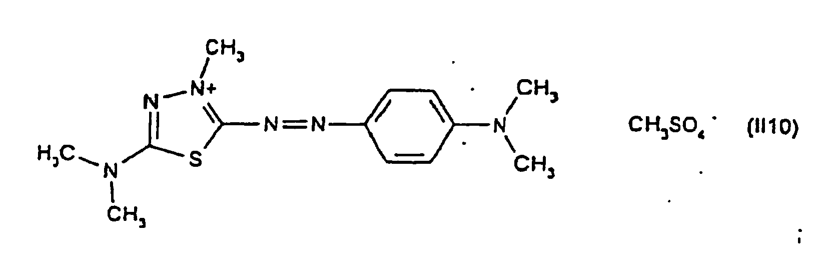 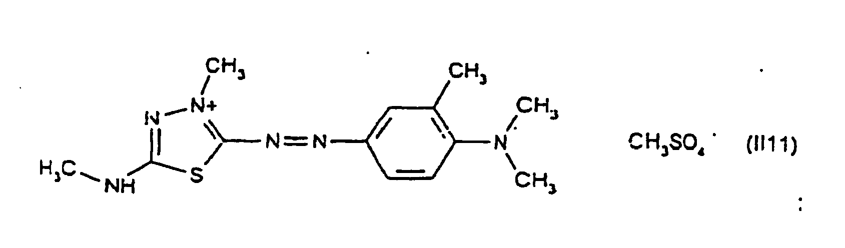 und

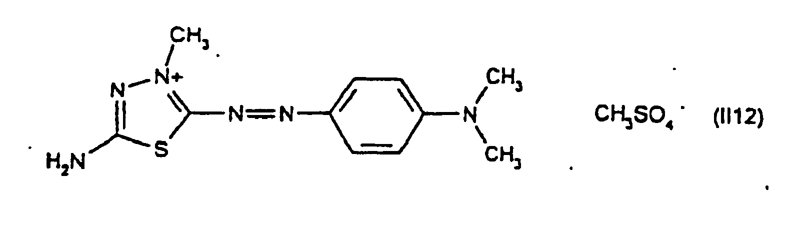

7. Zusammensetzung nach Anspruch 4, **dadurch gekennzeichnet, dass** die kationischen direktziehenden Farbstoffe der Formel (III) unter den Verbindungen ausgewählt sind, die den folgenden Strukturen (III1) bis (III18) entsprechen: 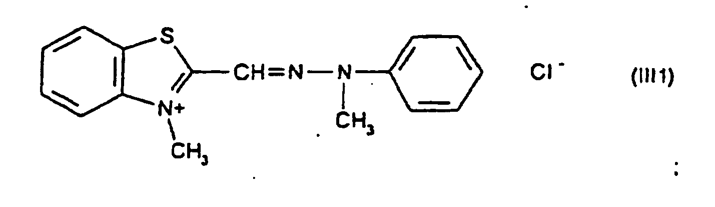 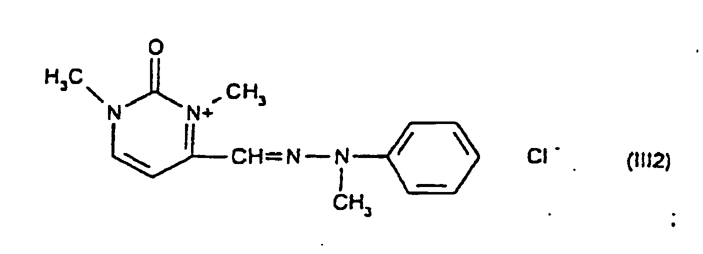 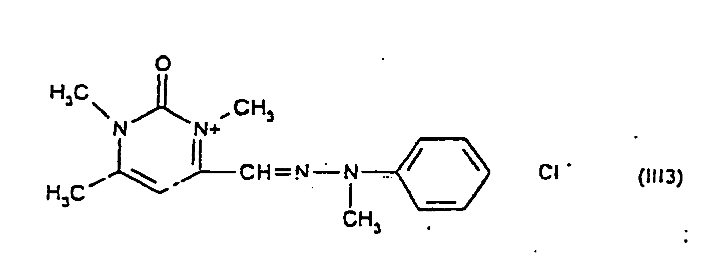 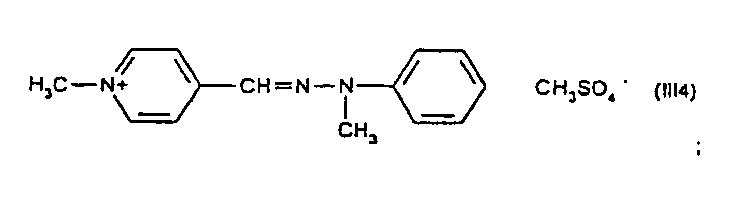 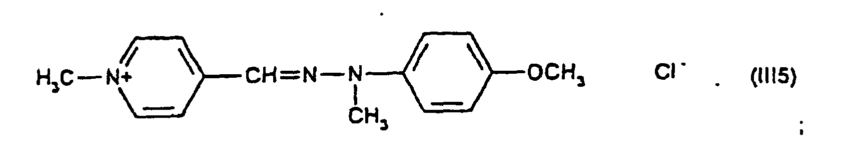 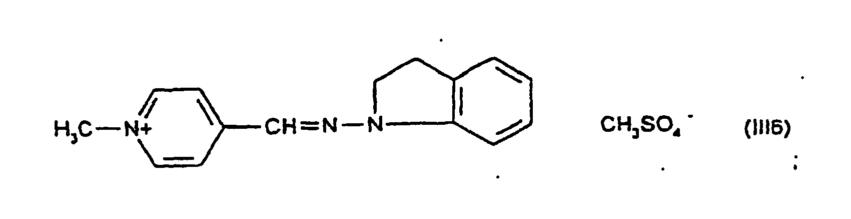 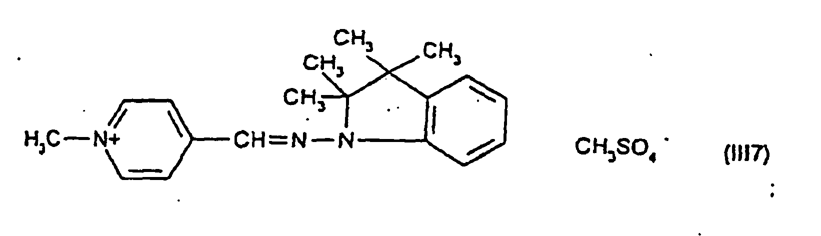 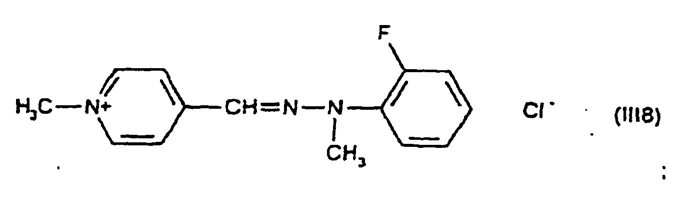 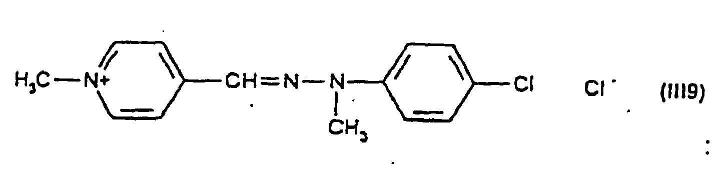 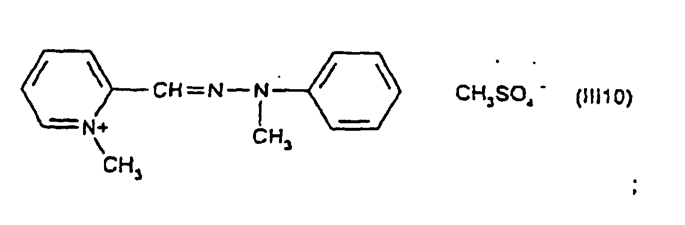 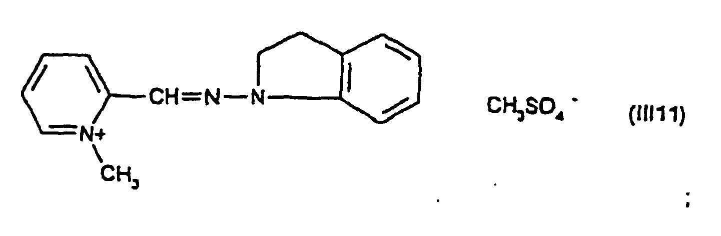 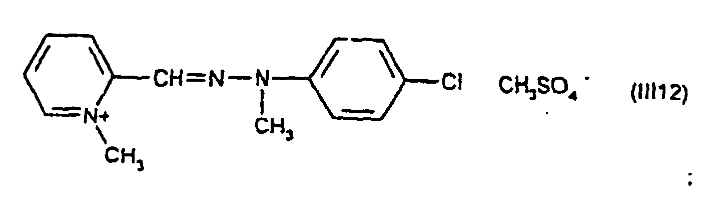 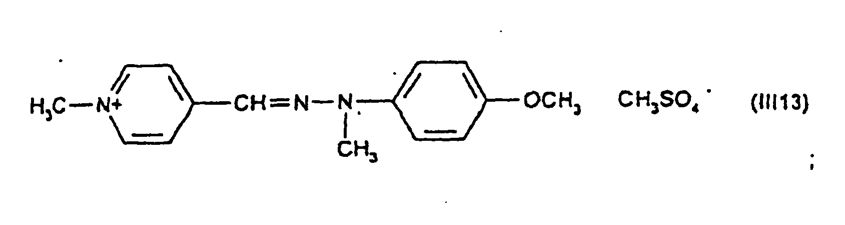 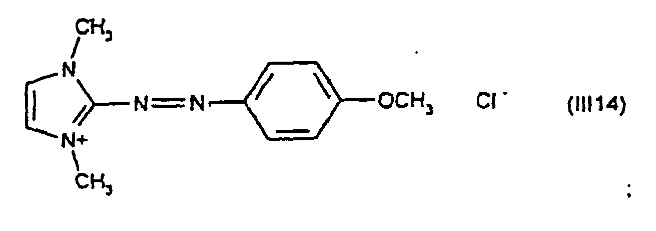 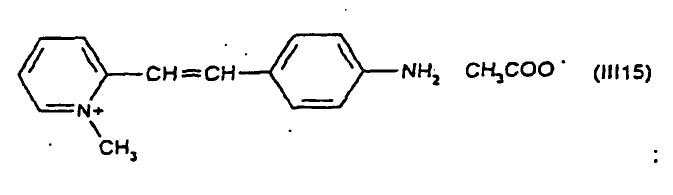 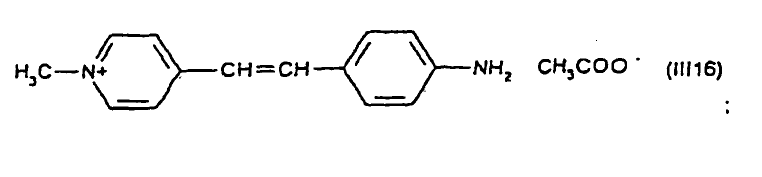 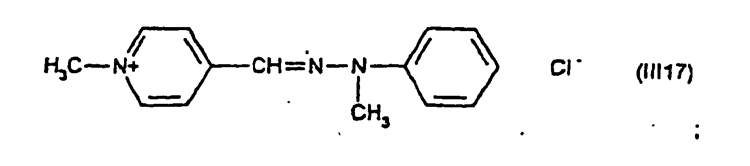 und

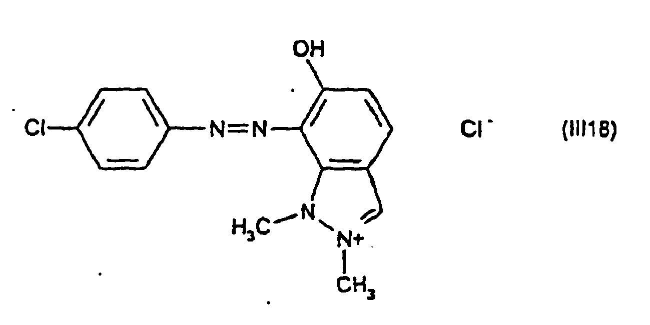

8. Zusammensetzung nach Anspruch 4, **dadurch gekennzeichnet, dass** die kationischen direktziehenden Farbstoffe der Formel (III') unter den Verbindungen ausgewählt sind, die den folgenden Strukturen (I-II'1) bis (III'3) entsprechen: 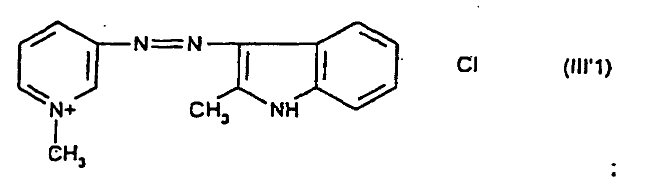 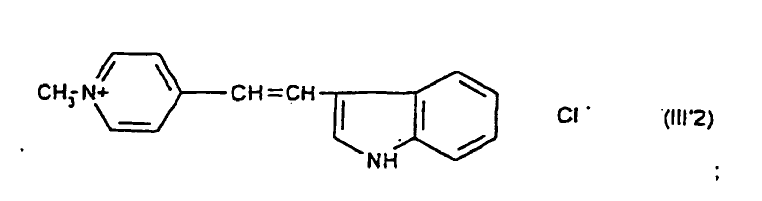 und

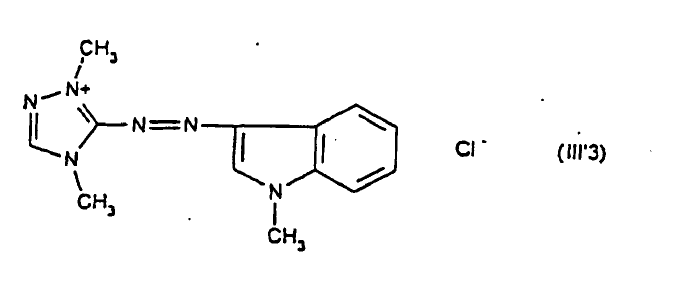

9. Zusammensetzung nach einem der vorhergehenden Ansprüche, **dadurch gekennzeichnet, dass** der Anteil des oder der kationischen direktziehenden Farbstoffe im Bereich von 0,001 bis 10 Gew.-% des Gesamtgewichts der gebrauchsfertigen Färbemittelzusammensetzung liegt.

10. Zusammensetzung nach Anspruch 9, **dadurch gekennzeichnet, dass** der Anteil des oder der kationischen direktziehenden Farbstoffe im Bereich von 0,05 bis 5 Gew.-% des Gesamtgewichts der gebrauchsfertigen Färbemittelzusammensetzung liegt.

11. Zusammensetzung nach einem der Ansprüche 1 bis 10, **dadurch gekennzeichnet, dass** die selbstoxidierenden Benzolfarbstoffe unter den Verbindungen der folgenden Formel (IV) und ihren Additionssalzen mit einer Säure ausgewählt sind, 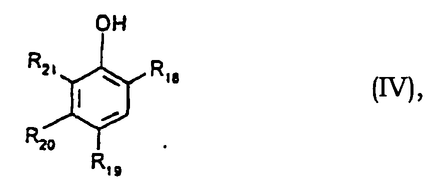 in der bedeuten:
- R₁₈ Wasserstoff, C₁₋₄-Alkyl oder eine Aminogruppe,
- R₁₉ C₁₋₄-Alkyl, Hydroxy, Amino, Mono-(C₁₋₄)-alkylamino oder Di-(C₁₋₄)-alkylamino,
- R₂₀ Wasserstoff, eine Hydroxygruppe oder Aminogruppe,
- R₂₁ Wasserstoff oder eine Aminogruppe,
mit der Maßgabe, dass mindestens zwei der Reste R₁₉ bis R₂₁ unabhängig voneinander eine Hydroxygruppe, Aminogruppe, Mono-(C₁₋₄)-alkylamino oder Di-(C₁₋₄)-alkylamino bedeuten.

12. Zusammensetzung nach Anspruch 11, **dadurch gekennzeichnet, dass** die selbstoxidierenden Benzolfarbstoffe der Formel (IV) ausgewählt sind unter 1,2,4-Trihydroxybenzol, 1-Methyl-2,4,5-trihydroxybenzol, 2,4-Diamino-6-methylphenol, 2-Amino-4-methylaminophenol, 2,5-Diamino-4-methylphenol, 2,6-Diamino-4-diethylaminophenol, 2,6-Diamino-1,4-dihydroxybenzol und ihren Additionssalzen mit einer Säure.

13. Zusammensetzung nach einem der Ansprüche 1 bis 10, **dadurch gekennzeichnet, dass** die selbstoxidierenden Indolfarbstoffe unter den Verbindungen der folgenden Formel (V) 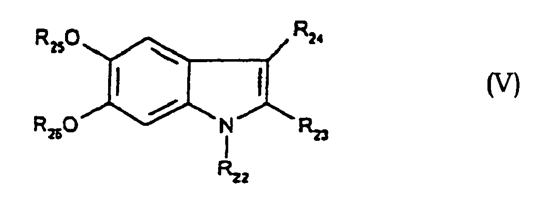 ausgewählt werden, in der bedeuten
- R₂₂, R₂₄, R₂₅ und R₂₆, gleich oder verschieden, Wasserstoff, C₁₋₄-Alkyl oder C₁₋₄-Acyl,
- R₂₃ Wasserstoff, C₁₋₄-Alkyl oder eine Carboxygruppe.

14. Zusammensetzung nach Anspruch 13, **dadurch gekennzeichnet, dass** die selbstoxidierenden Farbstoffe der Formel (V) unter 5,6-Dihydroxyindol, 2-Methyl-5,6-dihydroxyindol, 3-Methyl-5,6-dihydroxyindol, 1-Methyl-5,6-dihydroxyindol, 2,3-Dimethyl-5,6-dihydroxyindol, 5-Methoxy-6-hydroxyindol, 5-Acetoxy-6-hydroxyindol, 5,6-Diacetoxyindol, 5,6-Dihydroxyindol-2-carbonsäure und ihren Additionssalzen mit einer Säure ausgewählt sind.

15. Zusammensetzung nach einem der vorhergehenden Ansprüche, **dadurch gekennzeichnet, dass** der Mengenanteil des oder der selbstoxidierenden Farbstoffe im Bereich von 0,0005 bis 12 Gew.-% des Gesamtgewichts der Färbemittelzusammensetzung liegt.

16. Zusammensetzung nach Anspruch 15, **dadurch gekennzeichnet, dass** der Mengenanteil des oder der selbstoxidierenden Farbstoffe im Bereich von 0,005 bis 8 Gew.-% des Gesamtgewichts der Färbemittelzusammensetzung liegt.

17. Zusammensetzung nach einem der vorhergehenden Ansprüche, **dadurch gekennzeichnet, dass** sie ein oder mehrere Oxidationsmittel enthält, die unter Wasserstoffperoxid, Harnstoffperoxid, Alkalimetallbromaten, Persalzen, wie Perboraten und Persulfaten, und Enzymen ausgewählt sind.

18. Zusammensetzung nach Anspruch 17, **dadurch gekennzeichnet, dass** das oder die Enzyme unter den Peroxidasen und den Oxidoreduktasen, die zwei Elektronen übertragen (2-Elektronen-Oxidoreduktasen) ausgewählt sind.

19. Zusammensetzung nach Anspruch 18, **dadurch gekennzeichnet, dass** die 2-Elektronen-Oxidoreduktasen unter den Pyranoseoxidasen, den Glucoseoxidasen, den Glycerinoxidasen, den Lactatoxidasen, den Pyruvatoxidasen und den Uricasen ausgewählt sind.

20. Zusammensetzung nach Anspruch 18 oder 19, **dadurch gekennzeichnet, dass** die 2-Elektronen-Oxidoreduktase(n) unter den Uricasen tierischer, mikrobiologischer oder biotechnologischer Herkunft ausgewählt ist/sind.

21. Zusammensetzung nach einem der Ansprüche 18 bis 20, **dadurch gekennzeichnet, dass** die 2-Elektronen-Oxidoreduktase(n) in einem Mengenanteil von 0,01 bis 20 Gew.-% des Gesamtgewichts der gebrauchsfertigen Färbemittelzusammensetzung enthalten ist/sind.

22. Zusammensetzung nach Anspruch 21, **dadurch gekennzeichnet, dass** die Oxidoreduktase(n) für 2 Elektronen in einem Mengenanteil von 0,1 bis 5 Gew.-% des Gesamtgewichts der gebrauchsfertigen Färbemittelzusammensetzung enthalten ist/sind.

23. Zusammensetzung nach einem der Ansprüche 20 bis 22, **dadurch gekennzeichnet, dass** sie einen Donor (oder ein Substrat) für die 2-Elektronen-Oxidoreduktase enthält, der unter Harnsäure und ihren Salzen ausgewählt ist.

24. Zusammensetzung nach einem der Ansprüche 17 bis 23, **dadurch gekennzeichnet, dass** sie eine oder mehrere Oxidationsbasen, die unter den p-Phenylendiaminen, den p-Aminophenolen, den o-Phenylendiaminen und den heterocyclischen Basen ausgewählt sind, und/oder einen oder mehrere Kuppler enthält, die unter den m-Phenylendiaminen, den m-Aminophenolen, den m-Diphenolen, den heterocyclischen Kupplern, wie z.B. den Indol-Derivaten, den Indolin-Derivaten, den Benzimidazol-Derivaten, den Benzomorpholin-Derivaten, den Sesamol-Derivaten, den Pyridin-Derivaten, PyrimidinDerivaten und Pyrazol-Derivaten und ihren Additionssalzen mit einer Säure ausgewählt sind.

25. Zusammensetzung nach Anspruch 24, **dadurch gekennzeichnet, dass** die Oxidationsbase(n) in einem Mengenanteil von 0,0005 bis 12 Gew.-% des Gesamtgewichts der gebrauchsfertigen Färbemittelzusammensetzung enthalten sind und dass der oder die Kuppler in einem Mengenanteil von 0,0001 bis 10 Gew.-% des Gesamtgewichts der gebrauchsfertigen Färbemittelzusammensetzung enthalten sind.

26. Zusammensetzung nach Anspruch 25, **dadurch gekennzeichnet, dass** die Oxidationsbase(n) in einem Mengenanteil von 0,005 bis 8 Gew.-% des Gesamtgewichts der gebrauchsfertigen Färbemittelzusammensetzung enthalten sind und dass der oder die Kuppler in einem Mengenanteil von 0,005 bis 8 Gew.-% des Gesamtgewichts der gebrauchsfertigen Färbemittelzusammensetzung enthalten sind.

27. Zusammensetzung nach einem der Ansprüche 11, 12, 14 bis 16 und 24 bis 26, **dadurch gekennzeichnet, dass** die Additionssalze mit einer Säure unter Hydrochloriden, Hydrobromiden, Sulfaten, Tartraten, Lactaten und Acetaten ausgewählt sind.

28. Zusammensetzung nach einem der vorhergehenden Ansprüche, **dadurch gekennzeichnet, dass** das für die Färbung geeignete Medium aus Wasser oder einem Gemisch aus Wasser und mindestens einem organischen Lösemittel besteht.

29. Zusammensetzung nach einem der vorhergehenden Ansprüche, **dadurch gekennzeichnet, dass** sie eine pH-Wert im Bereich von 5 bis 11 aufweist.

30. Zusammensetzung nach einem der Ansprüche 1 und 9 bis 29, **dadurch gekennzeichnet, dass** der kationische direktziehende Farbstoff die folgende Formel 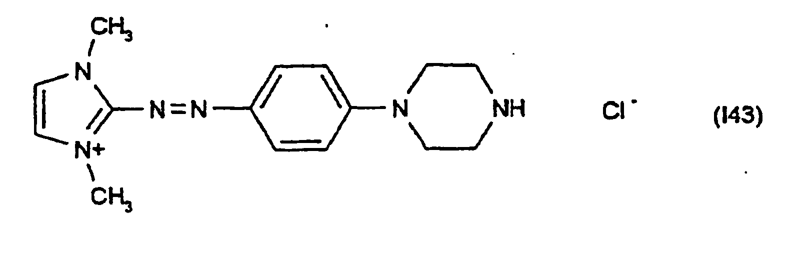 aufweist.

31. Verfahren zum Färben von Keratinfasern und insbesondere von menschlichen Keratinfasern, wie den Haaren, **dadurch gekennzeichnet, dass** auf diese Fasern mindestens eine gebrauchsfertige Färbemittelzusammensetzung, die wie in einem der vorhergehenden Ansprüche definiert ist, aufgetragen und während eines Zeitraums einwirken gelassen wird, der ausreichend ist, um die gewünschte Färbung zu entwickeln.

32. Verfahren nach Anspruch 31, **dadurch gekennzeichnet, dass** es einen vorbereitenden Schritt umfaßt, der darin besteht, in getrennter Form einerseits eine Zusammensetzung (A), die in einem für die Färbung geeigneten Medium mindestens einen kationischen direktziehenden Farbstoff und mindestens einen selbstoxidierenden Farbstoff enthält, und andererseits eine Zusammensetzung (B), die in einem für die Färbung geeigneten Medium mindestens ein Oxidationsmittel enthält, aufzubewahren, wonach diese beiden Zusammensetzungen zum Zeitpunkt ihrer Anwendung vor dem Auftragen dieses Gemischs auf die Keratinfasern vermischt werden.

33. Vorrichtung mit mehreren Abteilungen oder "Kit" zum Färben, **dadurch gekennzeichnet, dass** sie/er eine erste Abteilung, die die Zusammensetzung (A) enthält, die wie in Anspruch 32 definiert ist, und eine zweite Abteilung, die die Zusammensetzung (B) enthält, die wie in Anspruch 32 definiert ist, umfaßt.
